# EUROPEAN PATENT SPECIFICATION

(11) **EP 1 218 515 B1**
(45) Date of publication and mention of the grant of the patent: **11.02.2009**
(21) Application number: 00942914.3
(22) Date of filing: 16.06.2000
(51) Int. Cl.: C12N 15/52, C07K 14/705, C12Q 1/68, C07K 16/18, A61K 38/00

(54) **REGULATION WITH BINDING CASSETTE TRANSPORTER PROTEIN ABC1**
REGULIERUNG MITTELS ATP BINDING CASSETTE TRANSPORTER PROTEINS ABC1
REGULATION AU MOYEN DE LA PROTEINE DE TRANSPORT DE CASSETTES DE LIAISON D'ATP ABC1

(30) Priority: 18.06.1999 US 140264 P; 14.09.1999 US 153872 P; 19.11.1999 US 166573 P
(43) Date of publication of application: 03.07.2002
(73) Proprietor: CV THERAPEUTICS, INC., Palo Alto, CA 94304 (US)
(72) Inventor: LAWN, Richard, M., San Francisco, CA 94116 (US); WADE, David, Palo Alto, CA 94306 (US); GARVIN, Michael, San Francisco, CA 94019 (US)
(74) Representative: Schnappauf, Georg
(86) International application number: PCT/US2000/016765
(87) International publication number: WO 2000/078972

(56) References cited:
- WO-A-00/55318
- WO-A-91/15213
- CA-A1- 2 295 930
- KLIEWER ET AL.: "Orphan nuclear receptors: shifting endocrinology into reverse" SCIENCE, vol. 284, 30 April 1999 (1999-04-30), pages 757-760, XP002376812
- PETERS ET AL.: "Alterations in lipoprotein metabolism in peroxisome proliferator-activated receptor alpha-deficient mice" THE JOURNAL OF BIOLOGICAL CHEMISTRY, vol. 272, no. 43, 24 October 1997 (1997-10-24), pages 27307-27312, XP002376813
- LENHARD ET AL.: "The RXR agonist LG100268 causes hepatomegaly, improves glycaemic control and decrease cardiovascular risk and cachexia in diabetic mice suffering from pancreatic beta-cell dysfunction" DIABETOLOGIA, vol. 42, May 1999 (1999-05), pages 545-554, XP002376814
- LEHMANN ET AL.: "Activation of the nucelar receptor LXR by oxysterols defines a new hormone response pathway" THE JOURNAL OF BIOLOGICAL CHEMISTRY, vol. 272, no. 6, 7 February 1997 (1997-02-07), pages 3137-3140, XP002376815
- MORISAKI ET AL.: "inhibitory effect of prostaglandin E2 on cholesterol ester accumulation in macrophages" BIOCHEMICAL AND BIOCHEMICAL RESEARCH COMMUNICATIONS, vol. 137, 29 May 1986 (1986-05-29), pages 461-467, XP009065261
- MUKHERJEE R ET AL: "RXR AGONISTS ACTIVATE PPAR ALPHA-INDUCIBLE GENES, LOWER TRIGLYCERIDES, AND RAISE HDL LEVELS IN VIVO" 1998, ARTERIOSCLEROSIS, THROMBOSIS, AND VASCULAR BIOLOGY, XX, XX, PAGE(S) 272-276 , XP000913726 ISSN: 1079-5642 * the whole document *
- POMERANTZ ET AL.: "High-density-lipoprotein-induced cholesterol efflux from arterial smooth muscle cell derived foam cells: Functional relationship of the cholesteryl ester cycle and eicosanoid biosynthesis" BIOCHEMISTRY, vol. 29, 1990, pages 1892-1899, XP002376817
- ZOUBOULIS C C ET AL: "EFFECTS OF 13-CIS-RETINOIC ACID, ALL-TRANS-RETINOIC ACID, AND ACITRETIN ON THE PROLIFERATION, LIPID SYNTHESIS AND KERATIN EXPRESSION OF CULTURED HUMAN SEBOCYTES IN VITRO" JOURNAL OF INVESTIGATIVE DERMATOLOGY, NEW YORK, NY, US, vol. 96, no. 5, May 1991 (1991-05), pages 792-797, XP002048067 ISSN: 0022-202X
- PEET ET AL.: "Cholesterol and Bile Acid metabolism are impaired in mice lacking the nuclear oxysterol receptor LXRalpha" CELL, vol. 93, May 1998 (1998-05), pages 693-704, XP002376818
- PULLINGER C R ET AL: "ANALYSIS OF HABC1 GENE 5' END: ADDITIONAL PEPTIDE SEQUENCE, PROMOTER REGION, AND FOUR POLYMORPHISMS" BIOCHEMICAL AND BIOPHYSICAL RESEARCH COMMUNICATIONS, ACADEMIC PRESS INC. ORLANDO, FL, US, vol. 271, no. 2, 10 May 2000 (2000-05-10), pages 451-455, XP000991244 ISSN: 0006-291X
- CHAWLA ET AL.: "Nuclear receptors and lipid physilogy: opening the X-files" SCIENCE, vol. 294, 30 November 2001 (2001-11-30), pages 1867-1870, XP002376819
- LANGMANN T, KLUCKEN J, REIL M, LIEBISCH G, LUCIANI MF, CHIMINI G, KAMINSKI WE, SCHMITZ G: "Molecular cloning of the human ATP-binding cassette transporter 1 (hABC1): evidence for sterol-dependent regulation in macrophages" BIOCHEM BIOPHYS RES COMMUN, vol. 257, no. 1, 2 April 1999 (1999-04-02), pages 29-33, XP000877240
- PULLINGER CR, HAKAMATA H, DUCHATEAU PN, ENG C, AOUIZERAT BE, CHO MH, FIELDING CJ, KANE JP.: "Analysis of hABC1 gene 5' end: additional peptide sequence, promoter region, and four polymorphisms." BIOCHEM BIOPHYS RES COMMUN. 2000 MAY 10;271(2):451-5., XP000991244
- SCHWARTZ K ET AL: "ABC1 GENE EXPRESSION AND APOA-I-MEDIATED CHOLESTER0L EFFLUX ARE REGULATED BY LXR" BIOCHEMICAL AND BIOPHYSICAL RESEARCH COMMUNICATIONS,ACADEMIC PRESS INC. ORLANDO, FL,US, vol. 274, no. 3, 11 August 2000 (2000-08-11), pages 794-802, XP000960872 ISSN: 0006-291X

### SEQUENCE LISTING

<110> Lawn, Richard M.
   Wade, David
   Garvin, Michael
   Oram, John F.
<120> Compositions and Methods for Increasing Cholesterol Efflux and Raising HDL using ATP Binding Cassette Transporter Protein ABC1
<130> 99, 395-A
<140>
   <141>
<150> US 60/140,264
   <151> 1999-06-18
<150> US 60/153,872
   <151> 1999-08-14
<150> US 60/166,573
   <151> 1999-11-19
<160> 57
<170> PatentIn Ver. 2.0
<210> 1
   <211> 10442
   <212> DNA
   <213> Homo sapiens
<400> 1
   ggccgggacc cgcagagccg agccgaccct tctctcccgg gctgcggcag ggcagggcgg 60
   ggagctccgc gcaccaacag agccggttct cagggcgctt tgctccttgt tttttccccg 120
   gttctgtttt ctccccttct ccggaaggct tgtcaagggg taggagaaag agacgcaaac 180
   acaaaagtgg aaaacagtta atgaccagcc acgggcgtcc ctgctgtgag ctctggccgc 240
   tgccttccag ggctcccgag ccacacgctg ggcgtgctgg ctgagggaac atggcttgtt 300
   ggcctcagct gaggttgctg ctgtggaaga acctcacttt cagaagaaga caaacatgtc 360
   agctgttact ggaagtggcc tggcctctat ttatcttcct gatcctgatc tctgttcggc 420
   tgagctaccc accctatgaa caacatgaat gccattttcc aaataaagcc atgccctctg 480
   caggaacact tccttgggtt caggggatta tctgtaatgc caacaacccc tgtttccgtt 540
   acccgactcc tggggaggct cccggagttg ttggaaactt taacaaatcc attgtggctc 600
   gcctgttctc agatgctcgg aggcttcttt tatacagcca gaaagacacc agcatgaagg 660
   acatgcgcaa agttctgaga acattacagc agatcaagaa atccagctca aacttgaagc 720
   ttcaagattt cctggtggac aatgaaacct tctctgggtt cctatatcac aacctctctc 780
   tcccaaagtc tactgtggac aagatgctga gggctgatgt cattctccac aaggtatttt 840
   tgcaaggcta ccagttacat ttgacaagtc tgtgcaatgg atcaaaatca gaagagatga 900
   ttcaacttgg tgaccaagaa gtttctgagc tttgtggcct accaaaggag aaactggctg 960
   cagcagagcg agtacttcgt tccaacatgg acatcctgaa gccaatcctg agaacactaa 1020
   actctacatc tcccttcccg agcaaggagc tggctgaagc cacaaaaaca ttgctgcata 1080
   gtcttgggac tctggcccag gagctgttca gcatgagaag ctggagtgac atgcgacagg 1140
   aggtgatgtt tctgaccaat gtgaacagct ccagctcctc cacccaaatc taccaggctg 1200
   tgtctcgtat tgtctgcggg catcccgagg gaggggggct gaagatcaag tctctcaact 1260
   ggtatgagga caacaactac aaagccctct ttggaggcaa tggcactgag gaagatgctg 1320
   aaaccttcta tgacaactct acaactcctt actgcaatga tttgatgaag aatttggagt 1380
   ctagtcctct ttcccgcatt atctggaaag ctctgaagcc gctgctcgtt gggaagatcc 1440
   tgtatacacc tgacactcca gccacaaggc aggtcatggc tgaggtgaac aagaccttcc 1500
   aggaactggc tgtgttccat gatctggaag gcatgtggga ggaactcagc cccaagatct 1560
   ggaccttcat ggagaacagc caagaaatgg accttgtccg gatgctgttg gacagcaggg 1620
   acaatgacca cttttgggaa cagcagttgg atggcttaga ttggacagcc caagacatcg 1680
   tggcgttttt ggccaagcac ccagaggatg tccagtccag taatggttct gtgtacacct 1740
   ggagagaagc tttcaacgag actaaccagg caatccggac catatctcgc ttcatggagt 1800
   gtgtcaacct gaacaagcta gaacccatag caacagaagt ctggctcatc aacaagtcca 1860
   tggagctgct ggatgagagg aagttctggg ctggtattgt gttcactgga attactccag 1920
   gcagcattga gctgccccat catgtcaagt acaagatccg aatggacatt gacaatgtgg 1980
   agaggacaaa taaaatcaag gatgggtact gggaccctgg tcctcgagct gacccctttg 2040
   aggacatgcg gtacgtctgg gggggcttcg cctacttgca ggatgtggtg gagcaggcaa 2100
   tcatcagggt gctgacgggc accgagaaga aaactggtgt ctatatgcaa cagatgccct 2160
   atccctgtta cgttgatgac atctttctgc gggtgatgag ccggtcaatg cccctcttca 2220
   tgacgctggc ctggatttac tcagtggctg tgatcatcaa gggcatcgtg tatgagaagg 2280
   aggcacggct gaaagagacc atgcggatca tgggcctgga caacagcata ctctggttta 2340
   gctggttcat tagtagcctc attcctcttc ttgtgagcgc tggcctgcta gtggtcatcc 2400
   tgaagttagg aaacctgctg ccctacagtg atcccagcgt ggtgtttgtc ttcctgtccg 2460
   tgtttgctgt ggtgacaatc ctgcagtgct tcctgattag cacactcttc tccagagcca 2520
   acctggcagc agcctgtggg ggcatcatct acttcacgct gtacctgccc tacgtcctgt 2580
   gtgtggcatg gcaggactac gtgggcttca cactcaagat cttcgctagc ctgctgtctc 2640
   ctgtggcttt tgggtttggc tgtgagtact ttgccctttt tgaggagcag ggcattggag 2700
   tgcagtggga caacctgttt gagagtcctg tggaggaaga tggcttcaat ctcaccactt 2760
   cgatctccat gatgctgttt gacaccttcc tctatggggt gatgacctgg tacattgagg 2820
   ctgtctttcc aggccagtac ggaattccca ggccctggta ttttccttgc accaagtcct 2880
   actggtttgg cgaggaaagt gatgagaaga gccaccctgg ttccaaccag aagagaatgt 2940
   cagaaatctg catggaggag gaacccaccc acttgaagct gggcgtgtcc attcagaacc 3000
   tggtaaaagt ctaccgagat gggatgaagg tggctgtcga tggcctggca ctgaattttt 3060
   atgagggcca gatcacctcc ttcctgggcc acaatggagc ggggaagacg accaccatgt 3120
   caatcctgac cgggttgttc cccccgacct cgggcaccgc ctacatcctg ggaaaagaca 3180
   ttcgctctga gatgagcacc atccggcaga acctgggggt ctgtccccag cataacgtgc 3240
   tgtttgacat gctgactgtc gaagaacaca tctggttcta tgcccgcttg aaagggctct 3300
   ctgagaagca cgtgaaggcg gagatggagc agatggccct ggatgttggt ttgccatcaa 3360
   gcaagctgaa aagcaaaaca agccagctgt caggtggaat gcagagaaag ctatctgtgg 3420
   ccttggcctt tgtcggggga tctaaggttg tcattctgga tgaacccaca gctggtgtgg 3480
   acccttactc ccgcagggga atatgggagc tgctgctgaa ataccgacaa ggccgcacca 3540
   ttattctctc tacacaccac atggatgaag cggacgtcct gggggacagg attgccatca 3600
   tctcccatgg gaagctgtgc tgtgtgggct cctccctgtt tctgaagaac cagctgggaa 3660
   caggctacta cctgaccttg gtcaagaaag atgtggaatc ctccctcagt tcctgcagaa 3720
   acagtagtag cactgtgtca tacctgaaaa aggaggacag tgtttctcag agcagttctg 3780
   atgctggcct gggcagcgac catgagagtg acacgctgac catcgatgtc tctgctatct 3840
   ccaacctcat caggaagcat gtgtctgaag cccggctggt ggaagacata gggcatgagc 3900
   tgacctatgt gctgccatat gaagctgcta aggagggagc ctttgtggaa ctctttcatg 3960
   agattgatga ccggctctca gacctgggca tttctagtta tggcatctca gagacgaccc 4020
   tggaagaaat attcctcaag gtggccgaag agagtggggt ggatgctgag acctcagatg 4080
   gtaccttgcc agcaagacga aacaggcggg ccttcgggga caagcagagc tgtcttcgcc 4140
   cgttcactga agatgatgct gctgatccaa atgattctga catagaccca gaatccagag 4200
   agacagactt gctcagtggg atggatggca aagggtccta ccaggtgaaa ggctggaaac 4260
   ttacacagca acagtttgtg gcccttttgt ggaagagact gctaattgcc agacggagtc 4320
   ggaaaggatt ttttgctcag attgtcttgc cagctgtgtt tgtctgcatt gcccttgtgt 4380
   tcagcctgat cgtgccaccc tttggcaagt accccagcct ggaacttcag ccctggatgt 4440
   acaacgaaca gtacacattt gtcagcaatg atgctcctga ggacacggga accctggaac 4500
   tcttaaacgc cctcaccaaa gaccctggct tcgggacccg ctgtatggaa ggaaacccaa 4560
   tcccagacac gccctgccag gcaggggagg aagagtggac cactgcccca gttccccaga 4620
   ccatcatgga cctcttccag aatgggaact ggacaatgca gaacccttca cctgcatgcc 4680
   agtgtagcag cgacaaaatc aagaagatgc tgcctgtgtg tcccccaggg gcaggggggc 4740
   tgcctcctcc acaaagaaaa caaaacactg cagatatcct tcaggacctg acaggaagaa 4800
   acatttcgga ttatctggtg aagacgtatg tgcagatcat agccaaaagc ttaaagaaca 4860
   agatctgggt gaatgagttt aggtatggcg gcttttccct gggtgtcagt aatactcaag 4920
   cacttcctcc gagtcaagaa gttaatgatg ccatcaaaca aatgaagaaa cacctaaagc 4980
   tggccaagga cagttctgca gatcgatttc tcaacagctt gggaagattt atgacaggac 5040
   tggacaccag aaataatgtc aaggtgtggt tcaataacaa gggctggcat gcaatcagct 5100
   ctttcctgaa tgtcatcaac aatgccattc tccgggccaa cctgcaaaag ggagagaacc 5160
   ctagccatta tggaattact gctttcaatc atcccctgaa tctcaccaag cagcagctct 5220
   cagaggtggc tctgatgacc acatcagtgg atgtccttgt gtccatctgt gtcatctttg 5280
   caatgtcctt cgtcccagcc agctttgtcg tattcctgat ccaggagcgg gtcagcaaag 5340
   caaaacacct gcagttcatc agtggagtga agcctgtcat ctactggctc tctaattttg 5400
   tctgggatat gtgcaattac gttgtccctg ccacactggt cattatcatc ttcatctgct 5460
   tccagcagaa gtcctatgtg tcctccacca atctgcctgt gctagccctt ctacttttgc 5520
   tgtatgggtg gtcaatcaca cctctcatgt acccagcctc ctttgtgttc aagatcccca 5580
   gcacagccta tgtggtgctc accagcgtga acctcttcat tggcattaat ggcagcgtgg 5640
   ccacctttgt gctggagctg ttcaccgaca ataagctgaa taatatcaat gatatcctga 5700
   agtccgtgtt cttgatcttc ccacattttt gcctgggacg agggctcatc gacatggtga 5760
   aaaaccaggc aatggctgat gccctggaaa ggtttgggga gaatcgcttt gtgtcaccat 5820
   tatcttggga cttggtggga cgaaacctct tcgccatggc cgtggaaggg gtggtgttct 5880
   tcctcattac tgttctgatc cagtacagat tcttcatcag gcccagacct gtaaatgcaa 5940
   agctatctcc tctgaatgat gaagatgaag atgtgaggcg ggaaagacag agaattcttg 6000
   atggtggagg ccagaatgac atcttagaaa tcaaggagtt gacgaagata tatagaagga 6060
   agcggaagcc tgctgttgac aggatttgcg tgggcattcc tcctggtgag tgctttgggc 6120
   tcctgggagt taatggggct ggaaaatcat caactttcaa gatgttaaca ggagatacca 6180
   ctgttaccag aggagatgct ttccttaaca aaaatagtat cttatcaaac atccatgaag 6240
   tacatcagaa catgggctac tgccctcagt ttgatgccat cacagagctg ttgactggga 6300
   gagaacacgt ggagttcttt gcccttttga gaggagtccc agagaaagaa gttggcaagg 6360
   ttggtgagtg ggcgattcgg aaactgggcc tcgtgaagta tggagaaaaa tatgctggta 6420
   actatagtgg aggcaacaaa cgcaagctct ctacagccat ggctttgatc ggcgggcctc 6480
   ctgtggtgtt tctggatgaa cccaccacag gcatggatcc caaagcccgg cggttcttgt 6540
   ggaattgtgc cctaagtgtt gtcaaggagg ggagatcagt agtgcttaca tctcatagta 6600
   tggaagaatg tgaagctctt tgcactagga tggcaatcat ggtcaatgga aggttcaggt 6660
   gccttggcag tgtccagcat ctaaaaaata ggtttggaga tggttataca atagttgtac 6720
   gaatagcagg gtccaacccg gacctgaagc ctgtccagga tttctttgga cttgcatttc 6780
   ctggaagtgt tctaaaagag aaacaccgga acatgctaca ataccagctt ccatcttcat 6840
   tatcttctct ggccaggata ttcagcatcc tctcccagag caaaaagcga ctccacatag 6900
   aagactactc tgtttctcag acaacacttg accaagtatt tgtgaacttt gccaaggacc 6960
   aaagtgatga tgaccactta aaagacctct cattacacaa aaaccagaca gtagtggacg 7020
   ttgcagttct cacatctttt ctacaggatg agaaagtgaa agaaagctat gtatgaagaa 7080
   tcctgttcat acggggtggc tgaaagtaaa gaggaactag actttccttt gcaccatgtg 7140
   aagtgttgtg gagaaaagag ccagaagttg atgtgggaag aagtaaactg gatactgtac 7200
   tgatactatt caatgcaatg caattcaatg caatgaaaac aaaattccat tacaggggca 7260
   gtgcctttgt agcctatgtc ttgtatggct ctcaagtgaa agacttgaat ttagtttttt 7320
   acctatacct atgtgaaact ctattatgga acccaatgga catatgggtt tgaactcaca 7380
   cttttttttt ttttttgttc ctgtgtattc tcattggggt tgcaacaata attcatcaag 7440
   taatcatggc cagcgattat tgatcaaaat caaaaggtaa tgcacatcct cattcactaa 7500
   gccatgccat gcccaggaga ctggtttccc ggtgacacat ccattgctgg caatgagtgt 7560
   gccagagtta ttagtgccaa gtttttcaga aagtttgaag caccatggtg tgtcatgctc 7620
   acttttgtga aagctgctct gctcagagtc tatcaacatt gaatatcagt tgacagaatg 7680
   gtgccatgcg tggctaacat cctgctttga ttccctctga taagctgttc tggtggcagt 7740
   aacatgcaac aaaaatgtgg gtgtctctag gcacgggaaa cttggttcca ttgttatatt 7800
   gtcctatgct tcgagccatg ggtctacagg gtcatcctta tgagactctt aaatatactt 7860
   agatcctggt aagaggcaaa gaatcaacag ccaaactgct ggggctgcaa gctgctgaag 7920
   ccagggcatg ggattaaaga gattgtgcgt tcaaacctag ggaagcctgt gcccatttgt 7980
   cctgactgtc tgctaacatg gtacactgca tctcaagatg tttatctgac acaagtgtat 8040
   tatttctggc tttttgaatt aatctagaaa atgaaaagat ggagttgtat tttgacaaaa 8100
   atgtttgtac tttttaatgt tatttggaat tttaagttct atcagtgact tctgaatcct 8160
   tagaatggcc tctttgtaga accctgtggt atagaggagt atggccactg ccccactatt 8220
   tttattttct tatgtaagtt tgcatatcag tcatgactag tgcctagaaa gcaatgtgat 8280
   ggtcaggatc tcatgacatt atatttgagt ttctttcaga tcatttagga tactcttaat 8340
   ctcacttcat caatcaaata ttttttgagt gtatgctgta gctgaaagag tatgtacgta 8400
   cgtataagac tagagagata ttaagtctca gtacacttcc tgtgccatgt tattcagctc 8460
   actggtttac aaatataggt tgtcttgtgg ttgtaggagc ccactgtaac aatattgggc 8520
   agcctttttt tttttttttt aattgcaaca atgcaaaagc caagaaagta taagggtcac 8580
   aagtttaaac aatgaattct tcaacaggga aaacagctag cttgaaaact tgctgaaaaa 8640
   cacaacttgt gtttatggca tttagtacct tcaaataatt ggctttgcag atattggata 8700
   ccccattaaa tctgacagtc tcaaattttt catctcttca atcactagtc aagaaaaata 8760
   taaaaacaac aaatacttcc atatggagca tttttcagag ttttctaacc cagtcttatt 8820
   tttctagtca gtaaacattt gtaaaaatac tgtttcacta atacttactg ttaactgtct 8880
   tgagagaaaa gaaaaatatg agagaactat tgtttgggga agttcaagtg atctttcaat 8940
   atcattacta acttcttcca ctttttccaa aatttgaata ttaacgctaa aggtgtaaga 9000
   cttcagattt caaattaatc tttctatatt ttttaaattt acagaatatt atataaccca 9060
   ctgctgaaaa agaaaaaaat gattgtttta gaagttaaag tcaatattga ttttaaatat 9120
   aagtaatgaa ggcatatttc caataactag tgatatggca tcgttgcaat ttacagtatc 9180
   ttcaaaaata cagaatttat agaataattt ctcctcattt aatatttttc aaaatcaaag 9240
   ttatggtttc ctcattttac taaaatcgta ttctaattct tcattatagt aaatctatga 9300
   gcaactcctt acttcggttc ctctgatttc aaggccatat tttaaaaaat caaaaggcac 9360
   tgtgaactat tttgaagaaa acacgacatt ttaatacaga ttgaaaggac ctcttctgaa 9420
   gctagaaaca atctatagtt atacatcttc attaatactg tgttaccttt taaaatagta 9480
   attttttaca ttttcctgtg taaacctaat tgtggtagaa atttttacca actctatact 9540
   caatcaagca aaatttctgt atattccctg tggaatgtac ctatgtgagt ttcagaaatt 9600
   ctcaaaatac gtgttcaaaa atttctgctt ttgcatcttt gggacacctc agaaaactta 9660
   ttaacaactg tgaatatgag aaatacagaa gaaaataata agccctctat acataaatgc 9720
   ccagcacaat tcattgttaa aaaacaacca aacctcacac tactgtattt cattatctgt 9780
   actgaaagca aatgctttgt gactattaaa tgttgcacat cattcattca ctgtatagta 9840
   atcattgact aaagccattt gctgtgtttt cttcttgtgg ntgnatatat caggtaaaat 9900
   attttccaaa gagccatgtg tcatgtaata ctgaaccctt tgatattgag acattaattt 9960
   ggacccttgg tattatctac tagaataatg taatactgna gaaatattgc tctaattctt 10020
   tcaaaatggt gcatccccct taaaangttc tatttccata aggatttagc ttgcttatcc 10080
   cttcttatac cctaagatga agctgttttt gtgctctttg ttcatcattg gccctcattc 10140
   caagcacttt acgctgtctg taatgggatc tatttttgca ctggaatatc tgagaattgc 10200
   aaaactagac aaaagtttca caacagattt ctaagttaaa tcattttcat taaaaggaaa 10260
   aaagaaaaaa aattttgtat gtcaataact ttatatgaag tattaaaatg catatttcta 10320
   tgttgtaata taatgagtca caaaataaag ctgtgacagt tctgttaaaa aaaaaaaaaa 10380
   aaaaaaaaaa aaaaaaaaaa aaaaaaaaaa aaaaaaaaaa aaaaaaaaaa aaaaaaaaaa 10440
   aa                                                                10442
<210> 2
   <211> 2261
   <212> PRT
   <213> Homo sapiens
<400> 2
<210> 3
   <211> 1643
   <212> DNA
   <213> Homo sapiens
<400> 3
   gaattccttg ctggtggctc cacatgcact tccagggcct gcttggctct tctatgggtc 60
   tgtcctgagt gttgatagaa ccactgatgt gagtacctgg gcttgagcgt ggcctggaga 120
   tcctgttgac tgtagcatgg agggggcttg tcagctgaat gtctgtatgc aggtggtggg 180
   agttctggaa tatgatggag ctggaggtgg gaagagaagt aggcttgggg cagctctctc 240
   atgccacctc attctggcca aaactcaggt caaactgtga agagtctaaa tgtgaatctg 300
   cccttcaagg tggctacaaa ggtatctttg tcaaggtagg agaccttgtg gcctccacgt 360
   gcacttccag ggcctgcttg gcctcttcta cgggtctgtc ctgagtcttc tatgaatctc 420
   ccttcagggc agattcatat ttagactctt cacagtttga cctgagtttt ggccagaata 480
   aggtgacatt tagtttgttg gcttgatgaa tgacttaaat atttagacat atggtgtgta 540
   ggcctgcatt cctactcttg cctttttttt tgcccctcca gtgttttggg tagttttgct 600
   ccccctacag ccaaaggcaa acagataagt tggaggtctg gagtggctac ataattttac 660
   acgactgcaa ttctctggct gcacttcaca aatgtataca aactaaatac aagtcctgtg 720
   tttttatcac agggaggctg atcaatataa tgaaattaaa agggggctgg tcccatattg 780
   ttctgtgttt ttgtttgttt gtttcttttt ttgtttttgt ggcctccttc ctctcaattt 840
   atgaagagaa gcagtaagat gttcctctcg ggtcctctga gggacctggg gagctcaggc 900
   tgggaatctc caaggcagta ggtcgcctat caaaaatcaa agtccaggtt tgtgggggga 960
   aaacaaaagc agcccattac ccagaggact gtccgccttc ccctcacccc agcctaggcc 1020
   tttgaaagga aacaaaagac aagacaaaat gattggcgtc ctgagggaga ttcagcctag 1080
   agctctctct cccccaatcc ctccctccgg ctgaggaaac taacaaagga aaaaaaaatt 1140
   gcggaaagca ggatttagag gaagcaaatt ccactggtgc ccttggctgc cgggaacgtg 1200
   gactagagag tctgcggcgc agccccgagc ccagcgcttc ccgcgcgtct taggccggcg 1260
   ggcccgggcg ggggaagggg acgcagaccg cggaccctaa gacacctgct gtaccctcca 1320
   cccccacccc acccacctcc ccccaactcc ctagatgtgt cgtgggcggc tgaacgtcgc 1380
   ccgtttaagg ggcgggcccc ggctccacgt gctttctgct gagtgactga actacataaa 1440
   cagaggccgg gaacggggcg gggaggaggg agagcacagg ctttgaccga tagtaacctc 1500
   tgcgctcggt gcagccgaat ctataaaagg aactagtccc ggcaaaaacc ccgtaattgc 1560
   gagcgagagt gagtggggcc gggacccgca gagccgagcc gacccttctc tcccgggctg 1620
   cggcagggca gggcggggag ctc                                         1643
<210> 4
   <211> 748
   <212> DNA
   <213> Homo sapiens
<400> 4
   attccaanaa cattttccng catctgtggt tgccaactca caatgtcttt cattttctga 60
   acttacccnc caaatgaagn tcggcacgca attatgtagt agcnactctt aggcccccgg 120
   cttacactta tgcttccggc tngttgtgtg ggaattggac ggataccatt tcacncagga 180
   aacagatatg nccatgatta cgccaagtta tttaggtgcn cgatagaata ctcaagcttg 240
   gaattcgcgg ccgcagtcga cggacccccg ggaaagattc ctctcattac acaaaaacca 300
   gacagtagtg gacgttgcag ttctcacatc ttctctacag gatgagaaag tgaaagaaag 360
   ctatgtatga agaatcctgt tcatacgggg tggctgaaag taaagaggaa ctagactttc 420
   ctttgcacca tgtgaagtgt tgtggagaaa agagccagaa gttgatgtgg gaagaagtaa 480
   actggatact gtactgatac tattcaatgc aatgcaattc aatgcaatga aaacaaaatt 540
   ccattacagg ggcagtgcct ttgtagccta tgtcttgtat ggctctcaag tgaaagactt 600
   gaatttagtt ttttacctat acctatgtga aactctatta tggaacccaa tggacatatg 660
   ggtttgaact cacacttttt tttttttgtt cctgtgtatt ctcattgggg ttgcaacaat 720
   aattcatcaa gtaaaaaaaa aaaaaaaa                                    748
<210> 5
   <211> 2011
   <212> DNA
   <213> Homo sapiens
<400> 5
   agaatcctgt tcatactggg gtggcttgaa agtaaatgga ggaactagac tttcctttgc 60
   accatgtgaa gtgttgtgga gaaaagagcc agaagttgat gtgggaagaa gtaaactgga 120
   tactgtactg atactattca atgcaatgca attcaatgca atgaaaacaa aattccatta 180
   caggggcaag tgcctttgta gcccatgtct tgtatggctc tcaagtgaaa gacttgaatt 240
   tagtttttta cctataccta tgtgaaactc tattatggaa cccaatggac atatgggttt 300
   gaactcacac tttttttttt ttttgttcct gtgtattctc attggggttg caacaataat 360
   tcatcaagta atcatggcca gcgattattg atcaaaatca aaaggtaatg cacatcctca 420
   ttcactaagc catgccatgc ccaggagact ggtttcccgg tgacacatcc attgctggca 480
   atgagtgtgc cagagttatt agtgccaagt ttttcagaaa gtttgaagca ccatggtgtg 540
   tcatgctcac ttttgtgaaa gctgctctgc tcagagtcta tcaacattga atatcagttg 600
   acagaatggt gccatgcgtg gctaacatcc tgctttgatt ccctctgata agctgttctg 660
   gtggcagtaa catgcaacaa aaatgtgggt gtctctaggc acgggaaact tggttccatt 720
   gttatattgt cctatgcttc gagccatggg tctacagggt catccttatg agactcttaa 780
   atatacttag atcctggtaa gaggcaaaga atcaacagcc aaactgctgg ggctgcaagc 840
   tgctgaagcc agggcatggg attaaagaga ttgtgcgttc aaacctaggg aagcctgtgc 900
   ccatttgtcc tgactgtctg ctaacatggt acactgcatc tcaagatgtt tatctgacac 960
   aagtgtatta tttctggctt tttgaattaa tctagaaaat gaaaagatgg agttgtattt 1020
   tgacaaaaat gtttgtactt tttaatgtta tttggaattt taagttctat cagtgacttc 1080
   tgaatcctta gaatggcctc tttgtagaac cctgtggtat agaggagtat ggccactgcc 1140
   ccactatttt tattttctta tgtaagtttg catatcagtc atgactagtg cctagaaagc 1200
   aatgtgatgg tcaggatctc atgacattat atttgagttt ctttcagatc atttaggata 1260
   ctcttaatct cacttcatca atcaaatatt ttttgagtgt atgctgtagc tgaaagagta 1320
   tgtacgtacg tataagacta gagagatatt aagtctcagt acacttcctg tgccatgtta 1380
   ttcagctcac tggtttacaa atataggttg tcttgtggtt gtaggagccc actgtaacaa 1440
   tattgggcag cctttttttt tttttttttt aattgcaaca atgcaaaagc caagaaagta 1500
   taagggtcac aagtctaaac aatgaattct tcaacaggga aaacagctag ctagaaaact 1560
   tgctgaaaac acaacttgtg tttatggcat ttagtacctt caaataattg gctttgcaga 1620
   tattggatac cccattaaat ctgacagtct caaatttttc atctcttcaa tcactagtca 1680
   agaaaaatat aaaaacaaca aatacttcca tatggagcat ttttcagagt tttctaaccc 1740
   agtcttattt ttctagtcag taaacatttg taaaaatact gtttcactaa tacttactgt 1800
   taactgtctt gagagaaaag aaaaatatga gagaactatt gtttggggaa gttcaagtga 1860
   tctttcaata tcattactaa cttcttccac tttttccaaa atttgaatat taacgctaaa 1920
   ggtgtaagga cttcagattt caaattaatc tttctatatt ttttaaattt acagaatatt 1980
   atataaccca ctgctgaaaa aaaaaaaaaa a 2011
<210> 6
   <211> 3366
   <212> DNA
   <213> Homo sapiens
<400> 6
   agaatcctgt tcatacgggg tggctgaaag taaagaggaa ctagactttc ctttgcacca 60
   tgtgaagtgt tgtggagaaa agagccagaa gttgatgtgg gaagaagtaa actggatact 120
   gtactgatac tattcaatgc aatgcaattc aatgcaatga aaacaaaatt ccattacagg 180
   ggcagtgcct ttgtagccta tgtcttgtat ggctctcaag tgaaagactt gaatttagtt 240
   ttttacctat acctatgtga aactctatta tggaacccaa tggacatatg ggtttgaact 300
   cacacttttt tttttttttt gttcctgtgt attctcattg gggttgcaac aataattcat 360
   caagtaatca tggccagcga ttattgatca aaatcaaaag gtaatgcaca tcctcattca 420
   ctaagccatg ccatgcccag gagactggtt tcccggtgac acatccattg ctggcaatga 480
   gtgtgccaga gttattagtg ccaagttttt cagaaagttt gaagcaccat ggtgtgtcat 540
   gctcactttt gtgaaagctg ctctgctcag agtctatcaa cattgaatat cagttgacag 600
   aatggtgcca tgcgtggcta acatcctgct ttgattccct ctgataagct gttctggtgg 660
   cagtaacatg caacaaaaat gtgggtgtct ctaggcacgg gaaacttggt tccattgtta 720
   tattgtccta tgcttcgagc catgggtcta cagggtcatc cttatgagac tcttaaatat 780
   acttagatcc tggtaagagg caaagaatca acagccaaac tgctggggct gcaagctgct 840
   gaagccaggg catgggatta aagagattgt gcgttcaaac ctagggaagc ctgtgcccat 900
   ttgtcctgac tgtctgctaa catggtacac tgcatctcaa gatgtttatc tgacacaagt 960
   gtattatttc tggctttttg aattaatcta gaaaatgaaa agatggagtt gtattttgac 1020
   aaaaatgttt gtacttttta atgttatttg gaattttaag ttctatcagt gacttctgaa 1080
   tccttagaat ggcctctttg tagaaccctg tggtatagag gagtatggcc actgccccac 1140
   tatttttatt ttcttatgta agtttgcata tcagtcatga ctagtgccta gaaagcaatg 1200
   tgatggtcag gatctcatga cattatattt gagtttcttt cagatcattt aggatactct 1260
   taatctcact tcatcaatca aatatttttt gagtgtatgc tgtagctgaa agagtatgta 1320
   cgtacgtata agactagaga gatattaagt ctcagtacac ttcctgtgcc atgttattca 1380
   gctcactggt ttacaaatat aggttgtctt gtggttgtag gagcccactg taacaatatt 1440
   gggcagcctt tttttttttt ttttaattgc aacaatgcaa aagccaagaa agtataaggg 1500
   tcacaagttt aaacaatgaa ttcttcaaca gggaaaacag ctagcttgaa aacttgctga 1560
   aaaacacaac ttgtgtttat ggcatttagt accttcaaat aattggcttt gcagatattg 1620
   gataccccat taaatctgac agtctcaaat ttttcatctc ttcaatcact agtcaagaaa 1680
   aatataaaaa caacaaatac ttccatatgg agcatttttc agagttttct aacccagtct 1740
   tatttttcta gtcagtaaac atttgtaaaa atactgtttc actaatactt actgttaact 1800
   gtcttgagag aaaagaaaaa tatgagagaa ctattgtttg gggaagttca agtgatcttt 1860
   caatatcatt actaacttct tccacttttt ccaaaatttg aatattaacg ctaaaggtgt 1920
   aagacttcag atttcaaatt aatctttcta tattttttaa atttacagaa tattatataa 1980
   cccactgctg aaaaagaaaa aaatgattgt tttagaagtt aaagtcaata ttgattttaa 2040
   atataagtaa tgaaggcata tttccaataa ctagtgatat ggcatcgttg caatttacag 2100
   tatcttcaaa aatacagaat ttatagaata atttctcctc atttaatatt tttcaaaatc 2160
   aaagttatgg tttcctcatt ttactaaaat cgtattctaa ttcttcatta tagtaaatct 2220
   atgagcaact ccttacttcg gttcctctga tttcaaggcc atattttaaa aaatcaaaag 2280
   gcactgtgaa ctattttgaa gaaaacacga cattttaata cagattgaaa ggacctcttc 2340
   tgaagctaga aacaatctat agttatacat cttcattaat actgtgttac cttttaaaat 2400
   agtaattttt tacattttcc tgtgtaaacc taattgtggt agaaattttt accaactcta 2460
   tactcaatca agcaaaattt ctgtatattc cctgtggaat gtacctatgt gagtttcaga 2520
   aattctcaaa atacgtgttc aaaaatttct gcttttgcat ctttgggaca cctcagaaaa 2580
   cttattaaca actgtgaata tgagaaatac agaagaaaat aataagccct ctatacataa 2640
   atgcccagca caattcattg ttaaaaaaca accaaacctc acactactgt atttcattat 2700
   ctgtactgaa agcaaatgct ttgtgactat taaatgttgc acatcattca ttcactgtat 2760
   agtaatcatt gactaaagcc atttgctgtg ttttcttctt gtggntgnat atatcaggta 2820
   aaatattttc caaagagcca tgtgtcatgt aatactgaac cctttgatat tgagacatta 2880
   atttggaccc ttggtattat ctactagaat aatgtaatac tgnagaaata ttgctctaat 2940
   tctttcaaaa tggtgcatcc cccttaaaan gttctatttc cataaggatt tagcttgctt 3000
   atcccttctt ataccctaag atgaagctgt ttttgtgctc tttgttcatc attggccctc 3060
   attccaagca ctttacgctg tctgtaatgg gatctatttt tgcactggaa tatctgagaa 3120
   ttgcaaaact agacaaaagt ttcacaacag atttctaagt taaatcattt tcattaaaag 3180
   gaaaaaagaa aaaaaatttt gtatgtcaat aactttatat gaagtattaa aatgcatatt 3240
   tctatgttgt aatataatga gtcacaaaat aaagctgtga cagttctgtt aaaaaaaaaa 3300
   aaaaaaaaaa aaaaaaaaaa aaaaaaaaaa aaaaaaaaaa aaaaaaaaaa aaaaaaaaaa 3360
   aaaaaa 3366
<210> 7
   <211> 10474
   <212> DNA
   <213> Homo sapiens
<400> 7
<210> 8
   <211> 2261
   <212> PRT
   <213> Homo sapiens
<400> 8
   Met Ala Cys Trp Pro Gln Leu Arg Leu Leu Leu Trp Lys Asn Leu Thr
   1 5 10 15
   Phe Arg Arg Arg Gln Thr Cys Gln Leu Leu Leu Glu Val Ala Trp Pro
   20 25 30
   Leu Phe Ile Phe Leu Ile Leu Ile Ser Val Arg Leu Ser Tyr Pro Pro
   35 40 45
   Tyr Glu Gln His Glu Cys His Phe Pro Asn Lys Ala Met Pro Ser Ala
   50 55 60
   Gly Thr Leu Pro Trp Val Gln Gly Ile Ile Cys Asn Ala Asn Asn Pro
   65 70 75 80
   Cys Phe Arg Tyr Pro Thr Pro Gly Glu Ala Pro Gly Val Val Gly Asn
   85 90 95
   Phe Asn Lys Ser Ile Val Ala Arg Leu Phe Ser Asp Ala Arg Arg Leu
   100 105 110
   Leu Leu Tyr Ser Gln Lys Asp Thr Ser Met Lys Asp Met Arg Lys Val
   115 120 125
   Leu Arg Thr Leu Gln Gln Ile Lys Lys Ser Ser Ser Asn Leu Lys Leu
   130 135 140
   Gln Asp Phe Leu Val Asp Asn Glu Thr Phe Ser Gly Phe Leu Tyr His
   145 150 155 160
   Asn Leu Ser Leu Pro Lys Ser Thr Val Asp Lys Met Leu Arg Ala Asp
   165 170 175
   Val Ile Leu His Lys Val Phe Leu Gln Gly Tyr Gln Leu His Leu Thr
   180 185 190
   Ser Leu Cys Asn Gly Ser Lys Ser Glu Glu Met Ile Gln Leu Gly Asp
   195 200 205
   Gln Glu Val Ser Glu Leu Cys Gly Leu Pro Lys Glu Lys Leu Ala Ala
   210 215 220
   Ala Glu Arg Val Leu Arg Ser Asn Met Asp Ile Leu Lys Pro Ile Leu
   225 230 235 240
   Arg Thr Leu Asn Ser Thr Ser Pro Phe Pro Ser Lys Glu Leu Ala Glu
   245 250 255
   Ala Thr Lys Thr Leu Leu His Ser Leu Gly Thr Leu Ala Gln Glu Leu
   260 265 270
   Phe Ser Met Arg Ser Trp Ser Asp Met Arg Gln Glu Val Met Phe Leu
   275 280 285
   Thr Asn Val Asn Ser Ser Ser Ser Ser Thr Gln Ile Tyr Gln Ala Val
   290 295 300
   Ser Arg Ile Val Cys Gly His Pro Glu Gly Gly Gly Leu Lys Ile Lys
   305 310 315 320
   Ser Leu Asn Trp Tyr Glu Asp Asn Asn Tyr Lys Ala Leu Phe Gly Gly
   325 330 335
   Asn Gly Thr Glu Glu Asp Ala Glu Thr Phe Tyr Asp Asn Ser Thr Thr
   340 345 350
   Pro Tyr Cys Asn Asp Leu Met Lys Asn Leu Glu Ser Ser Pro Leu Ser
   355 360 365
   Arg Ile Ile Trp Lys Ala Leu Lys Pro Leu Leu Val Gly Lys Ile Leu
   370 375 380
   Tyr Thr Pro Asp Thr Pro Ala Thr Arg Gln Val Met Ala Glu Val Asn
   385 390 395 400
   Lys Thr Phe Gln Glu Leu Ala Val Phe His Asp Leu Glu Gly Met Trp
   405 410 415
   Glu Glu Leu Ser Pro Lys Ile Trp Thr Phe Met Glu Asn Ser Gln Glu
   420 425 430
   Met Asp Leu Val Arg Met Leu Leu Asp Ser Arg Asp Asn Asp His Phe
   435 440 445
   Trp Glu Gln Gln Leu Asp Gly Leu Asp Trp Thr Ala Gln Asp Ile Val
   450 455 460
   Ala Phe Leu Ala Lys His Pro Glu Asp Val Gln Ser Ser Asn Gly Ser
   465 470 475 480
   Val Tyr Thr Trp Arg Glu Ala Phe Asn Glu Thr Asn Gln Ala Ile Arg
   485 490 495
   Thr Ile Ser Arg Phe Met Glu Cys Val Asn Leu Asn Lys Leu Glu Pro
   500 505 510
   Ile Ala Thr Glu Val Trp Leu Ile Asn Lys Ser Met Glu Leu Leu Asp
   515 520 525
   Glu Arg Lys Phe Trp Ala Gly Ile Val Phe Thr Gly Ile Thr Pro Gly
   530 535 540
   Ser Ile Glu Leu Pro His His Val Lys Tyr Lys Ile Arg Met Asp Ile
   545 550 555 560
   Asp Asn Val Glu Arg Thr Asn Lys Ile Lys Asp Gly Tyr Trp Asp Pro
   565 570 575
   Gly Pro Arg Ala Asp Pro Phe Glu Asp Met Arg Tyr Val Trp Gly Gly
   580 585 590
   Phe Ala Tyr Leu Arg Asp Val Val Glu Gln Ala Ile Ile Arg Val Leu
   595 600 605
   Thr Gly Thr Glu Lys Lys Thr Gly Val Tyr Met Gln Gln Met Pro Tyr
   610 615 620
   Pro Cys Tyr Val Asp Asp Ile Phe Leu Arg Val Met Ser Arg Ser Met
   625 630 635 640
   Pro Leu Phe Met Thr Leu Ala Trp Ile Tyr Ser Val Ala Val Ile Ile
   645 650 655
   Lys Gly Ile Val Tyr Glu Lys Glu Ala Arg Leu Lys Glu Thr Met Arg
   660 665 670
   Ile Met Gly Leu Asp Asn Ser Ile Leu Trp Phe Ser Trp Phe Ile Ser
   675 680 685
   Ser Leu Ile Pro Leu Leu Val Ser Ala Gly Leu Leu Val Val Ile Leu
   690 695 700
   Lys Leu Gly Asn Leu Leu Pro Tyr Ser Asp Pro Ser Val Val Phe Val
   705 710 715 720
   Phe Leu Ser Val Phe Ala Val Val Thr Ile Leu Gln Cys Phe Leu Ile
   725 730 735
   Ser Thr Leu Phe Ser Arg Ala Asn Leu Ala Ala Ala Cys Gly Gly Ile
   740 745 750
   Ile Tyr Phe Thr Leu Tyr Leu Pro Tyr Val Leu Cys Val Ala Trp Gln
   755 760 765
   Asp Tyr Val Gly Phe Thr Leu Lys Ile Phe Ala Ser Leu Leu Ser Pro
   770 775 780
   Val Ala Phe Gly Phe Gly Cys Glu Tyr Phe Ala Leu Phe Glu Glu Gln
   785 790 795 800
   Gly Ile Gly Val Gln Trp Asp Asn Leu Phe Glu Ser Pro Val Glu Glu
   805 810 815
   Asp Gly Phe Asn Leu Thr Thr Ser Ile Ser Met Met Leu Phe Asp Thr
   820 825 830
   Phe Leu Tyr Gly Val Met Thr Trp Tyr Ile Glu Ala Val Phe Pro Gly
   835 840 845
   Gln Tyr Gly Ile Pro Arg Pro Trp Tyr Phe Pro Cys Thr Lys Ser Tyr
   850 855 860
   Trp Phe Gly Glu Glu Ser Asp Glu Lys Ser His Pro Gly Ser Asn Gln
   865 870 875 880
   Lys Arg Met Ser Glu Ile Cys Met Glu Glu Glu Pro Thr His Leu Lys
   885 890 895
   Leu Gly Val Ser Ile Gln Asn Leu Val Lys Val Tyr Arg Asp Gly Met
   900 905 910
   Lys Val Ala Val Asp Gly Leu Ala Leu Asn Phe Tyr Glu Gly Gln Ile
   915 920 925
   Thr Ser Phe Leu Gly His Asn Gly Ala Gly Lys Thr Thr Thr Met Ser
   930 935 940
   Ile Leu Thr Gly Leu Phe Pro Pro Thr Ser Gly Thr Ala Tyr Ile Leu
   945 950 955 960
   Gly Lys Asp Ile Arg Ser Glu Met Ser Thr Ile Arg Gln Asn Leu Gly
   965 970 975
   Val Cys Pro Gln His Asn Val Leu Phe Asp Met Leu Thr Val Glu Glu
   980 985 990
   His Ile Trp Phe Tyr Ala Arg Leu Lys Gly Leu Ser Glu Lys His Val
   995 1000 1005
   Lys Ala Glu Met Glu Gln Met Ala Leu Asp Val Gly Leu Pro Ser Ser
   1010 1015 1020
   Lys Leu Lys Ser Lys Thr Ser Gln Leu Ser Gly Gly Met Gln Arg Lys
   1025 1030 1035 1040
   Leu Ser Val Ala Leu Ala Phe Val Gly Gly Ser Lys Val Val Ile Leu
   1045 1050 1055
   Asp Glu Pro Thr Ala Gly Val Asp Pro Tyr Ser Arg Arg Gly Ile Trp
   1060 1065 1070
   Glu Leu Leu Leu Lys Tyr Arg Gln Gly Arg Thr Ile Ile Leu Ser Thr
   1075 1080 1085
   His His Met Asp Glu Ala Asp Val Leu Gly Asp Arg Ile Ala Ile Ile
   1090 1095 1100
   Ser His Gly Lys Leu Cys Cys Val Gly Ser Ser Leu Phe Leu Lys Asn
   1105 1110 1115 1120
   Gln Leu Gly Thr Gly Tyr Tyr Leu Thr Leu Val Lys Lys Asp Val Glu
   1125 1130 1135
   Ser Ser Leu Ser Ser Cys Arg Asn Ser Ser Ser Thr Val Ser Tyr Leu
   1140 1145 1150
   Lys Lys Glu Asp Ser Val Ser Gln Ser Ser Ser Asp Ala Gly Leu Gly
   1155 1160 1165
   Ser Asp His Glu Ser Asp Thr Leu Thr Ile Asp Val Ser Ala Ile Ser
   1170 1175 1180
   Asn Leu Ile Arg Lys His Val Ser Glu Ala Arg Leu Val Glu Asp Ile
   1185 1190 1195 1200
   Gly His Glu Leu Thr Tyr Val Leu Pro Tyr Glu Ala Ala Lys Glu Gly
   1205 1210 1215
   Ala Phe Val Glu Leu Phe His Glu Ile Asp Asp Arg Leu Ser Asp Leu
   1220 1225 1230
   Gly Ile Ser Ser Tyr Gly Ile Ser Glu Thr Thr Leu Glu Glu Ile Phe
   1235 1240 1245
   Leu Lys Val Ala Glu Glu Ser Gly Val Asp Ala Glu Thr Ser Asp Gly
   1250 1255 1260
   Thr Leu Pro Ala Arg Arg Asn Arg Arg Ala Phe Gly Asp Lys Gln Ser
   1265 1270 1275 1280
   Cys Leu Arg Pro Phe Thr Glu Asp Asp Ala Ala Asp Pro Asn Asp Ser
   1285 1290 1295
   Asp Ile Asp Pro Glu Ser Arg Glu Thr Asp Leu Leu Ser Gly Met Asp
   1300 1305 1310
   Gly Lys Gly Ser Tyr Gln Val Lys Gly Trp Lys Leu Thr Gln Gln Gln
   1315 1320 1325
   Phe Val Ala Leu Leu Trp Lys Arg Leu Leu Ile Ala Arg Arg Ser Arg
   1330 1335 1340
   Lys Gly Phe Phe Ala Gln Ile Val Leu Pro Ala Val Phe Val Cys Ile
   1345 1350 1355 1360
   Ala Leu Val Phe Ser Leu Ile Val Pro Pro Phe Gly Lys Tyr Pro Ser
   1365 1370 1375
   Leu Glu Leu Gln Pro Trp Met Tyr Asn Glu Gln Tyr Thr Phe Val Ser
   1380 1385 1390
   Asn Asp Ala Pro Glu Asp Thr Gly Thr Leu Glu Leu Leu Asn Ala Leu
   1395 1400 1405
   Thr Lys Asp Pro Gly Phe Gly Thr Arg Cys Met Glu Gly Asn Pro Ile
   1410 1415 1420
   Pro Asp Thr Pro Cys Gln Ala Gly Glu Glu Glu Trp Thr Thr Ala Pro
   1425 1430 1435 1440
   Val Pro Gln Thr Ile Met Asp Leu Phe Gln Asn Gly Asn Trp Thr Met
   1445 1450 1455
   Gln Asn Pro Ser Pro Ala Cys Gln Cys Ser Ser Asp Lys Ile Lys Lys
   1460 1465 1470
   Met Leu Pro Val Cys Pro Pro Gly Ala Gly Gly Leu Pro Pro Pro Gln
   1475 1480 1485
   Arg Lys Gln Asn Thr Ala Asp Ile Leu Gln Asp Leu Thr Gly Arg Asn
   1490 1495 1500
   Ile Ser Asp Tyr Leu Val Lys Thr Tyr Val Gln Ile Ile Ala Lys Ser
   1505 1510 1515 1520
   Leu Lys Asn Lys Ile Trp Val Asn Glu Phe Arg Tyr Gly Gly Phe Ser
   1525 1530 1535
   Leu Gly Val Ser Asn Thr Gln Ala Leu Pro Pro Ser Gln Glu Val Asn
   1540 1545 1550
   Asp Ala Ile Lys Gln Met Lys Lys His Leu Lys Leu Ala Lys Asp Ser
   1555 1560 1565
   Ser Ala Asp Arg Phe Leu Asn Ser Leu Gly Arg Phe Met Thr Gly Leu
   1570 1575 1580
   Asp Thr Arg Asn Asn Val Lys Val Trp Phe Asn Asn Lys Gly Trp His
   1585 1590 1595 1600
   Ala Ile Ser Ser Phe Leu Asn Val Ile Asn Asn Ala Ile Leu Arg Ala
   1605 1610 1615
   Asn Leu Gln Lys Gly Glu Asn Pro Ser His Tyr Gly Ile Thr Ala Phe
   1620 1625 1630
   Asn His Pro Leu Asn Leu Thr Lys Gln Gln Leu Ser Glu Val Ala Leu
   1635 1640 1645
   Met Thr Thr Ser Val Asp Val Leu Val Ser Ile Cys Val Ile Phe Ala
   1650 1655 1660
   Met Ser Phe Val Pro Ala Ser Phe Val Val Phe Leu Ile Gln Glu Arg
   1665 1670 1675 1680
   Val Ser Lys Ala Lys His Leu Gln Phe Ile Ser Gly Val Lys Pro Val
   1685 1690 1695
   Ile Tyr Trp Leu Ser Asn Phe Val Trp Asp Met Cys Asn Tyr Val Val
   1700 1705 1710
   Pro Ala Thr Leu Val Ile Ile Ile Phe Ile Cys Phe Gln Gln Lys Ser
   1715 1720 1725
   Tyr Val Ser Ser Thr Asn Leu Pro Val Leu Ala Leu Leu Leu Leu Leu
   1730 1735 1740
   Tyr Gly Trp Ser Ile Thr Pro Leu Met Tyr Pro Ala Ser Phe Val Phe
   1745 1750 1755 1760
   Lys Ile Pro Ser Thr Ala Tyr Val Val Leu Thr Ser Val Asn Leu Phe
   1765 1770 1775
   Ile Gly Ile Asn Gly Ser Val Ala Thr Phe Val Leu Glu Leu Phe Thr
   1780 1785 1790
   Asp Asn Lys Leu Asn Asn Ile Asn Asp Ile Leu Lys Ser Val Phe Leu
   1795 1800 1805
   Ile Phe Pro His Phe Cys Leu Gly Arg Gly Leu Ile Asp Met Val Lys
   1810 1815 1820
   Asn Gln Ala Met Ala Asp Ala Leu Glu Arg Phe Gly Glu Asn Arg Phe
   1825 1830 1835 1840
   Val Ser Pro Leu Ser Trp Asp Leu Val Gly Arg Asn Leu Phe Ala Met
   1845 1850 1855
   Ala Val Glu Gly Val Val Phe Phe Leu Ile Thr Val Leu Ile Gln Tyr
   1860 1865 1870
   Arg Phe Phe Ile Arg Pro Arg Pro Val Asn Ala Lys Leu Ser Pro Leu
   1875 1880 1885
   Asn Asp Glu Asp Glu Asp Val Arg Arg Glu Arg Gln Arg Ile Leu Asp
   1890 1895 1900
   Gly Gly Gly Gln Asn Asp Ile Leu Glu Ile Lys Glu Leu Thr Lys Ile
   1905 1910 1915 1920
   Tyr Arg Arg Lys Arg Lys Pro Ala Val Asp Arg Ile Cys Val Gly Ile
   1925 1930 1935
   Pro Pro Gly Glu Cys Phe Gly Leu Leu Gly Val Asn Gly Ala Gly Lys
   1940 1945 1950
   Ser Ser Thr Phe Lys Met Leu Thr Gly Asp Thr Thr Val Thr Arg Gly
   1955 1960 1965
   Asp Ala Phe Leu Asn Lys Asn Ser Ile Leu Ser Asn Ile His Glu Val
   1970 1975 1980
   His Gln Asn Met Gly Tyr Cys Pro Gln Phe Asp Ala Ile Thr Glu Leu
   1985 1990 1995 2000
   Leu Thr Gly Arg Glu His Val Glu Phe Phe Ala Leu Leu Arg Gly Val
   2005 2010 2015
   Pro Glu Lys Glu Val Gly Lys Val Gly Glu Trp Ala Ile Arg Lys Leu
   2020 2025 2030
   Gly Leu Val Lys Tyr Gly Glu Lys Tyr Ala Gly Asn Tyr Ser Gly Gly
   2035 2040 2045
   Asn Lys Arg Lys Leu Ser Thr Ala Met Ala Leu Ile Gly Gly Pro Pro
   2050 2055 2060
   Val Val Phe Leu Asp Glu Pro Thr Thr Gly Met Asp Pro Lys Ala Arg
   2065 2070 2075 2080
   Arg Phe Leu Trp Asn Cys Ala Leu Ser Val Val Lys Glu Gly Arg Ser
   2085 2090 2095
   Val Val Leu Thr Ser His Ser Met Glu Glu Cys Glu Ala Leu Cys Thr
   2100 2105 2110
   Arg Met Ala Ile Met Val Asn Gly Arg Phe Arg Cys Leu Gly Ser Val
   2115 2120 2125
   Gln His Leu Lys Asn Arg Phe Gly Asp Gly Tyr Thr Ile Val Val Arg
   2130 2135 2140
   Ile Ala Gly Ser Asn Pro Asp Leu Lys Pro Val Gln Asp Phe Phe Gly
   2145 2150 2155 2160
   Leu Ala Phe Pro Gly Ser Val Leu Lys Glu Lys His Arg Asn Met Leu
   2165 2170 2175
   Gln Tyr Gln Leu Pro Ser Ser Leu Ser Ser Leu Ala Arg Ile Phe Ser
   2180 2185 2190
   Ile Leu Ser Gln Ser Lys Lys Arg Leu His Ile Glu Asp Tyr Ser Val
   2195 2200 2205
   Ser Gln Thr Thr Leu Asp Gln Val Phe Val Asn Phe Ala Lys Asp Gln
   2210 2215 2220
   Ser Asp Asp Asp His Leu Lys Asp Leu Ser Leu His Lys Asn Gln Thr
   2225 2230 2235 2240
   Val Val Asp Val Ala Val Leu Thr Ser Phe Leu Gln Asp Glu Lys Val
   2245 2250 2255
   Lys Glu Ser Tyr Val
   2260
<210> 9
   <211> 10474
   <212> DNA
   <213> Homo sapiens
<400> 9
   tctagaactg ggtaccagct gctagcaagc ttcggcacga gccgcagagc cgagccgacc 60
   cttctctccc gggctgcggc agggcagggc ggggagctcc gcgcaccaac agagccggtt 120
   ctcagggcgc tttgctcctt gtttttcccc cggttctgtt ttctcccctt ctccggaagg 180
   cttgtcaagg ggtaggagaa agagacgcaa acacaaaagt ggaaaacagt taatgaccag 240
   ccacgggcgt ccctgctgtg agctctggcc gctgccttcc agggctcccg agccacacgc 300
   tgggcgtgct ggctgaggga acatggcttg ttggcctcag ctgaggttgc tgctgtggaa 360
   gaacctcact ttcagaagaa gacaaacatg tcagctgtta ctggaagtgg cctggcctct 420
   atttatcttc ctgatcctga tctctgttcg gctgagctac ccaccctatg aacaacatga 480
   atgccatttt ccaaataaag ccatgccctc tgcaggaaca cttccttggg ttcaggggat 540
   tatctgtaat gccaacaacc cctgtttccg ttacccgact cctggggagg ctcccggagt 600
   tgttggaaac tttaacaaat ccattgtggc tcgcctgttc tcagatgctc ggaggcttct 660
   tttatacagc cagaaagaca ccagcatgaa ggacatgcgc aaagttctga gaacattaca 720
   gcagatcaag aaatccagct caaacttgaa gcttcaagat ttcctggtgg acaatgaaac 780
   cttctctggg ttcctatatc acaacctctc tctcccaaag tctactgtgg acaagatgct 840
   gagggctgat gtcattctcc acaaggtatt tttgcaaggc taccagttac atttgacaag 900
   tctgtgcaat ggatcaaaat cagaagagat gattcaactt ggtgaccaag aagtttctga 960
   gctttgtggc ctaccaaagg agaaactggc tgcagcagag cgagtacttc gttccaacat 1020
   ggacatcctg aagccaatcc tgagaacact aaactctaca tctcccttcc cgagcaagga 1080
   gctggctgaa gccacaaaaa cattgctgca tagtcttggg actctggccc aggagctgtt 1140
   cagcatgaga agctggagtg acatgcgaca ggaggtgatg tttctgacca atgtgaacag 1200
   ctccagctcc tccacccaaa tctaccaggc tgtgtctcgt attgtctgcg ggcatcccga 1260
   gggagggggg ctgaagatca agtctctcaa ctggtatgag gacaacaact acaaagccct 1320
   ctttggaggc aatggcactg aggaagatgc tgaaaccttc tatgacaact ctacaactcc 1380
   ttactgcaat gatttgatga agaatttgga gtctagtcct ctttcccgca ttatctggaa 1440
   agctctgaag ccgctgctcg ttgggaagat cctgtataca cctgacactc cagccacaag 1500
   gcaggtcatg gctgaggtga acaagacctt ccaggaactg gctgtgttcc atgatctgga 1560
   aggcatgtgg gaggaactca gccccaagat ctggaccttc atggagaaca gccaagaaat 1620
   ggaccttgtc cggatgctgt tggacagcag ggacaatgac cacttttggg aacagcagtt 1680
   ggatggctta gattggacag cccaagacat cgtggcgttt ttggccaagc acccagagga 1740
   tgtccagtcc agtaatggtt ctgtgtacac ctggagagaa gctttcaacg agactaacca 1800
   ggcaatccgg accatatctc gcttcatgga gtgtgtcaac ctgaacaagc tagaacccat 1860
   agcaacagaa gtctggctca tcaacaagtc catggagctg ctggatgaga ggaagttctg 1920
   ggctggtatt gtgttcactg gaattactcc aggcagcatt gagctgcccc atcatgtcaa 1980
   gtacaagatc cgaatggaca ttgacaatgt ggagaggaca aataaaatca aggatgggta 2040
   ctgggaccct ggtcctcgag ctgacccctt tgaggacatg tggtacgtct gggggggctt 2100
   cgcctacttg caggatgtgg tggagcaggc aatcatcagg gtgctgacgg gcaccgagaa 2160
   gaaaactggt gtctatatgc aacagatgcc ctatccctgt tacgttgatg acatctttct 2220
   gcgggtgatg agccggtcaa tgcccctctt catgacgctg gcctggattt actcagtggc 2280
   tgtgatcatc aagggcatcg tgtatgagaa ggaggcacgg ctgaaagaga ccatgcggat 2340
   catgggcctg gacaacagca tactctggtt tagctggttc attagtagcc tcattcctct 2400
   tcttgtgagc gctggcctgc tagtggtcat cctgaagtta ggaaacctgc tgccctacag 2460
   tgatcccagc gtggtgtttg tcttcctgtc cgtgtttgct gtggtgacaa tcctgcagtg 2520
   cttcctgatt agcacactct tctccagagc caacctggca gcagcctgtg ggggcatcat 2580
   ctacttcacg ctgtacctgc cctacgtcct gtgtgtggca tggcaggact acgtgggctt 2640
   cacactcaag atcttcgcta gcctgctgtc tcctgtggct tttgggtttg gctgtgagta 2700
   ctttgccctt tttgaggagc agggcattgg agtgcagtgg gacaacctgt ttgagagtcc 2760
   tgtggaggaa gatggcttca atctcaccac ttcgatctcc atgatgctgt ttgacacctt 2820
   cctctatggg gtgatgacct ggtacattga ggctgtcttt ccaggccagt acggaattcc 2880
   caggccctgg tattttcctt gcaccaagtc ctactggttt ggcgaggaaa gtgatgagaa 2940
   gagccaccct ggttccaacc agaagagaat gtcagaaatc tgcatggagg aggaacccac 3000
   ccacttgaag ctgggcgtgt ccattcagaa cctggtaaaa gtctaccgag atgggatgaa 3060
   ggtggctgtc gatggcctgg cactgaattt ttatgagggc cagatcacct ccttcctggg 3120
   ccacaatgga gcggggaaga cgaccaccat gtcaatcctg accgggttgt tccccccgac 3180
   ctcgggcacc gcctacatcc tgggaaaaga cattcgctct gagatgagca ccatccggca 3240
   gaacctgggg gtctgtcccc agcataacgt gctgtttgac atgctgactg tcgaagaaca 3300
   catctggttc tatgcccgct tgaaagggct ctctgagaag cacgtgaagg cggagatgga 3360
   gcagatggcc ctggatgttg gtttgccatc aagcaagctg aaaagcaaaa caagccagct 3420
   gtcaggtgga atgcagagaa agctatctgt ggccttggcc tttgtcgggg gatctaaggt 3480
   tgtcattctg gatgaaccca cagctggtgt ggacccttac tcccgcaggg gaatatggga 3540
   gctgctgctg aaataccgac aaggccgcac cattattctc tctacacacc acatggatga 3600
   agcggacgtc ctgggggaca ggattgccat catctcccat gggaagctgt gctgtgtggg 3660
   ctcctccctg tttctgaaga accagctggg aacaggctac tacctgacct tggtcaagaa 3720
   agatgtggaa tcctccctca gttcctgcag aaacagtagt agcactgtgt catacctgaa 3780
   aaaggaggac agtgtttctc agagcagttc tgatgctggc ctgggcagcg accatgagag 3840
   tgacacgctg accatcgatg tctctgctat ctccaacctc atcaggaagc atgtgtctga 3900
   agcccggctg gtggaagaca tagggcatga gctgacctat gtgctgccat atgaagctgc 3960
   taaggaggga gcctttgtgg aactctttca tgagattgat gaccggctct cagacctggg 4020
   catttctagt tatggcatct cagagacgac cctggaagaa atattcctca aggtggccga 4080
   agagagtggg gtggatgctg agacctcaga tggtaccttg ccagcaagac gaaacaggcg 4140
   ggccttcggg gacaagcaga gctgtcttcg cccgttcact gaagatgatg ctgctgatcc 4200
   aaatgattct gacatagacc cagaatccag agagacagac ttgctcagtg ggatggatgg 4260
   caaagggtcc taccaggtga aaggctggaa acttacacag caacagtttg tggccctttt 4320
   gtggaagaga ctgctaattg ccagacggag tcggaaagga ttttttgctc agattgtctt 4380
   gccagctgtg tttgtctgca ttgcccttgt gttcagcctg atcgtgccac cctttggcaa 4440
   gtaccccagc ctggaacttc agccctggat gtacaacgaa cagtacacat ttgtcagcaa 4500
   tgatgctcct gaggacacgg gaaccctgga actcttaaac gccctcacca aagaccctgg 4560
   cttcgggacc cgctgtatgg aaggaaaccc aatcccagac acgccctgcc aggcagggga 4620
   ggaagagtgg accactgccc cagttcccca gaccatcatg gacctcttcc agaatgggaa 4680
   ctggacaatg cagaaccctt cacctgcatg ccagtgtagc agcgacaaaa tcaagaagat 4740
   gctgcctgtg tgtcccccag ggcagggggg gctgcctcct ccacaaagaa aacaaaacac 4800
   tgcagatatc cttcaggacc tgacaggaag aaacatttcg gattatctgg tgaagacgta 4860
   tgtgcagatc atagccaaaa gcttaaagaa caagatctgg gtgaatgagt ttaggtatgg 4920
   cggcttttcc ctgggtgtca gtaatactca agcacttcct ccgagtcaag aagttaatga 4980
   tgccatcaaa caaatgaaga aacacctaaa gctggccaag gacagttctg cagatcgatt 5040
   tctcaacagc ttgggaagat ttatgacagg actggacacc agaaataatg tcaaggtgtg 5100
   gttcaataac aagggctggc atgcaatcag ctctttcctg aatgtcatca acaatgccat 5160
   tctccgggcc aacctgcaaa agggagagaa ccctagccat tatggaatta ctgctttcaa 5220
   tcatcccctg aatctcacca agcagcagct ctcagaggtg gctctgatga ccacatcagt 5280
   ggatgtcctt gtgtccatct gtgtcatctt tgcaatgtcc ttcgtcccag ccagctttgt 5340
   cgtattcctg atccaggagc gggtcagcaa agcaaaacac ctgcagttca tcagtggagt 5400
   gaagcctgtc atctactggc tctctaattt tgtctgggat atgtgcaatt acgttgtccc 5460
   tgccacactg gtcattatca tcttcatctg cttccagcag aagtcctatg tgtcctccac 5520
   caatctgcct gtgctagccc ttctactttt gctgtatggg tggtcaatca cacctctcat 5580
   gtacccagcc tcctttgtgt tcaagatccc cagcacagcc tatgtggtgc tcaccagcgt 5640
   gaacctcttc attggcatta atggcagcgt ggccaccttt gtgctggagc tgttcaccga 5700
   caataagctg aataatatca atgatatcct gaagtccgtg ttcttgatct tcccacattt 5760
   ttgcctggga cgagggctca tcgacatggt gaaaaaccag gcaatggctg atgccctgga 5820
   aaggtttggg gagaatcgct ttgtgtcacc attatcttgg gacttggtgg gacgaaacct 5880
   cttcgccatg gccgtggaag gggtggtgtt cttcctcatt actgttctga tccagtacag 5940
   attcttcatc aggcccagac ctgtaaatgc aaagctatct cctctgaatg atgaagatga 6000
   agatgtgagg cgggaaagac agagaattct tgatggtgga ggccagaatg acatcttaga 6060
   aatcaaggag ttgacgaaga tatatagaag gaagcggaag cctgctgttg acaggatttg 6120
   cgtgggcatt cctcctggtg agtgctttgg gctcctggga gttaatgggg ctggaaaatc 6180
   atcaactttc aagatgttaa caggagatac cactgttacc agaggagatg ctttccttaa 6240
   caaaaatagt atcttatcaa acatccatga agtacatcag aacatgggct actgccctca 6300
   gtttgatgcc atcacagagc tgttgactgg gagagaacac gtggagttct ttgccctttt 6360
   gagaggagtc ccagagaaag aagttggcaa ggttggtgag tgggcgattc ggaaactggg 6420
   cctcgtgaag tatggagaaa aatatgctgg taactatagt ggaggcaaca aacgcaagct 6480
   ctctacagcc atggctttga tcggcgggcc tcctgtggtg tttctggatg aacccaccac 6540
   aggcatggat cccaaagccc ggcggttctt gtggaattgt gccctaagtg ttgtcaagga 6600
   ggggagatca gtagtgctta catctcatag tatggaagaa tgtgaagctc tttgcactag 6660
   gatggcaatc atggtcaatg gaaggttcag gtgccttggc agtgtccagc atctaaaaaa 6720
   taggtttgga gatggttata caatagttgt acgaatagca gggtccaacc cggacctgaa 6780
   gcctgtccag gatttctttg gacttgcatt tcctggaagt gttctaaaag agaaacaccg 6840
   gaacatgcta caataccagc ttccatcttc attatcttct ctggccagga tattcagcat 6900
   cctctcccag agcaaaaagc gactccacat agaagactac tctgtttctc agacaacact 6960
   tgaccaagta tttgtgaact ttgccaagga ccaaagtgat gatgaccact taaaagacct 7020
   ctcattacac aaaaaccaga cagtagtgga cgttgcagtt ctcacatctt ttctacagga 7080
   tgagaaagtg aaagaaagct atgtatgaag aatcctgttc atacggggtg gctgaaagta 7140
   aagaggaact agactttcct ttgcaccatg tgaagtgttg tggagaaaag agccagaagt 7200
   tgatgtggga agaagtaaac tggatactgt actgatacta ttcaatgcaa tgcaattcaa 7260
   tgcaatgaaa acaaaattcc attacagggg cagtgccttt gtagcctatg tcttgtatgg 7320
   ctctcaagtg aaagacttga atttagtttt ttacctatac ctatgtgaaa ctctattatg 7380
   gaacccaatg gacatatggg tttgaactca cacttttttt ttttttttgt tcctgtgtat 7440
   tctcattggg gttgcaacaa taattcatca agtaatcatg gccagcgatt attgatcaaa 7500
   atcaaaaggt aatgcacatc ctcattcact aagccatgcc atgcccagga gactggtttc 7560
   ccggtgacac atccattgct ggcaatgagt gtgccagagt tattagtgcc aagtttttca 7620
   gaaagtttga agcaccatgg tgtgtcatgc tcacttttgt gaaagctgct ctgctcagag 7680
   tctatcaaca ttgaatatca gttgacagaa tggtgccatg cgtggctaac atcctgcttt 7740
   gattccctct gataagctgt tctggtggca gtaacatgca acaaaaatgt gggtgtctct 7800
   aggcacggga aacttggttc cattgttata ttgtcctatg cttcgagcca tgggtctaca 7860
   gggtcatcct tatgagactc ttaaatatac ttagatcctg gtaagaggca aagaatcaac 7920
   agccaaactg ctggggctgc aagctgctga agccagggca tgggattaaa gagattgtgc 7980
   gttcaaacct agggaagcct gtgcccattt gtcctgactg tctgctaaca tggtacactg 8040
   catctcaaga tgtttatctg acacaagtgt attatttctg gctttttgaa ttaatctaga 8100
   aaatgaaaag atggagttgt attttgacaa aaatgtttgt actttttaat gttatttgga 8160
   attttaagtt ctatcagtga cttctgaatc cttagaatgg cctctttgta gaaccctgtg 8220
   gtatagagga gtatggccac tgccccacta tttttatttt cttatgtaag tttgcatatc 8280
   agtcatgact agtgcctaga aagcaatgtg atggtcagga tctcatgaca ttatatttga 8340
   gtttctttca gatcatttag gatactctta atctcacttc atcaatcaaa tattttttga 8400
   gtgtatgctg tagctgaaag agtatgtacg tacgtataag actagagaga tattaagtct 8460
   cagtacactt cctgtgccat gttattcagc tcactggttt acaaatatag gttgtcttgt 8520
   ggttgtagga gcccactgta acaatattgg gcagcctttt tttttttttt ttaattgcaa 8580
   caatgcaaaa gccaagaaag tataagggtc acaagtttaa acaatgaatt cttcaacagg 8640
   gaaaacagct agcttgaaaa cttgctgaaa aacacaactt gtgtttatgg catttagtac 8700
   cttcaaataa ttggctttgc agatattgga taccccatta aatctgacag tctcaaattt 8760
   ttcatctctt caatcactag tcaagaaaaa tataaaaaca acaaatactt ccatatggag 8820
   catttttcag agttttctaa cccagtctta tttttctagt cagtaaacat ttgtaaaaat 8880
   actgtttcac taatacttac tgttaactgt cttgagagaa aagaaaaata tgagagaact 8940
   attgtttggg gaagttcaag tgatctttca atatcattac taacttcttc cactttttcc 9000
   aaaatttgaa tattaacgct aaaggtgtaa gacttcagat ttcaaattaa tctttctata 9060
   ttttttaaat ttacagaata ttatataacc cactgctgaa aaagaaaaaa atgattgttt 9120
   tagaagttaa agtcaatatt gattttaaat ataagtaatg aaggcatatt tccaataact 9180
   agtgatatgg catcgttgca atttacagta tcttcaaaaa tacagaattt atagaataat 9240
   ttctcctcat ttaatatttt tcaaaatcaa agttatggtt tcctcatttt actaaaatcg 9300
   tattctaatt cttcattata gtaaatctat gagcaactcc ttacttcggt tcctctgatt 9360
   tcaaggccat attttaaaaa atcaaaaggc actgtgaact attttgaaga aaacacgaca 9420
   ttttaataca gattgaaagg acctcttctg aagctagaaa caatctatag ttatacatct 9480
   tcattaatac tgtgttacct tttaaaatag taatttttta cattttcctg tgtaaaccta 9540
   attgtggtag aaatttttac caactctata ctcaatcaag caaaatttct gtatattccc 9600
   tgtggaatgt acctatgtga gtttcagaaa ttctcaaaat acgtgttcaa aaatttctgc 9660
   ttttgcatct ttgggacacc tcagaaaact tattaacaac tgtgaatatg agaaatacag 9720
   aagaaaataa taagccctct atacataaat gcccagcaca attcattgtt aaaaaacaac 9780
   caaacctcac actactgtat ttcattatct gtactgaaag caaatgcttt gtgactatta 9840
   aatgttgcac atcattcatt cactgtatag taatcattga ctaaagccat ttgctgtgtt 9900
   ttcttcttgt ggntgnatat atcaggtaaa atattttcca aagagccatg tgtcatgtaa 9960
   tactgaaccc tttgatattg agacattaat ttggaccctt ggtattatct actagaataa 10020
   tgtaatactg nagaaatatt gctctaattc tttcaaaatg gtgcatcccc cttaaaangt 10080
   tctatttcca taaggattta gcttgcttat cccttcttat accctaagat gaagctgttt 10140
   ttgtgctctt tgttcatcat tggccctcat tccaagcact ttacgctgtc tgtaatggga 10200
   tctatttttg cactggaata tctgagaatt gcaaaactag acaaaagttt cacaacagat 10260
   ttctaagtta aatcattttc attaaaagga aaaaagaaaa aaaattttgt atgtcaataa 10320
   ctttatatga agtattaaaa tgcatatttc tatgttgtaa tataatgagt cacaaaataa 10380
   agctgtgaca gttctgttaa aaaaaaaaaa aaaaaaaaaa aaaaaaaaaa aaaaaaaaaa 10440
   aaaaaaaaaa aaaaaaaaaa aaaaaaaaaa aaaa                             10474
<210> 10
   <211> 2261
   <212> PRT
   <213> Homo sapiens
<400> 10
   Met Ala Cys Trp Pro Gln Leu Arg Leu Leu Leu Trp Lys Asn Leu Thr
   1               5                   10                  15
   Phe Arg Arg Arg Gln Thr Cys Gln Leu Leu Leu Glu Val Ala Trp Pro
               20                  25                  30
   Leu Phe Ile Phe Leu Ile Leu Ile Ser Val Arg Leu Ser Tyr Pro Pro
           35                  40                  45
   Tyr Glu Gln His Glu Cys His Phe Pro Asn Lys Ala Met Pro Ser Ala
       50                  55                  60
   Gly Thr Leu Pro Trp Val Gln Gly Ile Ile Cys Asn Ala Asn Asn Pro
   65                  70                  75                  80
   Cys Phe Arg Tyr Pro Thr Pro Gly Glu Ala Pro Gly Val Val Gly Asn
                   85                  90                  95
   Phe Asn Lys Ser Ile Val Ala Arg Leu Phe Ser Asp Ala Arg Arg Leu
   100 105 110
   Leu Leu Tyr Ser Gln Lys Asp Thr Ser Met Lys Asp Met Arg Lys Val
   115 120 125
   Leu Arg Thr Leu Gln Gln Ile Lys Lys Ser Ser Ser Asn Leu Lys Leu
   130 135 140
   Gln Asp Phe Leu Val Asp Asn Glu Thr Phe Ser Gly Phe Leu Tyr His
   145 150 155 160
   Asn Leu Ser Leu Pro Lys Ser Thr Val Asp Lys Met Leu Arg Ala Asp
   165 170 175
   Val Ile Leu His Lys Val Phe Leu Gln Gly Tyr Gln Leu His Leu Thr
   180 185 190
   Ser Leu Cys Asn Gly Ser Lys Ser Glu Glu Met Ile Gln Leu Gly Asp
   195 200 205
   Gln Glu Val Ser Glu Leu Cys Gly Leu Pro Lys Glu Lys Leu Ala Ala
   210 215 220
   Ala Glu Arg Val Leu Arg Ser Asn Met Asp Ile Leu Lys Pro Ile Leu
   225 230 235 240
   Arg Thr Leu Asn Ser Thr Ser Pro Phe Pro Ser Lys Glu Leu Ala Glu
   245 250 255
   Ala Thr Lys Thr Leu Leu His Ser Leu Gly Thr Leu Ala Gln Glu Leu
   260 265 270
   Phe Ser Met Arg Ser Trp Ser Asp Met Arg Gln Glu Val Met Phe Leu
   275 280 285
   Thr Asn Val Asn Ser Ser Ser Ser Ser Thr Gln Ile Tyr Gln Ala Val
   290 295 300
   Ser Arg Ile Val Cys Gly His Pro Glu Gly Gly Gly Leu Lys Ile Lys
   305 310 315 320
   Ser Leu Asn Trp Tyr Glu Asp Asn Asn Tyr Lys Ala Leu Phe Gly Gly
   325 330 335
   Asn Gly Thr Glu Glu Asp Ala Glu Thr Phe Tyr Asp Asn Ser Thr Thr
   340 345 350
   Pro Tyr Cys Asn Asp Leu Met Lys Asn Leu Glu Ser Ser Pro Leu Ser
   355 360 365
   Arg Ile Ile Trp Lys Ala Leu Lys Pro Leu Leu Val Gly Lys Ile Leu
   370 375 380
   Tyr Thr Pro Asp Thr Pro Ala Thr Arg Gln Val Met Ala Glu Val Asn
   385 390 395 400
   Lys Thr Phe Gln Glu Leu Ala Val Phe His Asp Leu Glu Gly Met Trp
   405 410 415
   Glu Glu Leu Ser Pro Lys Ile Trp Thr Phe Met Glu Asn Ser Gln Glu
   420 425 430
   Met Asp Leu Val Arg Met Leu Leu Asp Ser Arg Asp Asn Asp His Phe
   435 440 445
   Trp Glu Gln Gln Leu Asp Gly Leu Asp Trp Thr Ala Gln Asp Ile Val
   450 455 460
   Ala Phe Leu Ala Lys His Pro Glu Asp Val Gln Ser Ser Asn Gly Ser
   465 470 475 480
   Val Tyr Thr Trp Arg Glu Ala Phe Asn Glu Thr Asn Gln Ala Ile Arg
   485 490 495
   Thr Ile Ser Arg Phe Met Glu Cys Val Asn Leu Asn Lys Leu Glu Pro
   500 505 510
   Ile Ala Thr Glu Val Trp Leu Ile Asn Lys Ser Met Glu Leu Leu Asp
   515 520 525
   Glu Arg Lys Phe Trp Ala Gly Ile Val Phe Thr Gly Ile Thr Pro Gly
   530 535 540
   Ser Ile Glu Leu Pro His His Val Lys Tyr Lys Ile Arg Met Asp Ile
   545 550 555 560
   Asp Asn Val Glu Arg Thr Asn Lys Ile Lys Asp Gly Tyr Trp Asp Pro
   565 570 575
   Gly Pro Arg Ala Asp Pro Phe Glu Asp Met Trp Tyr Val Trp Gly Gly
   580 585 590
   Phe Ala Tyr Leu Gln Asp Val Val Glu Gln Ala Ile Ile Arg Val Leu
   595 600 605
   Thr Gly Thr Glu Lys Lys Thr Gly Val Tyr Met Gln Gln Met Pro Tyr
   610 615 620
   Pro Cys Tyr Val Asp Asp Ile Phe Leu Arg Val Met Ser Arg Ser Met
   625 630 635 640
   Pro Leu Phe Met Thr Leu Ala Trp Ile Tyr Ser Val Ala Val Ile Ile
   645 650 655
   Lys Gly Ile Val Tyr Glu Lys Glu Ala Arg Leu Lys Glu Thr Met Arg
   660 665 670
   Ile Met Gly Leu Asp Asn Ser Ile Leu Trp Phe Ser Trp Phe Ile Ser
   675 680 685
   Ser Leu Ile Pro Leu Leu Val Ser Ala Gly Leu Leu Val Val Ile Leu
   690 695 700
   Lys Leu Gly Asn Leu Leu Pro Tyr Ser Asp Pro Ser Val Val Phe Val
   705 710 715 720
   Phe Leu Ser Val Phe Ala Val Val Thr Ile Leu Gln Cys Phe Leu Ile
   725 730 735
   Ser Thr Leu Phe Ser Arg Ala Asn Leu Ala Ala Ala Cys Gly Gly Ile
   740 745 750
   Ile Tyr Phe Thr Leu Tyr Leu Pro Tyr Val Leu Cys Val Ala Trp Gln
   755 760 765
   Asp Tyr Val Gly Phe Thr Leu Lys Ile Phe Ala Ser Leu Leu Ser Pro
   770 775 780
   Val Ala Phe Gly Phe Gly Cys Glu Tyr Phe Ala Leu Phe Glu Glu Gln
   785 790 795 800
   Gly Ile Gly Val Gln Trp Asp Asn Leu Phe Glu Ser Pro Val Glu Glu
   805 810 815
   Asp Gly Phe Asn Leu Thr Thr Ser Ile Ser Met Met Leu Phe Asp Thr
   820 825 830
   Phe Leu Tyr Gly Val Met Thr Trp Tyr Ile Glu Ala Val Phe Pro Gly
   835 840 845
   Gln Tyr Gly Ile Pro Arg Pro Trp Tyr Phe Pro Cys Thr Lys Ser Tyr
   850 855 860
   Trp Phe Gly Glu Glu Ser Asp Glu Lys Ser His Pro Gly Ser Asn Gln
   865 870 875 880
   Lys Arg Met Ser Glu Ile Cys Met Glu Glu Glu Pro Thr His Leu Lys
   885 890 895
   Leu Gly Val Ser Ile Gln Asn Leu Val Lys Val Tyr Arg Asp Gly Met
   900 905 910
   Lys Val Ala Val Asp Gly Leu Ala Leu Asn Phe Tyr Glu Gly Gln Ile
   915 920 925
   Thr Ser Phe Leu Gly His Asn Gly Ala Gly Lys Thr Thr Thr Met Ser
   930 935 940
   Ile Leu Thr Gly Leu Phe Pro Pro Thr Ser Gly Thr Ala Tyr Ile Leu
   945 950 955 960
   Gly Lys Asp Ile Arg Ser Glu Met Ser Thr Ile Arg Gln Asn Leu Gly
   965 970 975
   Val Cys Pro Gln His Asn Val Leu Phe Asp Met Leu Thr Val Glu Glu
   980 985 990
   His Ile Trp Phe Tyr Ala Arg Leu Lys Gly Leu Ser Glu Lys His Val
   995 1000 1005
   Lys Ala Glu Met Glu Gln Met Ala Leu Asp Val Gly Leu Pro Ser Ser
   1010 1015 1020
   Lys Leu Lys Ser Lys Thr Ser Gln Leu Ser Gly Gly Met Gln Arg Lys
   1025 1030 1035 1040
   Leu Ser Val Ala Leu Ala Phe Val Gly Gly Ser Lys Val Val Ile Leu
   1045 1050 1055
   Asp Glu Pro Thr Ala Gly Val Asp Pro Tyr Ser Arg Arg Gly Ile Trp
   1060 1065 1070
   Glu Leu Leu Leu Lys Tyr Arg Gln Gly Arg Thr Ile Ile Leu Ser Thr
   1075 1080 1085
   His His Met Asp Glu Ala Asp Val Leu Gly Asp Arg Ile Ala Ile Ile
   1090 1095 1100
   Ser His Gly Lys Leu Cys Cys Val Gly Ser Ser Leu Phe Leu Lys Asn
   1105 1110 1115 1120
   Gln Leu Gly Thr Gly Tyr Tyr Leu Thr Leu Val Lys Lys Asp Val Glu
   1125 1130 1135
   Ser Ser Leu Ser Ser Cys Arg Asn Ser Ser Ser Thr Val Ser Tyr Leu
   1140 1145 1150
   Lys Lys Glu Asp Ser Val Ser Gln Ser Ser Ser Asp Ala Gly Leu Gly
   1155 1160 1165
   Ser Asp His Glu Ser Asp Thr Leu Thr Ile Asp Val Ser Ala Ile Ser
   1170 1175 1180
   Asn Leu Ile Arg Lys His Val Ser Glu Ala Arg Leu Val Glu Asp Ile
   1185 1190 1195 1200
   Gly His Glu Leu Thr Tyr Val Leu Pro Tyr Glu Ala Ala Lys Glu Gly
   1205 1210 1215
   Ala Phe Val Glu Leu Phe His Glu Ile Asp Asp Arg Leu Ser Asp Leu
   1220 1225 1230
   Gly Ile Ser Ser Tyr Gly Ile Ser Glu Thr Thr Leu Glu Glu Ile Phe
   1235 1240 1245
   Leu Lys Val Ala Glu Glu Ser Gly Val Asp Ala Glu Thr Ser Asp Gly
   1250 1255 1260
   Thr Leu Pro Ala Arg Arg Asn Arg Arg Ala Phe Gly Asp Lys Gln Ser
   1265 1270 1275 1280
   Cys Leu Arg Pro Phe Thr Glu Asp Asp Ala Ala Asp Pro Asn Asp Ser
   1285 1290 1295
   Asp Ile Asp Pro Glu Ser Arg Glu Thr Asp Leu Leu Ser Gly Met Asp
   1300 1305 1310
   Gly Lys Gly Ser Tyr Gln Val Lys Gly Trp Lys Leu Thr Gln Gln Gln
   1315 1320 1325
   Phe Val Ala Leu Leu Trp Lys Arg Leu Leu Ile Ala Arg Arg Ser Arg
   1330 1335 1340
   Lys Gly Phe Phe Ala Gln Ile Val Leu Pro Ala Val Phe Val Cys Ile
   1345 1350 1355 1360
   Ala Leu Val Phe Ser Leu Ile Val Pro Pro Phe Gly Lys Tyr Pro Ser
   1365 1370 1375
   Leu Glu Leu Gln Pro Trp Met Tyr Asn Glu Gln Tyr Thr Phe Val Ser
   1380 1385 1390
   Asn Asp Ala Pro Glu Asp Thr Gly Thr Leu Glu Leu Leu Asn Ala Leu
   1395 1400 1405
   Thr Lys Asp Pro Gly Phe Gly Thr Arg Cys Met Glu Gly Asn Pro Ile
   1410 1415 1420
   Pro Asp Thr Pro Cys Gln Ala Gly Glu Glu Glu Trp Thr Thr Ala Pro
   1425 1430 1435 1440
   Val Pro Gln Thr Ile Met Asp Leu Phe Gln Asn Gly Asn Trp Thr Met
   1445 1450 1455
   Gln Asn Pro Ser Pro Ala Cys Gln Cys Ser Ser Asp Lys Ile Lys Lys
   1460 1465 1470
   Met Leu Pro Val Cys Pro Pro Gly Ala Gly Gly Leu Pro Pro Pro Gln
   1475 1480 1485
   Arg Lys Gln Asn Thr Ala Asp Ile Leu Gln Asp Leu Thr Gly Arg Asn
   1490 1495 1500
   Ile Ser Asp Tyr Leu Val Lys Thr Tyr Val Gln Ile Ile Ala Lys Ser
   1505 1510 1515 1520
   Leu Lys Asn Lys Ile Trp Val Asn Glu Phe Arg Tyr Gly Gly Phe Ser
   1525 1530 1535
   Leu Gly Val Ser Asn Thr Gln Ala Leu Pro Pro Ser Gln Glu Val Asn
   1540 1545 1550
   Asp Ala Ile Lys Gln Met Lys Lys His Leu Lys Leu Ala Lys Asp Ser
   1555 1560 1565
   Ser Ala Asp Arg Phe Leu Asn Ser Leu Gly Arg Phe Met Thr Gly Leu
   1570 1575 1580
   Asp Thr Arg Asn Asn Val Lys Val Trp Phe Asn Asn Lys Gly Trp His
   1585 1590 1595 1600
   Ala Ile Ser Ser Phe Leu Asn Val Ile Asn Asn Ala Ile Leu Arg Ala
   1605 1610 1615
   Asn Leu Gln Lys Gly Glu Asn Pro Ser His Tyr Gly Ile Thr Ala Phe
   1620 1625 1630
   Asn His Pro Leu Asn Leu Thr Lys Gln Gln Leu Ser Glu Val Ala Leu
   1635 1640 1645
   Met Thr Thr Ser Val Asp Val Leu Val Ser Ile Cys Val Ile Phe Ala
   1650 1655 1660
   Met Ser Phe Val Pro Ala Ser Phe Val Val Phe Leu Ile Gln Glu Arg
   1665 1670 1675 1680
   Val Ser Lys Ala Lys His Leu Gln Phe Ile Ser Gly Val Lys Pro Val
   1685 1690 1695
   Ile Tyr Trp Leu Ser Asn Phe Val Trp Asp Met Cys Asn Tyr Val Val
   1700 1705 1710
   Pro Ala Thr Leu Val Ile Ile Ile Phe Ile Cys Phe Gln Gln Lys Ser
   1715 1720 1725
   Tyr Val Ser Ser Thr Asn Leu Pro Val Leu Ala Leu Leu Leu Leu Leu
   1730 1735 1740
   Tyr Gly Trp Ser Ile Thr Pro Leu Met Tyr Pro Ala Ser Phe Val Phe
   1745 1750 1755 1760
   Lys Ile Pro Ser Thr Ala Tyr Val Val Leu Thr Ser Val Asn Leu Phe
   1765 1770 1775
   Ile Gly Ile Asn Gly Ser Val Ala Thr Phe Val Leu Glu Leu Phe Thr
   1780 1785 1790
   Asp Asn Lys Leu Asn Asn Ile Asn Asp Ile Leu Lys Ser Val Phe Leu
   1795 1800 1805
   Ile Phe Pro His Phe Cys Leu Gly Arg Gly Leu Ile Asp Met Val Lys
   1810 1815 1820
   Asn Gln Ala Met Ala Asp Ala Leu Glu Arg Phe Gly Glu Asn Arg Phe
   1825 1830 1835 1840
   Val Ser Pro Leu Ser Trp Asp Leu Val Gly Arg Asn Leu Phe Ala Met
   1845 1850 1855
   Ala Val Glu Gly Val Val Phe Phe Leu Ile Thr Val Leu Ile Gln Tyr
   1860 1865 1870
   Arg Phe Phe Ile Arg Pro Arg Pro Val Asn Ala Lys Leu Ser Pro Leu
   1875 1880 1885
   Asn Asp Glu Asp Glu Asp Val Arg Arg Glu Arg Gln Arg Ile Leu Asp
   1890 1895 1900
   Gly Gly Gly Gln Asn Asp Ile Leu Glu Ile Lys Glu Leu Thr Lys Ile
   1905 1910 1915 1920
   Tyr Arg Arg Lys Arg Lys Pro Ala Val Asp Arg Ile Cys Val Gly Ile
   1925 1930 1935
   Pro Pro Gly Glu Cys Phe Gly Leu Leu Gly Val Asn Gly Ala Gly Lys
   1940 1945 1950
   Ser Ser Thr Phe Lys Met Leu Thr Gly Asp Thr Thr Val Thr Arg Gly
   1955 1960 1965
   Asp Ala Phe Leu Asn Lys Asn Ser Ile Leu Ser Asn Ile His Glu Val
   1970 1975 1980
   His Gln Asn Met Gly Tyr Cys Pro Gln Phe Asp Ala Ile Thr Glu Leu
   1985 1990 1995 2000
   Leu Thr Gly Arg Glu His Val Glu Phe Phe Ala Leu Leu Arg Gly Val
   2005 2010 2015
   Pro Glu Lys Glu Val Gly Lys Val Gly Glu Trp Ala Ile Arg Lys Leu
   2020 2025 2030
   Gly Leu Val Lys Tyr Gly Glu Lys Tyr Ala Gly Asn Tyr Ser Gly Gly
   2035 2040 2045
   Asn Lys Arg Lys Leu Ser Thr Ala Met Ala Leu Ile Gly Gly Pro Pro
   2050 2055 2060
   Val Val Phe Leu Asp Glu Pro Thr Thr Gly Met Asp Pro Lys Ala Arg
   2065 2070 2075 2080
   Arg Phe Leu Trp Asn Cys Ala Leu Ser Val Val Lys Glu Gly Arg Ser
   2085 2090 2095
   Val Val Leu Thr Ser His Ser Met Glu Glu Cys Glu Ala Leu Cys Thr
   2100 2105 2110
   Arg Met Ala Ile Met Val Asn Gly Arg Phe Arg Cys Leu Gly Ser Val
   2115 2120 2125
   Gln His Leu Lys Asn Arg Phe Gly Asp Gly Tyr Thr Ile Val Val Arg
   2130 2135 2140
   Ile Ala Gly Ser Asn Pro Asp Leu Lys Pro Val Gln Asp Phe Phe Gly
   2145 2150 2155 2160
   Leu Ala Phe Pro Gly Ser Val Leu Lys Glu Lys His Arg Asn Met Leu
   2165 2170 2175
   Gln Tyr Gln Leu Pro Ser Ser Leu Ser Ser Leu Ala Arg Ile Phe Ser
   2180 2185 2190
   Ile Leu Ser Gln Ser Lys Lys Arg Leu His Ile Glu Asp Tyr Ser Val
   2195 2200 2205
   Ser Gln Thr Thr Leu Asp Gln Val Phe Val Asn Phe Ala Lys Asp Gln
   2210 2215 2220
   Ser Asp Asp Asp His Leu Lys Asp Leu Ser Leu His Lys Asn Gln Thr
   2225 2230 2235 2240
   Val Val Asp Val Ala Val Leu Thr Ser Phe Leu Gln Asp Glu Lys Val
   2245 2250 2255
   Lys Glu Ser Tyr Val
   2260
<210> 11
   <211> 24
   <212> DNA
   <213> Artificial Sequence
<220>
<220>
<220>
<220>
   <223> Description of Artificial Sequence: ABC1 amplification primer
<400> 11
   cctctcatta cacaaaaacc agac 24
<210> 12
   <211> 23
   <212> DNA
   <213> Artificial Sequence
<220>
<220>
<220>
<220>
   <223> Description of Artificial Sequence: ABC1 amplification primer
<400> 12
   gctttctttc acttctcatc ctg 23
<210> 13
   <211> 22
   <212> DNA
   <213> Artificial Sequence
<220>
<220>
   <223> Description of Artificial Sequence: ABC1 RT-PCR primer
<400> 13
   tccttgggtt caggggatta tc 22
<210> 14
   <211> 21
   <212> DNA
   <213> Artificial Sequence
<220>
<220>
   <223> Description of Artificial Sequence: ABC1 RT-PCR primer
<400> 14
   caatgttttt gtggcttcgg c 21
<210> 15
   <211> 40
   <212> DNA
   <213> Artificial Sequence
<220>
<220>
   <223> Description of Artificial Sequence: ABC1 RT-PCR primer
<400> 15
   agtcgagctc caaacatgtc agctgttact ggaagtggcc 40
<210> 16
   <211> 25
   <212> DNA
   <213> Artificial Sequence
<220>
<220>
   <223> Description of Artificial Sequence: ABC1 RT-PCR primer
<400> 16
   tctctggatt ctgggtctat gtcag 25
<210> 17
   <211> 23
   <212> DNA
   <213> Artificial Sequence
<220>
<220>
   <223> Description of Artificial Sequence: ABC1 RT-PCR primer
<400> 17
   gggagccttt gtggaactct ttc 23
<210> 18
   <211> 41
   <212> DNA
   <213> Artificial Sequence
<220>
<220>
   <223> Description of Artificial Sequence: ABC1 RT-PCR primer
<400> 18
   actggtcgac cattgaattg cattgcattg aatagtatca g 41
<210> 19
   <211> 19
   <212> DNA
   <213> Artificial Sequence
<220>
<220>
   <223> Description of Artificial Sequence: ABC1 sequencing primer
<400> 19
   tttcctggtg gacaatgaa 19
<210> 20
   <211> 18
   <212> DNA
   <213> Artificial Sequence
<220>
<220>
   <223> Description of Artificial Sequence: ABC1 sequencing primer
<400> 20
   agtgacatgc gacaggag 18
<210> 21
   <211> 18
   <212> DNA
   <213> Artificial Sequence
<220>
<220>
   <223> Description of Artificial Sequence: ABC1 sequencing primer
<400> 21
   gatctggaag gcatgtgg 18
<210> 22
   <211> 18
   <212> DNA
   <213> Artificial Sequence
<220>
<220>
   <223> Description of Artificial Sequence: ABC1 sequencing primer
<400> 22
   ccaggcagca ttgagctg 18
<210> 23
   <211> 18
   <212> DNA
   <213> Artificial Sequence
<220>
<220>
   <223> Description of Artificial Sequence: ABC1 sequencing primer
<400> 23
   ggcctggaca acagcata 18
<210> 24
   <211> 19
   <212> DNA
   <213> Artificial Sequence
<220>
<220>
   <223> Description of Artificial Sequence: ABC1 sequencing primer
<400> 24
   ggacaacctg tttgagagt 19
<210> 25
   <211> 18
   <212> DNA
   <213> Artificial Sequence
<220>
<220>
   <223> Description of Artificial Sequence: ABC1 sequencing primer
<400> 25
   aagacgacca ccatgtca 18
<210> 26
   <211> 18
   <212> DNA
   <213> Artificial Sequence
<220>
<220>
   <223> Description of Artificial Sequence: ABC1 sequencing primer
<400> 26
   atatgggagc tgctgctg 18
<210> 27
   <211> 24
   <212> DNA
   <213> Artificial Sequence
<220>
<220>
   <223> Description of Artificial Sequence: ABC1 sequencing primer
<400> 27
   gggcatgagc tgacctatgt gctg 24
<210> 28
   <211> 18
   <212> DNA
   <213> Artificial Sequence
<220>
<220>
   <223> Description of Artificial Sequence: ABC1 sequencing primer
<400> 28
   aagagactgc taattgcc 18
<210> 29
   <211> 18
   <212> DNA
   <213> Artificial Sequence
<220>
<220>
   <223> Description of Artificial Sequence: ABC1 sequencing primer
<400> 29
   agcgacaaaa tcaagaag 18
<210> 30
   <211> 18
   <212> DNA
   <213> Artificial Sequence
<220>
<220>
   <223> Description of Artificial Sequence: ABC1 sequencing primer
<400> 30
   tggcatgcaa tcagctct 18
<210> 31
   <211> 18
   <212> DNA
   <213> Artificial Sequence
<220>
<220>
   <223> Description of Artificial Sequence: ABC1 sequencing primer
<400> 31
   tcctccacca atctgcct 18
<210> 32
   <211> 18
   <212> DNA
   <213> Artificial Sequence
<220>
<220>
   <223> Description of Artificial Sequence: ABC1 sequencing primer
<400> 32
   ttcttcctca ttactgtt 18
<210> 33
   <211> 18
   <212> DNA
   <213> Artificial Sequence
<220>
<220>
   <223> Description of Artificial Sequence: ABC1 sequencing primer
<400> 33
   gatgccatca cagagctg 18
<210> 34
   <211> 17
   <212> DNA
   <213> Artificial Sequence
<220>
<220>
   <223> Description of Artificial Sequence: ABC1 sequencing primer
<400> 34
   agtgtccagc atctaaa 17
<210> 35
   <211> 18
   <212> DNA
   <213> Artificial Sequence
<220>
<220>
   <223> Description of Artificial Sequence: ABC1 sequencing primer
<400> 35
   caaagttcac aaatactt 18
<210> 36
   <211> 19
   <212> DNA
   <213> Artificial Sequence
<220>
<220>
   <223> Description of Artificial Sequence: ABC1 sequencing primer
<400> 36
   cttagggcac aattccaca 19
<210> 37
   <211> 18
   <212> DNA
   <213> Artificial Sequence
<220>
<220>
   <223> Description of Artificial Sequence: ABC1 sequencing primer
<400> 37
   tgaaagttga tgattttc 18
<210> 38
   <211> 19
   <212> DNA
   <213> Artificial Sequence
<220>
<220>
   <223> Description of Artificial Sequence: ABC1 sequencing primer
<400> 38
   tttttcacca tgtcgatga 19
<210> 39
   <211> 17
   <212> DNA
   <213> Artificial Sequence
<220>
<220>
   <223> Description of Artificial Sequence: ABC1 sequencing primer
<400> 39
   ctccactgat gaactgc 17
<210> 40
   <211> 18
   <212> DNA
   <213> Artificial Sequence
<220>
<220>
   <223> Description of Artificial Sequence: ABC1 sequencing primer
<400> 40
   gtttcttcat ttgtttga 18
<210> 41
   <211> 18
   <212> DNA
   <213> Artificial Sequence
<220>
<220>
   <223> Description of Artificial Sequence: ABC1 sequencing primer
<400> 41
   agggcgtgtc tgggattg 18
<210> 42
   <211> 18
   <212> DNA
   <213> Artificial Sequence
<220>
<220>
   <223> Description of Artificial Sequence: ABC1 sequencing primer
<400> 42
   cagaatcatt tggatcag 18
<210> 43
   <211> 18
   <212> DNA
   <213> Artificial Sequence
<220>
<220>
   <223> Description of Artificial Sequence: ABC1 sequencing primer
<400> 43
   catcagaact gctctgag 18
<210> 44
   <211> 19
   <212> DNA
   <213> Artificial Sequence
<220>
<220>
   <223> Description of Artificial Sequence: ABC1 sequencing primer
<400> 44
   agctggcttg ttttgcttt 19
<210> 45
   <211> 18
   <212> DNA
   <213> Artificial Sequence
<220>
<220>
   <223> Description of Artificial Sequence: ABC1 sequencing primer
<400> 45
   tggacacgcc cagcttca 18
<210> 46
   <211> 18
   <212> DNA
   <213> Artificial Sequence
<220>
<220>
   <223> Description of Artificial Sequence: ABC1 sequencing primer
<400> 46
   cctgccatgc cacacaca 18
<210> 47
   <211> 18
   <212> DNA
   <213> Artificial Sequence
<220>
<220>
   <223> Description of Artificial Sequence: ABC1 sequencing primer
<400> 47
   ctcatcaccc gcagaaag 18
<210> 48
   <211> 18
   <212> DNA
   <213> Artificial Sequence
<220>
<220>
   <223> Description of Artificial Sequence: ABC1 sequencing primer
<400> 48
   cacactccat gaagcgag 18
<210> 49
   <211> 18
   <212> DNA
   <213> Artificial Sequence
<220>
<220>
   <223> Description of Artificial Sequence: ABC1 sequencing primer
<400> 49
   tccagataat gcgggaaa 18
<210> 50
   <211> 18
   <212> DNA
   <213> Artificial Sequence
<220>
<220>
   <223> Description of Artificial Sequence: ABC1 sequencing primer
<400> 50
   tcaggattgg cttcagga 18
<210> 51
   <211> 21
   <212> DNA
   <213> Artificial Sequence
<220>
<220>
   <223> Description of Artificial Sequence: ABC1 sequencing primer
<400> 51
   aagtttgagc tggatttctt g 21
<210> 52
   <211> 25
   <212> DNA
   <213> Artificial Sequence
<220>
<220>
   <223> Description of Artificial Sequence: beta-globin antisense oligonucleotide
<400> 52
   cctcttacct cagttacaat ttata 25
<210> 53
   <211> 26
   <212> DNA
   <213> Artificial Sequence
<220>
<220>
   <223> Description of Artificial Sequence: ABC1 antisense oligonucleotide
<400> 53
   catgttgttc atagggtggg tagctc 26
<210> 54 '
   <211> 24
   <212> DNA
   <213> Artificial Sequence
<220>
<220>
   <223> Description of Artificial Sequence: beta-actin amplification primer
<400> 54
   tcacccacac tgtgccatct acga 24
<210> 55
   <211> 25
   <212> DNA
   <213> Artificial Sequence
<220>
<220>
   <223> Description of Artificial Sequence: beta-actin amplification primer
<400> 55
   cagcggaacc gctcattgcc aatgg 25
<210> 56
   <211> 26
   <212> DNA
   <213> Artificial Sequence
<220>
<220>
   <223> Description of Artificial Sequence: sterol response element oligonucleotide
<400> 56
   tcgagtgacc gatagtaacc tctcga 26
<210> 57
   <211> 26
   <212> DNA
   <213> Artificial Sequence
<220>
<220>
   <223> Description of Artificial Sequence: mutated sterol response element oligonucleotide
<400> 57
   tcgagctgca catagtaacc tctcga 26

## Description

### TECHNICAL FIELD OF INVENTION

The present invention relates to isolated polynucleotides of the 5' flanking region of the mammalian ABC1 gene. The invention also relates to recombinant vectors, and compositions comprising said polynucleotides, as well as to host cells comprising said vectors. The present invention further relates to methods for identifying compounds that modulate the expression of ABC1. The present invention also provides kits suitable for screening compounds to determine the ABC1 expression modulating activity of the compound.

### BACKGROUND OF THE INVENTION

Circulating lipids in human plasma or lymphatic fluid consist of cholesterol, cholesteryl esters, triglycerides and phospholipids. These lipids are transported in large molecular complexes called lipoproteins, which consist of a core of cholesteryl esters and/or triglycerides, an envelope of phospholipids and free cholesterol, and apolipoproteins (Scriver et al., Eds., The Metabolic and Molecular Basis of Inherited Disease, 7th Ed., p.1841-1851 (McGraw-Hill, New York 1995)). Apolipoproteins are involved in the assembly and secretion of the lipoprotein, as well as the activation of lipoprotein modifying enzymes, such as lecithin cholesterol acyl transferase (LCAT). In addition, apolipoproteins provide structural integrity and are ligands for a large spectrum of receptors and membrane docking proteins. The plasma lipoproteins are categorized into five types according to size: chylomicrons (largest in size and lowest in density), very low density lipoproteins (VLDL), intermediate density lipoproteins (IDL), low density lipoproteins (LDL) and high density lipoprotein (HDL).

Chylomicrons, VLDLs, IDLs, and LDLs transport exogenous and endogenous cholesterol and triacylglycerols to peripheral sites, where the lipids play a role in various metabolic pathways and serve as a major constituent of cell membranes. Chylomicrons are assembled in the intestinal mucosa as a means to transport dietary cholesterol and triacylglycerols to various tissues. VLDLs are formed in the liver to transport endogenous cholesterol and triacylglycerols synthesized by the liver to extra-hepatic tissues, such as muscle and adipose tissue. In fasting serum, VLDLs contain 10-15% of the total serum cholesterol and most of the triglycerides. In circulation, VLDLs are converted to LDLs through the action of lipoprotein lipase. LDLs are the primary plasma carriers of cholesterol for delivery to all tissues, typically containing 60-70% of the total fasting serum cholesterol.

In contrast, HDLs are involved in "reverse cholesterol transport", the pathway by which excess cholesterol is transporter from peripheral sites back to the liver, where it is excreted in the form of bile salts (Glomset, J.A., J. Lipid Res., 9, 155-167 (1968)). Nascent HDLs are synthesized *de novo* in the liver and small intestine, as protein-rich disc-shaped particles devoid of cholesterol and cholesterol esters. In fact, a major function of HDLs is to act as circulating stores of apolipoproteins, primarily apo C-I, apo C**-**II, and apoE. The nascent or protein-rich HDLs are converted into spherical lipoprotein particles through the accumulation of cholesteryl esters obtained from cellular sources. The HDLs normally contain 20-30% of the total fasting serum cholesterol.

According to current theories, the reverse efflux of cellular cholesterol to HDL is mediated through two mechanisms: an aqueous diffusion pathway and an apolipoprotein-mediated pathway. The relative importance of these distinguishable mechanisms depends on the cell type and metabolic state (Oram et al., J. Lipid Res., 37:2743-2491 (1996); Rothblat et al., J. Lipid Res., 40:781-796 (1999); Stein et al., Atherosclerosis, 144:285-301 (1999)). For many cells, the aqueous diffusion pathway is the principle pathway through which cholesterol efflux occurs (Johnson et al., Biochim. Biophys. Acta, 1085:273-298 (1991)). This pathway involves the bidirectional exchange of cholesterol between cell membranes and a lipoprotein acceptor, such as HDL, in the extracellular space through a process of passive transport (Remaley et al., Arterioscler. Thromb. Vasc. Biol., 17:1813-1821 (1997); Rothblat et al., J. Lip. Res., 40:781-796 (1999)). The exchange may occur primarily at surface microdomains known as caveolae (Fielding et al., Biochemistry, 34:14288-14292 91995)). Net efflux can be driven by conversion of cholesterol in the exteracellular compartment to cholesteryl ester by the action of LCAT.

Alternatively, in macrophage and fibroblast cells, cholesterol and phospholipid efflux is primarily mediated through apolipoproteins, such as apo A-I, apo A-II, and Apo E (Remaley, *supra* (1997); Francis, et al., J. Clin. Invest., 96:78-87 (1995); Vega et al., J. Intern. Med., 226:5-15 (1989); Sakar et al., Biochim. Biophys. Acta, 1438: 85-98 (1999); Hara et al., J. Biol. Chem., 266:3080-3086 (1991); Fielding et al., J. Lipid Res., 38, 1503-1521 (1997); Oram et al., J. Lipid Res., 37, 2743-2491 (1996)). The process of apolipoprotein-mediated lipid efflux particularly dominates in macrophages and other scavenger cells when they are cholesterol-loaded and/or growth-arrested. Apolipoprotein-mediated efflux is an active transport process that requires the direct interaction of the apolipoprotein with the cell surface, the lipidation of the apolipoprotein, and the subsequent dissociation of the lipid-apolipoprotein particle from the cell (Oram, *supra* (1996); Mendez, A.J., J. Lipid Res., 38, 1807-1821 (1997); Remaley, *supra* (1997); Mendez, A.J., J.Lipid Res., 37, 2510-2524 (1996)). Once removed from the cell, the cholesterol-rich HDL particles are transported to the liver and removed from the body as described.

Abnormal lipoprotein function and/or metabolism resulting from genetic defect or as a secondary effect of another disorder can have serious biological consequences. In addition to dietary influences, disorders such as diabetes, hypothyroidism, and liver disease can result in elevated plasma levels of LDL-cholesterol and triglycerides. Elevated levels of LDL-cholesterol and triglycerides have been identified as major risk factors associated with the incidence of coronary heart disease, which is the primary cause of death in the United States and other ' industrialized nations (Hokanson et al., J. Cardiovasc. Risk., 3:213-219 (1996); The Expert Panel, JAMA, 269:3015-3023 (1993)). The accumulation of excess LDL-cholesterol on arterial walls can lead to the formation of atherosclerotic plaques, which play a major role in the development of heart disease. A plaque is believed to form when free radicals released from arterial walls oxidize LDL. According to theory, the oxidized form of LDL triggers an inflammatory response, attracting circulating cells to the site which contribute to the formation of a lipid plaque. Among these are macrophages and other cells that contain scavenger receptors that accumulate cholesterol in an unregulated manner (Brown et al., Ann. Rev. Biochem., 52:223-261 (1986)). Vast stores of internal cholesterol result in conversion to a foam cell phenotype, which is believed to be a major contributor to the development of vascular lesions. As the plaque builds up, the arterial walls constrict, reducing blood flow to the heart.

Interestingly, however, an estimated 60% of heart attacks occur in persons who do not have elevated blood levels of LDL-cholesterol. Of these, an estimated 45% are associated with below average blood levels of HDL-cholesterol, indicating that low HDL-cholesterol level is a significant risk factor for coronary heart disease. In fact, recent studies have indicated that a decreased HDL-cholesterol level is the most common lipoprotein abnormality seen in patients with premature coronary artery disease (Genest J., Circulation, 85:2025-2033 (1992); Genest et al., Arterioscler. Thromb., 13:1728-1737 (1993)). Although the basis for the inverse association between HDL-cholesterol and coronary heart disease is not well understood, it has been suggested that the cardioprotective role of HDL may stem from its activity relating to the promotion of cholesterol efflux from macrophage foam cells in atherosclerotic lesions.

One example of cardiovascular disease associated with low HDL is Tangier disease (TD), a rare genetic disorder characterized by a near or complete absence of circulating HDL. In addition to near zero plasma levels of HDL, patients with TD have a massive deposition and accumulation of cholesteryl esters in several tissues, including tonsils, lymph nodes, liver; spleen, thymus, intestine, and Schwann cells (Fredrickson, D.S., J. Clin. Invest., 43, 228-236 (1964); Assmann et al., The Metabolic Basis of Inherited Disease, (McGraw-Hill, New York, 1995)). Although the cellular mechanisms have not been previously identified, recent studies have shown that cells from subjects with TD are defective in the process of apolipoprotein-mediated removal of cholesterol and phospholipids (Remaley et al., Arterioscler. Thromb. Vasc. Biol., 17, 1813-1821 (1997); Francis et al., J. Clin. Invest., 96, 78-87 (1995); Rogler et al., Arterioscler. Thromb. Vasc. Biol., 15, 683-690 (1995)). These results have led to the proposal that the severe HDL deficiency in TD patients stems from the inability of nascent apo A-I to acquire lipids. Because they do not mature into lipid-rich particles, the nascent HDL in TD patients is rapidly catabolized and removed from the plasma, resulting in the near zero levels of circulating HDL (Remaley, *supra* (1997); Francis, *supra* (1995); Horowitz et al., J. Clin. Invest., 91, 1743-1752 (1993); Schaefer et al., J. Lip. Res., 22:217-228 (1981)). Due to evidence linking mutations in the ATP-binding-cassette transporter gene 1 (ABC1) to TD, the 5' end of the hABC1 gene has been analyzed (Pullinger et al., Biochem. Biophys.Res. Commun., 271:451-455 (2000)).

Other disorders associated with severe premature atherosclerosis and high risk for coronary heart disease resulting from diminished HDL-cholesterol levels are hypoalphalipoproteinemia and familial HDL deficiency syndrome (FHA). Persons with these disorders often have normal LDL-cholesterol and triglyceride levels. In addition, disorders such as diabetes, alcoholism, hypothyroidism, liver disease, and elevated blood pressure can result in diminished plasma levels of HDL-cholesterol, although many of these disorders are also accompanied by elevated LDL-cholesterol and triglceride levels.

Current treatments for coronary heart disease have focused primarily on diet manipulations and/or drug therapies aimed at lowering the plasma level of LDL-cholesterol by inhibiting LDL secretion or promoting LDL turnover. Derivatives of fibric acid, such as clofibrate, gemfibrozil, and fenofibrate, promote rapid VLDL turnover by activating lipoprotein lipase. Nicotinic acid reduces plasma levels of VLDL and LDL by inhibiting hepatic VLDL secretion. In addition, HMG-CoA reductase inhibitors, such as mevinolin, mevastatin, pravastatin, sirnvastatin, fluvastatin, and lovastatin reduce plasma LDL levels by inhibiting the intracellular synthesis of cholesterol, which causes an increase in the cellular uptake of LDL. In addition, bile acid-binding resins, such as cholestyrine, colestipol and probucol decrease the level of LDL-cholesterol by increasing the catabolism of LDL-cholesterol in the liver.

However, many of these therapies are associated with low efficacy and/or side effects that may prevent long-term use. For example, use of HMG-CoA reductase inhibitors carry a significant risk of toxicity because they inhibit the synthesis of mevalonate, which is required for the synthesis of other important isoprenoid compounds in addition to cholesterol. Also, gemfibrozil and nicotinic acid are associated with serious adverse effects, including renal injury, myopathy, myoglobinuria and intolerable skin flushing and itching. In addition, the role of probucol in treating patients with coronary heart disease is uncertain because its administration results in lower HDL-cholesterol levels as a side effect of reducing LDL-cholesterol.

Furthermore, treating patients who have isolated low HDL-cholesterol levels provides a particularly difficult therapeutic challenge. For instance, patients with Tangier disease exhibit a 4- to 6-fold increase in cardiovascular disease even though their LDL levels are already reduced by about 50%. While there is some evidence that gemfibrozil and nicotinic acid may simultaneously elevate HDL levels, in general, therapies aimed at lowering plasma LDL-cholesterol levels are not effective for Tangier patients who suffer from coronary heart disease as a result of diminished HDL levels. Likewise, patients with hypoalphalipoproteinemia, familial HDL deficiency syndrome, or other cardiovascular disease resulting from low levels of HDL will not benefit from therapies aimed at lowering the level of plasma LDL.

The problems associated with current therapies for cardiovascular disease stem partially from the fact that the biology involved in the movement of cholesterol in and out of cells is not fully understood. Furthermore, the proteins that play a role in cholesterol movement are not fully known. Therefore, there remains a need for a better understanding of cholesterol cell biology, as well as new methods for treating humans suffering from cardiovascular disease and other disorders associated with hypercholesterolemia. Additionally, there remains a need for new methods of diagnosing cardiovascular disease and new methods of screening patients to identify those at high risk for developing cardiovascular disease.

The identification of genes and proteins involved in cholesterol transport would be useful in the development of pharmaceutical agents for the treatment of heart disease and other disorders associated with hypercholesterolemia and atherosclerosis. In addition; the identification of such genes would be useful in the development of screening assays to screen for compounds that regulate the expression of genes associated with cholesterol transport. The identification of such regulatory compounds would be useful in the development of further therapeutic agents. Furthermore, the identification of genes and proteins involved in cholesterol transport would be useful as diagnostic indicators of cardiovascular disease and other disorders associated with hypercholesterolemia.

### SUMMARY OF THE INVENTION

The present invention provides ABC1 polynucleotides corresponding to the 5' flanking region of the ABC1 gene. In one embodiment, the invention provides an isolated polynucleotide having the sequence identified as SEQ ID NO: 3. In other embodiments, the invention provides an isolated polynucleotide having the sequence identified as nucleotides 1-1532, 1080-1643, 1181-1643, 1292-1643, 1394-1643, or 1394-1532 of SEQ ID NO: 3. Preferably, the isolated polynucleotide has the sequence identified as nucleotides 1394-1532 of SEQ ID NO: 3. In another embodiment, the invention provides an isolated polynucleotide having a nucleotide sequence that is at least 80% identical to the entire length of the polynucleotide having the sequence identified as SEQ ID NO: 3. More preferably, the polynucleotide is at least 90% identical to the entire length of the polynucleotide having the sequence identified as SEQ ID NO: 3. Even more preferably, the polynucleotide is at least 95% identical to the entire length of the polynucleotide having the sequence identified as SEQ ID NO: 3. Also provided in preferred embodiments is an isolated polynucleotide that is at least 80% identical to the entire length of the polynucleotide having the sequence identified as nucleotides 1-1532, 1080-1643, 1181-1643, 1292-1643, 1394-1643 or 1394-1532 of SEQ ID NO: 3. More preferably, the polynucleotide is at least 90% identical, and even more preferably at least 95% identical, to the entire length of the polynucleotide having the sequence identified as nucleotides 1-1532, 1080-1643, 1181-1643, 1292-1643, 1394-1643, or 1394-1532 of SEQ ID NO: 3. In addition, the present invention provides an isolated polynucleotide that is complementary to the entire length of the above described 5' flanking ABC1 polynucleotides. In one embodiment, the invention provides an isolated polynucleotide that is complementary to the entire length of the polynucleotide having the sequence identified as SEQ ID NO: 3. In another embodiment, the present invention provides an isolated polynucleotide that is complementary to the entire length of the polynucleotide having the sequence identified as nucleotides 1-1532, 1080-1643, 1181-1643, 1292-1643, 1394-1643 or 1394-1532 of SEQ ID NO:3.

In another aspect, the present invention provides a composition comprising any of the above described polynucleotides and a suitable carrier.

In one embodiment, the composition comprises an isolated polynucleotide having the sequence identified as SEQ ID NO: 3 or an isolated polynucleotide having the sequence identified as nucleotides 1-1532, 1080-1643, 1181-1643, 1292-1643, 1394-1643, or 1394-1532 of SEQ ID NO: 3 and a suitable carrier.

The present invention also provides a recombinant vector comprising an isolated polynucleotide having the ABC1 5' flanking sequence. In one embodiment, the invention provides a recombinant vector comprising an isolated polynucleotide having the sequence identified as SEQ ID NO: 3 or an isolated polynucleotide having the sequence identified as nucleotides 1-1532, 1080-1643, 1181-1643, 1292-1643, 1394-1643 or 1394-1532 of SEQ ID NO: 3. In yet another embodiment, the recombinant vector comprises any of the described polynucleotides and further comprises at least one polynucleotide encoding a heterologous polypeptide. Suitable heterologous polypeptides include luciferase, β-galactosidase, chloramphenicol acetyl transferase, and green fluorescent proteins. Preferably, the heterologous polypeptide is a luciferase protein. In a particularly preferred embodiment, the recombinant vector is pAPR1.

In addition, the present invention provides host cells comprising any of the described recombinant vectors.

In addition, the present invention provides a method for screening a test compound for ABC1 expression modulating activity comprising the steps of: (a) operatively linking a reporter cDNA with the 5' flanking region of the mammalian ABC1 gene to produce a recombinant reporter construct; (b) transfecting the recombinant reporter construct into a population of isolated host cells; (c) assaying the level of reporter gene expression in a sample of the host cells; (d) contacting the host cells with the test compound being screened; (e) assaying the level of reporter gene expression in a sample of the host cells after contact with the test compound; and (f) comparing the relative change in the level of reporter gene expression caused by exposure to the test compound, thereby determining the ABC1 expression modulating activity, wherein the 5' flanking region of the mammalian ABC1 gene comprises SEQ ID NO: 3 or a polynucleotide comprising nucleotides 1-1532, 1080-1643, 1181-1643, 1292-1643, 1394-1643, or 1394-1532 of SEQ ID NO: 3. Suitable reporter cDNAs include luciferase, β-galactosidase, chloramphenicol acetyl transferase, and green fluorescent protein cDNA. Preferably, the host cell is a mammalian cell. In a particularly preferred embodiment of the method, the recombinant reporter construct is pAPR1.

In addition, the present invention provides kits suitable for screening a compound to determine the ABC1 expression modulating activity of the compound comprising a recombinant reporter construct comprising a reporter cDNA operatively linked to an expression modulating portion of the mammalian ABC1 gene in an amount sufficient for at least one assay and instructions for use, wherein the expression modulating portion of the mammalian ABC1 gene comprises the isolated polynucleotide having the sequence identified as SEQ ID NO: 3 or the isolated polynucleotide having the sequence identified as nucleotides 1-1532, 1080-1643, 1181-1643, 1292-1643, 1394-1643, or 1394-1532 of SEQ ID NO: 3. Suitable reporter cDNAs include luciferase, β-galactosidase, chloramphenicol acetyl transferase, and green fluorescent protein cDNA. Preferably, the reporter cDNA is luciferase. In a particularly preferred embodiment of the method, the recombinant reporter construct is pAPR1. In one preferred embodiment, the kit further comprises means for detecting the reporter gene.

### BRIEF DESCRIPTION OF THE FIGURES

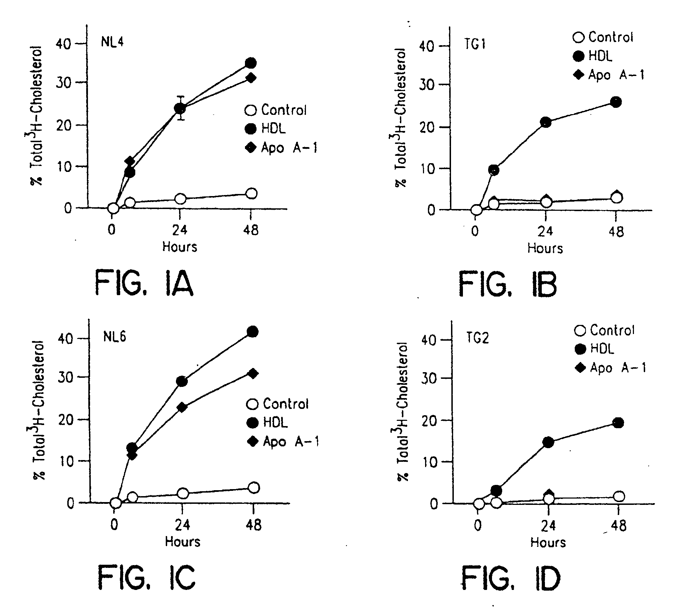

Figure 1A-D is a graphical representation showing the results of control cholesterol efflux and cholesterol efflux in the presence of HDL and apo A-I from normal fibroblast cells (1A, C) and fibroblast cells from Tangier disease patients (1B, D). The open circles represent the cholesterol efflux from cells that were not exposed to HDL or apo A-I, the closed circles represent the cholesterol efflux from cells exposed to HDL, and the closed diamonds represent the cholesterol efflux from cells exposed to apo A-I;

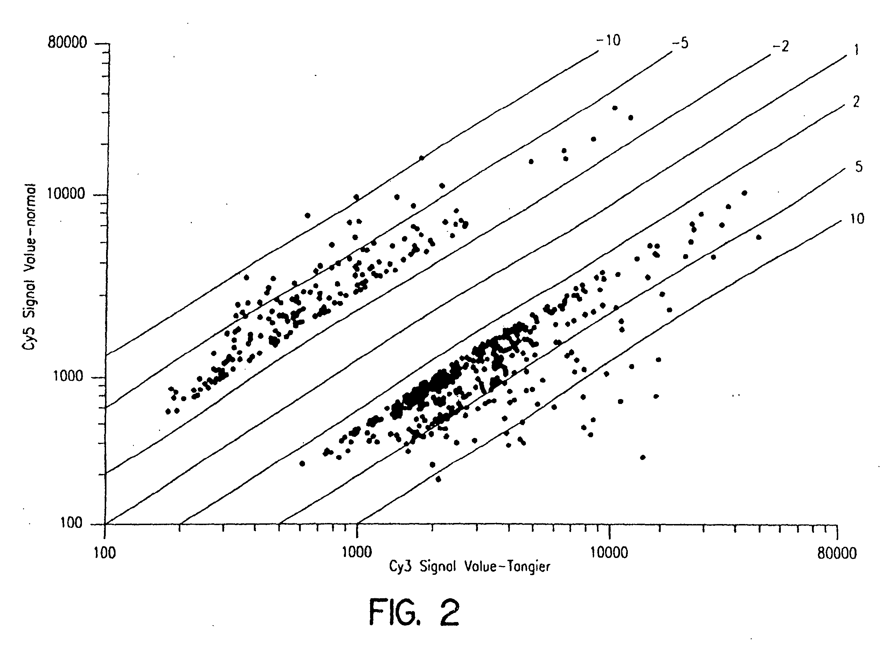

Figure 2 is a graphical representation of a gene expression microarray analysis showing a comparison of the gene expression found in cells from a Tangier patient (TD1) and that found in normal cells, whereby a total of 58,800 human cDNAs were hybridized with cDNA prepared from mRNA of cAMP-treated TD1 cells cDNA (labeled with Cy3 dye) and with cDNA prepared from mRNA of cAMP-treated normal cells (labeled with Cy5 dye);

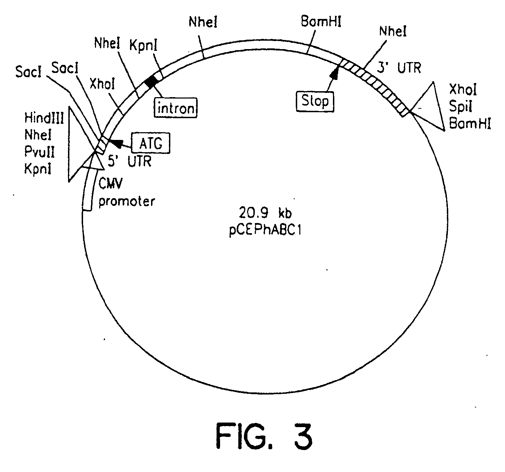

Figure 3 is a schematic diagram showing a restriction map of the recombinant expression vector pCEPhABC1, which contains the open reading frame of the human ABC1 gene;

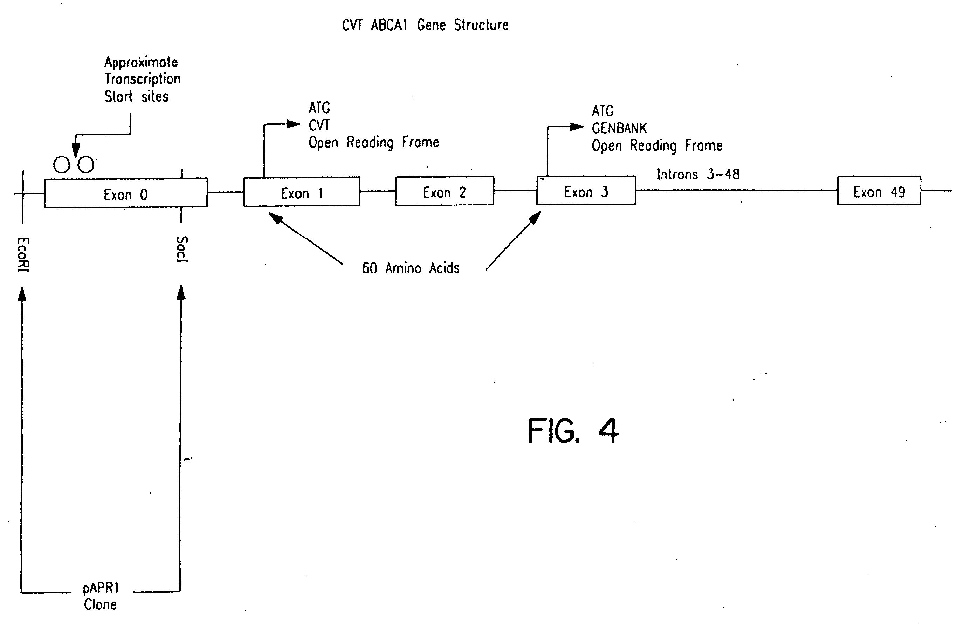

Figure 4 is a schematic diagram of the gene structure of human ABC1, showing a comparison between the published human ABC1 amino acid sequence (GenBank, Accession # AJ012376) and the presently disclosed and claimed human ABC1 amino acid sequence ("CVT") which has sixty additional amino acids at the N-terminal end;

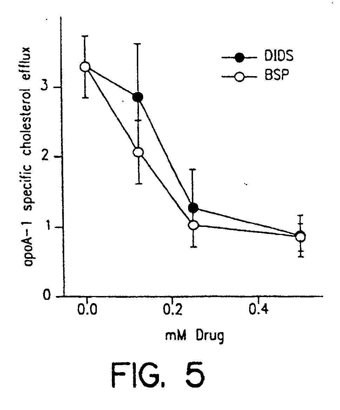

Figure 5 is a graphical representation showing the inhibitory effect that ABC1 transport inhibitors 4,4-diisothiocyanostilbene-2,2'-disulfonic acid (DIDS) and sulphobromophtaleine (BSP) have on apo A-I-mediated cholesterol efflux, wherein the open circles indicate the apo A-I-mediated cholesterol in the presence of BSP and the closed circles indicate the apo A-I-mediated cholesterol in the presence of DIDS;

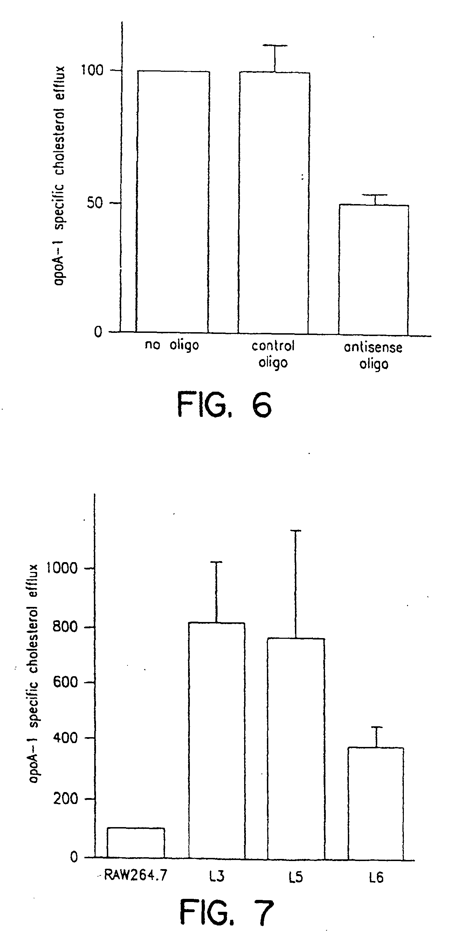

Figure 6 is a graphical representation showing the inhibitory effect of an antisense ABC1 oligonucleotide on apo A-I-mediated cholesterol efflux, showing the apo A-I-mediated cholesterol efflux in cells incubated without antisense oligonucleotide, the apo A-I-mediated cholesterol efflux in cells exposed to 30 µM β-globin antisense oligonucleotide, and the apo A-I-mediated cholesterol efflux in cells exposed to 30 µM ABC1 antisense oligonucleotide;
Figure 7 is a graphical representation demonstrating the stimulation of apo A-I-mediated cholesterol efflux caused by overexpression of the ABC1 gene using RAW 264.7 mouse macrophage cells stably transfected with an expression plasmid for human ABC1 (pCEPhABC1), showing the apo A-I-mediated cholesterol efflux in control parental cells (no pCEPhABC1) and the apo A-I-mediated cholesterol efflux in clonal cells transfected with pCEPhABC1 (L3, L5, L6);

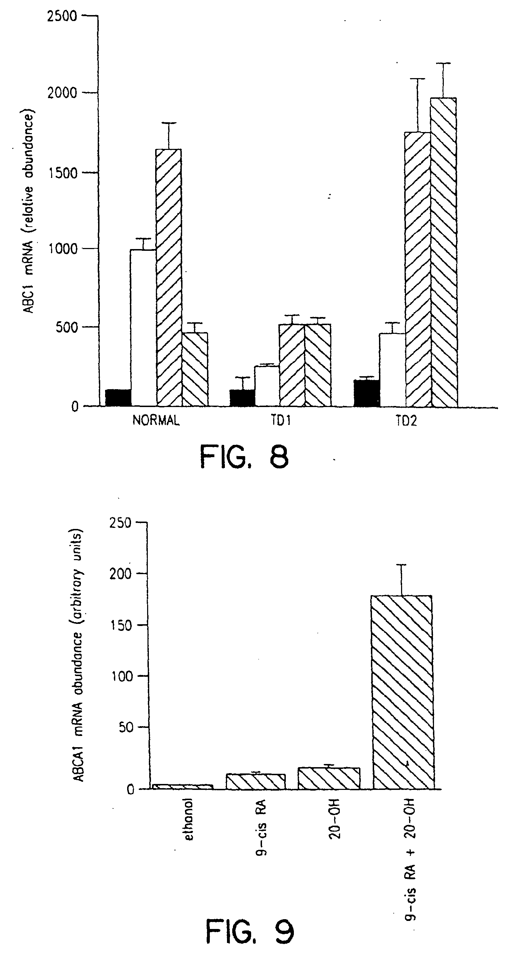

Figure 8 is a graphical representation of reverse transcription polymerase chain reaction (RT-PCR) analyses showing the level of ABC1 gene expression in normal cells and cells from Tangier's disease patients (TD1 and TD2) that have been either exposed to albumin (closed bars), exposed to 8-Br-cAMP (open bars), cholesterol-loaded (shaded bars), or cholesterol-loaded and subsequently exposed to apo A-I (hatched bars);
Figure 9 is a graphical representation of the results of RT-PCR analyses showing the level of ABC1 gene expression in RAW 264.7 cells exposed to either ethanol (0.1 % v/v), 9*-cis* retinoic acid (9*-cis* RA; 10µM), 20(S) hydroxycholesterol (20(S)-OH; 10µM), or 9-*cis* RA and 20(S)-OH (10µM each);

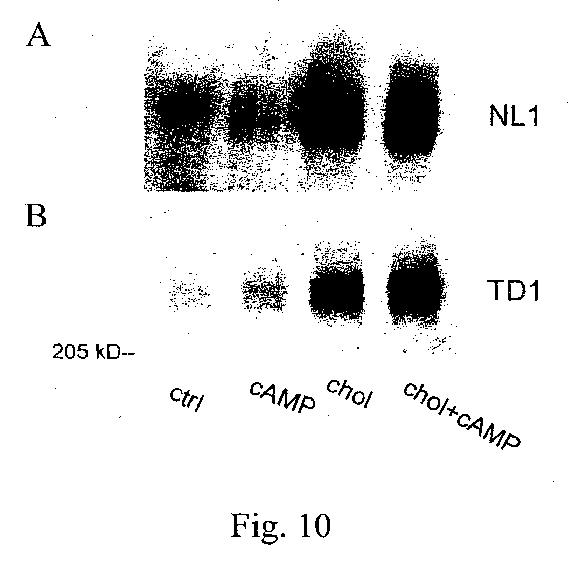

Figure 10 shows the results of immunoprecipitation analyses indicating the level of cell-surface ABC1 protein found in normal fibroblasts (NL1, 10A) and fibroblasts from a Tangier's disease patient (TD1, 10B) in the presence of either no additives (control), 8-Br-cAMP (1mM), cholesterol (30 µg/ml), or cholesterol and 8-Br-cAMP (30µg/ml and 1mM, respectively);

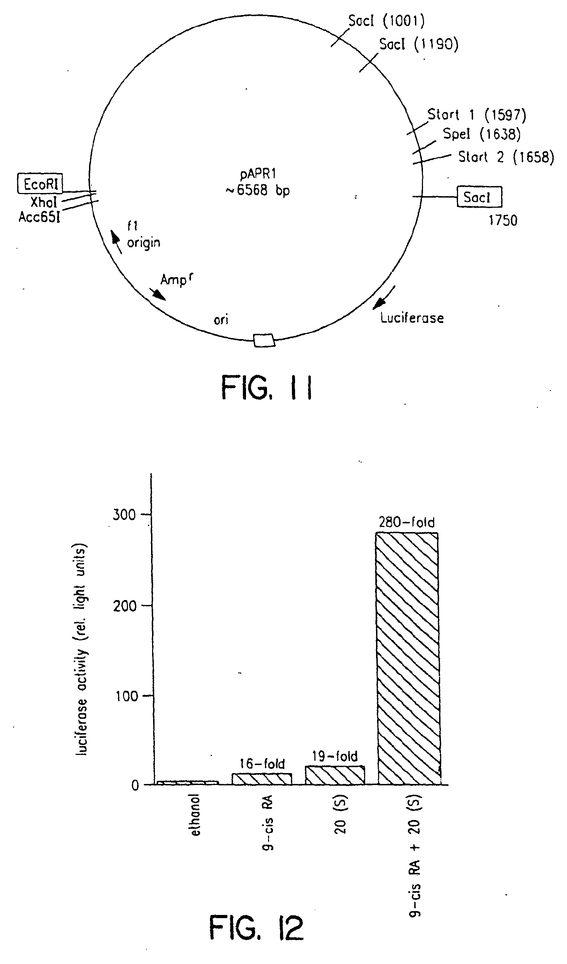

Figure 11 is a schematic diagram showing a restriction map of the recombinant expression vector pAPR1, which contains the 5' flanking region of the ABC1 gene positioned upstream of the open reading frame of the luciferase reporter gene;
Figure 12 is a graphical representation showing the level of luciferase reporter gene expression induced in RAW 264.7 cells transected with pAPR1 in the presence of either EtOH (control), 20(S)-OH (10µM), 9-*cis* RA (10µM), or both 20(S)-OH and 9-*cis* RA (10µM each);

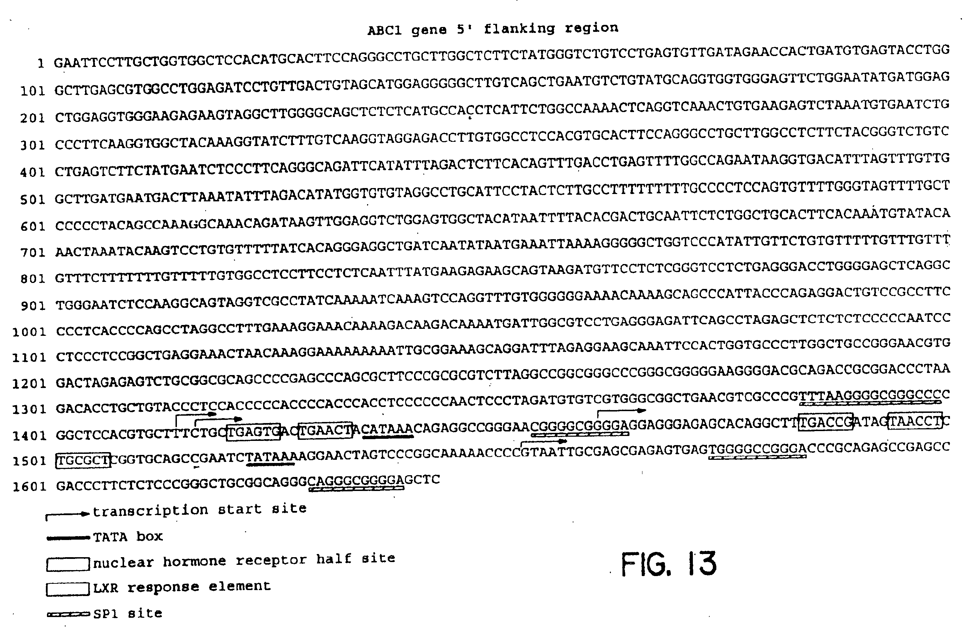

Figure 13 is a schematic diagram of the 5' flanking region of the ABC1 gene.

### DETAILED DESCRIPTION OF THE PREFERRED EMBODIMENTS

ABC1 is a member of the family of ATP-binding cassette proteins that reside in cell membranes and utilize ATP hydrolysis to transport a wide variety of substrates across the plasma membrane. It should be noted that the terms "ABC1" and "ABCA1" both refer to the same ATP-binding cassette protein. The term "ABCA1" was introduced in 1999 by a nomenclature committee and has received limited acceptance in the field. To date, more than 30 members of this family have been identified in the human genome. These homologous proteins contain channel-like structures through which molecules are transported through the cell membrane and one or two domains which bind to ATP to couple energy generating ATP-hydrolysis to transport. Family members include the multidrug resistance factors (MDR/P-glycoproteins; Chen et al., Cell, 47: 381-389 (1986); Stride et al., Mol. Pharmacol., 49:962-971 (1996)), transporters associated with antigen presentation (Neefjes et al., Science, 261:769-771 (1993); Shepherd et al., Cell, 74:577-584 (1993)), and the cystic fibrosis transmembrane conductance regulator (Chang et al., J. Biol. Chem., 269:18572-18575 (1994); Rommens et al., Science. 245: 1059-1065 (1989)). Members of the ABC transporter family are generally composed of 4 domains found within two symmetric halves that are linked by a long charged region and a highly hydrophobic segment. Each half contains a hydrophobic domain, containing 6 transmembrane segments and a hydrophilic nucleotide binding domain containing highly conserved Walker A and B sequence motifs typical of many ATPases (Hyde et al., Nature, 346:362-365 (1990); Luciani et al., Genomics', 21: 150-159 (1994)). The transporter activity is dependent on the interaction with ATP at the nucleotide binding domains and by regulation via phosphorylation of residues in the region linking the two symmetric halves (Becq et al., J. Biol. Chem., 272: 2695-2699 (1997)).

Several lines of evidence described herein identified ABC1 as a pivotal protein in the apolipoprotein-mediated mobilization of intracellular cholesterol stores. First, the studies presented herein showed that ABC1 is defective in Tangier disease, a genetic disorder characterized by abnormal HDL-cholesterol metabolism. As shown previously, and herein at Example 1, the genetic defect in Tangier disease causes a defect in the pathway of apolipoprotein mediated efflux of cholesterol from within cells, resulting in significantly decreased cholesterol efflux activity and low HDL-cholesterol levels (Oram et al., J. Lipid Res., 37:2743-2491 (1996); Francis et al., J. Clin. Invest., 96: 78-87 (1995)). Genetic linkage analysis of families with Tangier disease assigned the defective gene to an interval on chromosome 9q31 (Rust et al., Nature Genetics, 20: 96-98 (1998)). A search of public databases revealed that the ABC1 gene was localized to chromosome 9q22-9q31, which is broader than, but includes the interval revealed in Rust et al. (Luciani et al., *supra* (1994)). Based on that data, radiation hybrid mapping of the human ABC1 gene was performed, which placed the gene between two markers squarely within the 7-cM region of human chromosome 9q31 reported by Rust et al. In addition, as shown in Example 2, microarray analysis revealed that the ABC1 gene is 2.5-fold underexpressed in Tangier patient cells as compared with normal cells. These studies identified the defective gene in Tangier disease as ABC1. In addition, further studies presented herein linked ABC1 activity to cholesterol efflux activity. First, studies showed that inhibitors of ABC1 transport activity, such as 4,4-diisothiocyanostilbene-2,2'-disulfonic acid (DIDS) and sulphobromophtaleine (BSP), also inhibited apoAI-mediated cholesterol efflux from fibroblast cells (see Example 6). Also, inhibition of ABC1 gene expression, using an antisense ABC1 oligonucleotide, was shown to inhibit apoAI-mediated cholesterol efflux from fibroblast cells (Example 7). In contrast, transfection studies, in which the ABC1 gene was transfected into mousse monocyte cells, showed that overexpression of ABC1 results in an increase in apoAI-mediated efflux (Example 8). Finally, RT-PCR performed using wildtype and Tangier patient mRNA revealed that ABC1 mRNA expression is regulated by cellular conditions related to cholesterol efflux in normal skin fibroblast cells, but not in Tangier patient fibroblasts (Example 9). Based on these findings, it was determined that ABC1 plays a major role in cholesterol efflux.

It is postulated that ABC1 plays a role in the translocation of intracellular cholesterol to the outer leaflet of the plasma membrane. Deficient transport of intracellular cholesterol due to a lack of ABC1 or defective ABC1 results in a lack of cholesterol in specific membrane domains with which apoAI and other apolipoproteins specifically interact (Stangl et al., J. Biol. Chem., 273: 31002-31008 (1998); Babitt et al., J. Biol. Chem., 272: 13242-13249 (1997))). The failed delivery of cholesterol to apoAI leads to the formation of cholesterol-deficient HDL particles that are rapidly removed from the plasma. (Bojanovski et al., J. Clin. Invest., 80: 1742-1747 (1987)).

### Definitions

The following definitions are provided to facilitate understanding of certain terms used throughout this specification.

In the present invention, "isolated" refers to material removed from its original environment (e.g., the natural environment if it is naturally occurring), and thus is altered "by the hand of man" from its natural state. For example, an isolated polynucleotide could be part of a vector or a composition of matter, or could be contained within a cell, and still be "isolated" because that vector, composition of matter, or particular cell is not the original environment of the polynucleotide.

As used herein, the term "polynucleotide(s)" is defined to encompass DNA and RNA of both synthetic and natural origin. The polynucleotide may exist as single- or double-stranded DNA or RNA, or an RNA/DNA heteroduplex. Thus, the polynucleotide of the present invention can be composed of any polyribonucleotide or polydeoxribonucleotide, which may be unmodified RNA or DNA or modified RNA or DNA. For example, polynucleotides can be composed of single- and double-stranded DNA, DNA that is a mixture of single- and double-stranded regions, or single, double-, and triple-stranded regions, single- and double-stranded RNA, and RNA that is mixture of single- and double-stranded regions, hybrid molecules comprising DNA and RNA that may be single-stranded or, more typically, double-stranded or triple-stranded, or a mixture of single- and double-stranded regions. In addition, the polynucleotide can be composed of triple-stranded regions comprising RNA or DNA or both RNA and DNA. A polynucleotide may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms of polynucleotides. The term "polynucleotide(s)" also embraces short polynucleotides often referred to as oligonucleotide(s).

The term "polypeptide(s)" refers to any peptide or protein comprising two or more amino acids joined to each other by peptide bonds or modified peptide bonds. "Polypeptide" refers to both short amino acid sequences, commonly referred to as peptides, as well as longer amino acid sequences, generally referred to as proteins. The polypeptide may contain amino acids other than the 20 gene encoded amino acids. Moreover, the polypeptide may be modified either by natural processes, such as processing and other post-translational modifications, or by chemical modification techniques, which are well-known in the art. A given polypeptide may contain many types of modifications. Also, the same type of modification may be present in the same or varying degree at one or more sites in the polypeptide. Modifications may occur anywhere in the polypeptide, including the peptide backbone, the amino acid side-chains, and the amino or carboxyl termini. Modifications include, but are not limited to, acetylation, acylation, ADP-ribosylation, amidation, formylation, gamma-carboxylation, glycosylation, hydroxylation, iodination, methylation, myristoylation, oxidation, phosphorylation, prenylation, sulfation, and selenoylation, as well as the covalent attachment of a nucleotide or nucleotide derivative, a lipid or lipid derivative, or a phosphotidylinositol. Other modifications include cross-linking, cyclization, formation of pyroglutamate, GPI anchor formation, proteolytic processing, racemization, and t-RNA-mediated addition of amino acids, such as arginylation and ubiquitination. See, for example, Proteins- Structure and Molecular Properties, 2nd Ed., T.E. Creighton, W.H. Freedman and Co., New York (1993); Wold, F., Posttranslational Protein Modification: Perspectives and Prospects, in Posttranslational Covalent Modification of Proteins, B. C. Johnson, Ed., Academic Press, New York (1983); Seifter et al., Meth. Enzymol., 182: 626-646 (1990); and Rattan et al., Protein Synthesis: Posttranslational Modifications and Aging, Ann. N.Y. Acad. Sci., 663: 48-62 (1992)). The polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

As used herein, the term "complementary" refers to the hybridization or base pairing between nucleotides, such as, for example, between the two strands of a double-stranded polynucleotide or between an oligonucleotide primer and a primer binding site on a single-stranded polynucleotide to be amplified or sequenced. Two single-stranded nucleotide molecules are said to be complementary when the nucleotides of one strand, optimally aligned with appropriate nucleotide insertions, deletions or substitutions, pair with at least about 80% of the nucleotides of the other strand.

"Identity", as known in the art, is a relationship between two or more polypeptide sequences or two or more polynucleotide sequences, as determined by comparing the sequences. "Identity" or "similarity" also has an art-recognized meaning that refers to the degree of sequence relatedness between polypeptide or polynucleotide sequences, as determined by the match between strings of such sequences. "Identity"and "similarity" can be calculated using a number of well known methods, including those published in Computational Molecular Biology, Lesk, A.M., ed., Oxford University Press, New York, (1988); Biocomputing: Informatics and Genome Projects, Smith, D.W., ed., Academic Press, New York, (1993); Computer Analysis of Sequence Data, Part I, Griffin, A.M., and Griffin, H.G., eds., Humana Press, New Jersey, (1994); Sequence Analysis in Molecular Biology, von Heinje, G., Academic Press, (1987); and Sequence Analysis Primer, Gribskov, M. and Devereux, J., eds., M Stockton Press, New York, (1991); and Carillo, H., and Lipton, D., SIAM J Applied Math 48:1073 (1988)). Methods commonly employed to determine identity or similarity between two sequences include, but are not limited to, those disclosed in "Guide to Huge Computers," Martin J. Bishop, ed., Academic Press, San Diego, (1994), and Carillo, H., and Lipton, D., SIAM J Applied Math 48:1073 (1988). Preferred methods to determine identity are designed to give the largest match between the sequences tested. Methods for aligning polynucleotides or polypeptides are codified in computer programs, including the GCG program package (Devereux, J., et al., Nucleic Acids Research (1984) 12(1):387 (1984)), BLASTP, BLASTN, FASTA (Atschul, S.F. et al., J. Molec. Biol. 215:403 (1990), Bestfit program (Wisconsin Sequence Analysis Package, Version 8 for Unix, Genetics Computer Group, University Research Park, 575 Science Drive, Madison, WI 53711 (using the local homology algorithm of Smith and Waterman, Advances in Applied Mathematics 2:482-489 (1981)).

When using any of the sequence alignment programs to determine whether a particular sequence is, for instance, 90% identical to a reference sequence, the parameters are set so that the percentage of identity is calculated over the full length of the reference polypeptide or polynucleotide and that gaps in identity of up to 10% of the total number of nucleotides in the reference polynucleotide are allowed.

A preferred method for determining the best overall match between a query sequence (a sequence of the present invention) and a subject sequence, also referred to as a global sequence alignment, can be determined using the FASTDB computer program based on the algorithm of Brutlag et al. (Comp. App. Biosci. 6:237-245 (1990)). The term "sequence" includes nucleotide and amino acid sequences. In a sequence alignment the query and subject sequences are either both nucleotide sequences or both amino acid sequences. The result of said global sequence alignment is in percent identity. Preferred parameters used in a FASTDB search of a DNA sequence to calculate percent identity are: Matrix=Unitary, k-tuple=4, Mismatch Penalty=1, Joining Penalty=30, Randomization Group Length=0, and Cutoff Score=1, Gap Penalty=5, Gap Size Penalty 0.05, and Window Size=500 or query sequence length in nucleotide bases, whichever is shorter. Preferred parameters employed to calculate percent identity and similarity of an amino acid alignment are: Matrix=PAM 150, k-tuple=2, Mismatch Penalty= 1, Joining Penalty=20, Randomization Group Length=0, Cutoff Score=1, Gap Penalty=5, Gap Size Penalty=0.05, and Window Size=500 or query sequence length in amino acid residues, whichever is shorter.

As an illustration, a polynucleotide having a nucleotide sequence of at least 90% "identity" to a sequence contained in SEQ ID NO: 1 means that the nucleotide sequence of the polynucleotide is identical to a sequence contained in SEQ ID NO: 1 except that the polynucleotide sequence may include up to ten point mutations per each 100 nucleotides of the total length of SEQ ID NO: 1. In other words, to obtain a polynucleotide comprising a nucleotide sequence that has at least 90% identity to SEQ ID NO: 1, up to 10% of the nucleotides in the sequence contained in SEQ ID NO: 1 can be deleted, inserted, or substituted with other nucleotides. These changes may occur anywhere throughout the polynucleotide, and may be interspersed either individually among nucleotides or in one or more contiguous group within SEQ ID NO: 1.

Similarly, a polypeptide having an amino acid sequence of at least 98% "identity" to a sequence contained in SEQ ID NO: 2 means that the amino acid sequence of the polypeptide is identical to a sequence contained in SEQ ID NO: 2 except that the polypeptide sequence may include up to 2 amino acid alterations per each 100 amino acids of the total length of SEQ ID NO: 2. In other words, to obtain a polypeptide having an amino acid sequence at least 98% identical to SEQ ID NO: 2, up to 2% of the amino acid residues in the sequence contained in SEQ ID NO: 2 can be deleted, inserted, or substituted with other amino acid residues. These changes may occur anywhere throughout the polypeptide, and may be interspersed either individually among residues or in one or more contiguous groups within SEQ ID NO: 2.

"A polypeptide having biological activity" refers to a polypeptide exhibiting activity similar, but not necessarily identical, to an activity of a polypeptide described herein (e.g. cholesterol transport activity), as measured in a particular biological assay, with or without dose dependency. In the case where dose dependency does exist, it need not be identical to that of the polypeptide, but rather substantially similar to the dose-dependence in a given activity as compared to the polypeptide described herein (i.e., the candidate polypeptide will exhibit greater activity or not more than about 25-fold less and, preferably, not more than about tenfold less activity, and most preferably, not more than about three-fold less activity relative to the polypeptide of the present invention.).

"Polypeptide variant" refers to a polypeptide differing from the ABC1 polypeptide, but retaining essential properties thereof. Generally, variants are overall closely similar, and, in many regions, identical to the polypeptide comprising SEQ ID NO: 2. Preferably, the polypeptide variant retains its biological activity, i.e., cholesterol transport activity. Variants include, but are not limited to, splice variants and allelic variants, as well as addition, deletion, and substitution variants.

Likewise, "polynucleotide variant" refers to a polynucleotide differing from the polynucleotide of the present invention, but retaining essential properties thereof. The variants may contain alterations in the coding regions, non-coding regions, or both. Thus, for example, an ABC1 polynucleotide variant has a nucleotide sequence that differs from that of SEQ ID NO: 1, but encodes a polypeptide that has cholesterol transport activity. Also, for example, a polynucleotide variant has a nucleotide sequence that differs from that of SEQ ID NO: 3, but retains promoter activity. Especially preferred are polynucleotide variants containing alterations that produce silent substitutions, additions, or deletions, but do not alter the properties or activities of the encoded polypeptide. Nucleotide variants produced by silent substitutions due to the degeneracy of the genetic code are preferred. Moreover, variants in which 10-20, 5-10, 1-5, or 1-2 amino acids are substituted, deleted, or added in any combination are also preferred. Polynucleotide variants can be produced for a variety of reasons, e.g., to optimize codon expression for a particular host (e.g. changing codons in the human mRNA to those preferred by a bacterial host such as *E. coli).*

"Allelic variants" are naturally-occurring variants that refer to one of several alternate forms of a gene occupying a given locus on a chromosome of an organism. (Genes II, Lewin, B., ed., John Wiley & Sons, New York (1985).) These allelic variants can vary at either the polynucleotide and/or polypeptide level. Alternatively, non-naturally occurring variants may be produced by mutagenesis techniques or by direct synthesis.

The term "conservative amino acid substitution" refers to a substitution of a native amino acid residue with a nonnative residue such that there is little or no effect on the polarity or charge of the amino acid residue at that position. For example, a conservative substitution results from the replacement of a non-polar residue in a polypeptide with any other non-polar residue. Another example of a conservative substitution is the replacement of an acidic residue with another acidic residue. Conservative substitutions are expected to produce ABC1 polypeptides having functional and chemical characteristics similar to those of the naturally-occurring ABC1 polypeptide.

The term "ortholog" refers to a polypeptide that corresponds to a polypeptide identified from a different species. For example, mouse and human ABC1 polypeptides are considered orthologs.

The term "vector" is used to refer to any molecule (e.g. nucleic acid, plasmid, or virus) used to transfer coding information to a host cell.

The term "expression vector" refers to a vector that is suitable for transformation of a host cell and contains nucleic acid sequences that direct and/or control the expression of inserted heterologous nucleic acid sequences. Expression includes, but is not limited to, processes such as transcription, translation, and RNA splicing, if introns are present.

As used herein, the term "transcriptional regulatory region" or "expression modulating portion" refers to any region of the gene, including, but not limited to, promoters, enhancers, and repressors.

As used herein, the term "promoter" refers to an untranscribed sequence located upstream (*i.e.*, 5') to the start codon of a structural gene (generally within about 100 to 1000 bp) that controls the transcription of the structural gene.

As used herein the term "enhancers" refers to *cis*-acting elements of DNA, usually about 10-300 bp in length, that act on the promoter to increase transcription. Enhancers are relatively orientation and position independent. They have been found 5' and 3' to the transcription unit.

"Host cell" is a cell that has been transformed or transfected, or is capable of transformation or transfection by an exogenous polynucleotide sequence. The term includes the progeny of the parent cell, whether or not the progeny is identical in morphology or in genetic make-up to the original parent, so long as the selected gene is present.

The term "operatively linked" is used herein to refer to an arrangement of flanking sequences wherein the flanking sequences so described are configured or assembled so as to perform their usual function. Thus, a flanking sequence operably linked to a coding sequence may be capable of effecting the replication, transcription and/or translation of the coding sequence. For example, a coding sequence is operably linked to a promoter when the promoter is capable of directing transcription of that coding sequence. A flanking sequence need not be contiguous with the coding sequence, so long as it functions correctly. Thus, for example, intervening untranslated yet transcribed sequences can be present between a promoter sequence and the coding sequence and the promoter sequence can still be considered "operably linked" to the coding sequence.

The term "transfection" is used to refer to the uptake of foreign or exogenous DNA by a cell, and a cell has been "transfected" when the exogenous DNA has been introduced inside the cell membrane. A number of transfection techniques are well known in the art and are disclosed herein. *See, e.g.,* Graham et al., 1973, Virology 52:456; Sambrook et al., Molecular Cloning, A Laboratory Manual (Cold Spring Harbor Laboratories, 1989); Davis et al., Basic Methods in Molecular Biology (Elsevier, 1986); and Chu et al., 1981, Gene 13:197. Such techniques can be used to introduce one or more exogenous DNA moieties into suitable host cells.

### ABC1 Polypeptides

The human ABC1 polypeptide described herein comprises the amino acid sequence shown in SEQ ID NO: 2. In contrast to the human ABC1 protein reported by others, the ABC1 polypeptide shown in SEQ ID NO: 2 has an additional 60 amino acids at the amino terminus, revealing an ABC1 protein of 2261 amino acids rather than 2201 amino acids (see Langmann et al. in Biochem. Biophys. Res. Comm., 257, 29-33 (1999)). In addition, the ABC1 polypeptide shown in SEQ ID NO: 2 differs from other reported sequences at several amino acid residues. Specifically, the present ABC1 polypeptide shown in SEQ ID NO: 2 has a K for R at residue 159, I for V at 765, M for I at 823, I for T at 1495, L for P at 1588, K for R at 1914, and L for P at 2108. To remain consistent with published notation, the above amino acid numbers are those of Lawn et al., *J. Clin. Invest*., 104: R25-31 (1999), rather than those of SEQ ID NO: 2. As discussed in further detail below, the sequence difference likely arises from the fact that the first ABC1 cDNA was cloned from mouse using a PCR-based strategy and the subsequently reported human ABC1 cDNA sequences were predicted from the sequence of the mouse protein. The ABC1 protein has an approximate molecular weight of 240 kD as determined by SDS-PAGE.

### ABC1 Polynucleotides

The present invention provides, for example, isolated polynucleotides containing non-coding 5' sequences of ABC1.

In contrast to the published sequence of Langmann et al. which codes for a protein of 2201 amino acids based on a predicted start methionine found in exon 3 (Langmann et al., Biochem. Biophys. Res. Comm., 257: 29-33 (1999) (GenBank Accession No. AJO12376), the nucleotide sequence encoding the polypeptide comprising SEQ ID NO: 2 contains 50 exons and codes for a protein of 2261 amino acids (see Figure 4). The corresponding nucleotide sequence contains a coding sequence that includes an additional 180 nucleotides at the 5' end corresponding to the following 60 amino-terminal amino acids:
MACWPQLRLLLWKNLTFRRRQTCQLLLEVAWPLFIFLILISVRLSYPPYEQHECHFPNKA.
Given that there is an in-frame stop codon 6 to 9 nucleotides upstream from this location, the newly predicted start site is the first methionine codon that could produce a continuous open reading frame. Alignment of this new ABC1 cDNA sequence with related ABC transporter sequences ABCR and ABC-C (also known as ABC3) which also contain open reading frames for the 60 additional amino acids, indicates a high degree of similarity, implying that the homologous ABC transporter proteins begin with sequences related to the amino terminal extension sequence proposed for human ABC1. It is likely that the earlier published start site of the human ABC1 was predicted from the published mouse ABC1 cDNA sequence (Luciani et al., Genomics, 21150-159 (1994); GenBank Accession no.: X75926) which contains an extra nucleotide "n" in the extension region such that the newly disclosed methionine is not in-frame. However, if the "n" nucleotide in the mouse sequence is ignored, the mouse and human sequences of the extension region are identical. In light of these results, it is likely that the full length human ABC1 protein contains 2261 amino acids rather than 2201 amino acids, as previously suggested by Langmann et al. and others. Accordingly, Langmann et al. do not present the full open reading frame of human ABC1.

The 10.4 kb human ABC1 cDNA sequence shown in SEQ ID NO: 1 contains an open reading frame of 6783 nucleotides plus 5' and 3' untranslated regions. There is a start codon at position 291 and a stop codon at position 7074. The present ABC1 cDNA shown in SEQ ID NO: 1 differs from the published ABC1 cDNA (GenBank Accession No. AJO12376) in several respects. First, the present ABC1 cDNA includes an additional 350 nucleotides at the 5' end and an additional 3136 nucleotides at the 3' end (not including the poly(A) tail). The present ABC1 sequence also differs from the published ABC1 cDNA by the substitution of 10 nucleotides in the coding region. Of the ten differences, seven nucleotide differences predict amino acid changes. To remain consistent with published notation, the following nucleotide and amino acid numbers are those of Lawn et al., *J. Clin. Invest.,* 104: R25-31 (1999) and GenBank Accession No. AJO12376, rather than those of SEQ ID NO: 1. The nucleotide and amino acid changes are as follows: (1) A for G at nucleotide 414; (2) A for G at nucleotide 596 (K for R at amino acid 159); (3) T for C at nucleotide 705; (4) A for C at nucleotide 1980; (5) A for G at 2413 (I for V at amino acid 765); (6) G for A at 2589 (M for I at amino acid 823); (7) T for C at 4604 (I for T at amino acid 1495); (8) T for C at 4883 (L for P at amino acid 1588); (9) A for G at 5861 (K for R at amino acid 1914); and (10) T for C at 6443 (L for P at amino acid 2108). Five of the amino acid changes are conservative amino acid changes and may represent polymorphisms or sequence errors. In two instances, the present sequence predicts important amino acid differences from the GenBank sequence. The differences result in a leucine rather than a proline at residue 1588 and at residue 2108. Interestingly, at both positions, the predicted leucine was also found in each of the three TD samples analyzed as well as the highly conserved mouse ABC1 protein sequence.

The nucleotide sequences set forth in SEQ ID NO: 7 and SEQ ID NO: 9 encode mutant ABC1 polypeptides from Tangier patients, i.e., the polypeptides of SEQ ID NO: 8 (isolated from patient TD1) and SEQ ID NO: 10 (isolated from Tangier patient TD2). The nucleotide sequence set forth in SEQ ID NO: 7 contains the full open reading frame, as well as 5' and 3' flanking sequences. The open reading frame encodes a polypeptide of 2261 amino acids, containing, among other substitutions, a nucleotide substitution that results in an A to G substitution at position 537 (using the numbering of Lawn et al., *supra* (1999)).

The nucleotide sequence set forth in SEQ ID NO: 9 contains the full open reading frame, as well as 5' and 3' flanking sequences. The open reading frame encodes a polypeptide of 2261 amino acids, containing, among other substitutions, a polynucleotide substitution that results in an Arg to Tryp substitution at residue 527 (using the numbering of Lawn et al., *supra* (1999)).

One aspect of the present invention relates to isolated polynucleotides that have the sequence of the non-coding 5' flanking region of ABC1. In one embodiment, the isolated polynucleotide has the sequence shown in SEQ ID NO: 3. As demonstrated by heterologous reporter assays, discussed in Example 15, the polynucleotide set forth in SEQ ID NO: 3 contains the transcriptional regulatory region of the ABC1 gene. As shown in Figure 13, the polynucleotide set forth in SEQ ID NO: 3 is a 1643 b.p. non-coding sequence that contains several transcription regulatory elements, including a TATA box at positions 1522, 1435, and 1383, as well as transcription factor binding sites, including several putative SP1 sites, and several nuclear receptor half sites. In addition, an identified sterol response element is found at position 1483-1500. Further heterologous reporter assays described in Example 17 revealed that several discrete portions of SEQ ID NO: 3 retained promoter activity. Accordingly, in another preferred embodiment, the polynucleotide has the sequence identified as nucleotides 1-1532, 1080-1643, 1181-1643, 1292-1643, 1394-1532, or 1394-1343 of SEQ ID NO: 3. In an especially preferred embodiment, the polynucleotide has the sequence identified as nucleotides 1394-1532 of SEQ ID NO: 3, which sequence has been shown to have ABC1 promoter activity (see Example 17). The polynucleotide comprising nucleotides 1480-1510 of SEQ ID NO: 3 is shown to regulate the ABC1 transcriptional response to LXR ligands.

Several 3' untranslated regions have been identified which may represent alternate sites of polyadenylation of the ABC1 transcript. For example, the full length 3' UTR (SEQ ID NO: 6) contains 46 sequences (AA)nCU/UC(AA)n which have been shown to be necessary for binding of Vigilin. Vigilin, a ubiquitous protein with 14K homology domains, is the estrogen-inducible vitellogenin mRNA 3'-untranslated region binding protein (J. Biol. Chem., 272: 12249-12252 (1997)). In addition to binding HDL, Vigilin has been shown to bind to the 3' flanking region of mRNAs and to increase the half-life of the mRNA transcript (Mol. Cell. Biol., 18:3991-4003 (1998)). Thus, the 3' flanking region could be altered, for example, to increase the binding of Vigilin, thereby increasing the half-life of the ABC1 mRNA. Other examples of 3' untranslated regions are the sequence shown in SEQ ID NO: 4 and the sequence shown in SEQ ID NO: 5.

The present invention also includes related ABC1 5' flanking polynucleotides. Accordingly, the invention relates to polynucleotides having nucleotide sequences that have at least 80% identity over their entire length to the polynucleotide having the sequence identified as SEQ ID NO: 3, or the polynucleotide having the sequence identified as comprising nucleotides 1-1532, 1080-1643, 1181-1643, 1292-1643, 1394-1532, or 1394-1643 of SEQ ID NO: 3. Preferably, the polynucleotide has at least 80%, more preferably at least 90%, even more preferably at least 95%, and most preferably 100% identity over its entire length to any one of the aforementioned flanking polynucleotides. Preferred polynucleotides include those polynucleotides that possess biological activity, such as transcriptional regulatory activity.

It is understood that the present invention further relates to isolated polynucleotides that are complementary to any one of the above-described polynucleotide sequences. As used herein, the term "complementary" refers to the hybridization or base pairing between nucleotides, such as, for example, between the two strands of a double-stranded polynucleotide. Two single-stranded nucleotide molecules are said to be complementary when the nucleotides of one strand, optimally aligned with appropriate nucleotide insertions, deletions or substitutions, pair with at least about 80% of the nucleotides of the other strand.

Another aspect of the present invention relates to compositions comprising the novel ABC1 polynucleotides described above and a suitable carrier.

In one embodiment, the composition comprises a polynucleotide having the sequence identified as SEQ ID NO: 3 or a polynucleotide having the sequence identified as nucleotides 1-1532, 1080-1643, 1181-1643, 1292-1643, 1394-1532, or 1394-1643 of SEQ ID NO: 3 and a suitable carrier. Also preferably, the composition comprises a polynucleotide having at least 80%, 90%, or 95% identity over its entire length to the polynucleotide comprising any one of the described 5' flanking sequences and a suitable carrier.

In addition, a composition of the present invention may comprise, in any combination, two or more of the above-described ABC1 polynucleotides and a suitable carrier. Any suitable aqueous carrier can be used in the composition. Preferably, the carrier renders the composition stable at a desired temperature, such as room temperature or storage temperature (i.e. 4°C to -20°C), and is of approximately neutral pH. Examples of suitable carriers are known to those of skill in the art and include Tris-EDTA buffer and DEPC-H₂O.

### ABC1 Vectors and Host Cells

The present invention also relates to recombinant vectors that comprise one or more of the above-described ABC1 polynucleotides, and host cells that are genetically engineered with the vectors comprising ABC1 polynucleotides. As mentioned, the invention provides recombinant vectors that comprise one or more of the above-described wild-type ABC1 polynucleotides.

An isolated ABC1 polynucleotide is inserted into a vector using well-known ligation and cloning techniques. Cloning techniques have been described in several standard laboratory manuals, including Davis et al., Basic Methods in Molecular Biology (1986); Sambrook et al., Molecular Cloning: A Laboratory Manual, 2nd Ed., (Cold Spring Harbor Press, Cold Spring Harbor, N.Y. (1989)); Ausubel et al. eds., Current Protocols in Molecular Biology, (Wiley and Sons (1994)); Goeddel, ed., Gene Expression Technology (Methods in Enzymology (1991)); Murray, ed., Gene Transfer and Expression Protocols (Human Press, Clifton, NJ).

Any vector suitable for ABC1 polynucleotide insertion can be used. The vector is typically selected to be functional in the particular host cell employed (i.e., the vector is compatible with the host cell machinery such that amplification and/or expression of the gene can occur). Preferably, the vector is compatible with bacterial, insect, or mammalian host cells. Also preferably, the vector is an expression vector (for a review of expression vectors, see Goeddel, D.V. ed., Methods Enzymol., Academic Press Inc., San Diego, CA (1990)). The vector may be, for example, a phage, plasmid, viral, or retroviral vector. Retroviral vectors may be replication competent or replication defective. In the latter case, viral propagation generally will occur only in complementing host cells.

Typically, expression vectors used in any of the host cells will contain sequences for plasmid maintenance and for cloning and expression of exogenous nucleotide sequences. Such sequences, collectively referred to as "flanking sequences" should preferably include one or more of the following nucleotide sequences: a promoter, one or more enhancer sequences, an origin of replication, a transcriptional termination sequence, a complete intron sequence containing a donor and acceptor splice site, a sequence encoding a leader sequence for polypeptide secretion, a ribosome binding site, a polyadenylation sequence, a polylinker region for inserting the nucleic acid encoding the polypeptide to be expressed, and a selectable marker element.

The flanking sequences may be homologous (*i.e.*, from the same species and/or strain as the host cell), heterologous (*i.e.*, from a species other than the host cell species or strain), hybrid (*i.e.*, a combination of flanking sequences from more than one source), or synthetic. Also, the flanking sequences may be native sequences which normally function to regulate ABC1 polypeptide expression. The source of a flanking sequence may be any prokaryotic or eukaryotic organism, any vertebrate or invertebrate organism, or any plant, provided that the flanking sequence is functional in, and can be activated by, the host cell machinery.

The vector should also preferably include at least one selectable marker for propagation in a host. A selectable marker is a gene element that encodes a protein necessary for the survival and growth of a host cell grown in a selective culture medium. Suitable selection marker genes encode proteins that (a) confer resistance to antibiotics or other toxins, *e.g.*, ampicillin, tetracycline, or kanamycin for prokaryotic host cells; (b) complement auxotrophic deficiencies of the cell; or (c) supply critical nutrients not available from complex media. Preferred selectable markers include zeocin, G418, hygromycin, or neomycin resistence for eukaryotic cell culture and tetracycline, kanamycin, or ampicillin resistance for culturing in *E. coli* and other bacteria.

Other suitable selection genes include those that are used to amplify the expressed gene. Amplification is the process wherein genes that are in greater demand for the production of a protein critical for growth are reiterated in tandem within the chromosomes of successive generations of recombinant cells. Examples of suitable selectable markers for mammalian cells include dihydrofolate reductase (DHFR) and thymidine kinase. The mammalian cell transformants are placed under selection pressure wherein only the transformants are uniquely adapted to survive by virtue of the selection gene present in the vector. Selection pressure is imposed by culturing the transformed cells under conditions in which the concentration of selection agent in the medium is successively changed, thereby leading to the amplification of both the selection gene and the ABC1 gene. As a result, increased quantities of ABC1 polypeptide are synthesized from the amplified DNA.

The vector should also preferably contain a transcription termination sequence, which is typically located 3' of the end of a polypeptide coding region and serves to terminate transcription. Usually, the transcription termination sequence in prokaryote cells is a G-C rich fragment followed by a poly T sequence. The sequence can be purchased as part of a commercial vector or synthesized using well-known methods for nucleic acid synthesis.

The vector should also preferably contain a ribosome binding site, which is usually necessary for translation initiation of mRNA and is characterized by a Shine-Dalgarno sequence (prokaryotes) or a Kozak sequence (eukaryotes). The element is typically located 3' to the promoter and 5' to the coding sequence of the ABC1 polypeptide to be expressed. The Shine-Dalgarno sequence is varied but is typically a polypurine (*i.e.*, having a high A-G content). Many Shine-Dalgamo sequences have been identified, each of which can be readily synthesized using well-known methods.

The vector should also preferably contain a promoter that is recognized by the host organism and operably linked to the encoded polynucleotide. The promoter can be an inducible promoter or a constitutive promoter. Inducible promoters initiate increased levels of transcription from DNA under their control in response to some change in culture conditions, such as the presence or absence of a nutrient or a change in temperature. In contrast, constitutive promoters initiate continual gene product production; consequently there is little or no control over gene expression. A suitable promoter is operably linked to a polynucleotide, by removing the promoter from the source DNA by restriction enzyme digestion and inserting the desired promoter sequence into the vector. As mentioned, a native promoter can be used to direct amplification and/or expression of the polynucleotide. Thus, the recombinant vector can comprise any one of the above-described wild-type, variant, or mutant ABC1 polynulceotides and an ABC1 promoter, such as that found in SEQ ID NO: 3. The recombinant vector can also comprise, in any combination, two or more of the above-described wild-type, variant, or mutant ABC1 polynulceotides and an ABC1 promoter.

Preferably, a heterologous promoter is used if it permits greater transcription and higher yields of ABC1 protein as compared to the native ABC1 promoter, and if it is compatible with the host cell system that has been selected for use. The heterologous promoter can be used alone or in conjunction with the native ABC1 promoter. Thus, in one preferred embodiment, the recombinant vector comprises any one of the above-described wild-type ABC1 polynucleotides and a heterologous promoter. In another preferred embodiment, the recombinant vector comprises any one of the above-described variant ABC polynucleotides and a heterologous promoter. In yet another preferred embodiment, the recombinant vector comprises any one of the above-described mutant ABC1 polynucleotides and a heterologous promoter. Preferred embodiments also include recombinant vectors that contain any combination of two or more of the above-described wild-type, variant, and mutant ABC1 polynucleotides and a heterologous promoter.

Heterologous promoters suitable for use with prokaryotic hosts include, but are not limited to, the beta-lactamase and lactose promoter systems (Villa-Kamaroff et al., 1978, Proc. Natl. Acad. Sci. U.S.A., 75:3727-31), alkaline phosphatase, a tryptophan (trp) promoter system, and hybrid promoters such as the tac promoter (Villa-Kamaroff et al., 1978, Proc. Natl. Acad. Sci: U.S.A., 75:3727-31). Their sequences have been published, thereby enabling one skilled in the art to ligate them to the desired DNA sequence, using linkers or adapters as needed to supply any useful restriction sites. Other suitable heterologous promoters will be known to those skilled in the art.

Suitable heterologous promoters for use with mammalian host cells are also well known and include, but are not limited to, those obtained from the genomes of viruses such as polyoma virus, fowlpox virus, adenovirus (such as Adenovirus 2), bovine papilloma virus, avian sarcoma virus, cytomegalovirus, retroviruses, hepatitis-B virus and Simian Virus 40 (SV40). Other suitable mammalian promoters include heterologous mammalian promoters, for example, heat-shock promoters and the actin promoter. Additional suitable promoters include, but are not limited to: the SV40 early promoter and late promoter region (Bernoist and Chambon, 1981, Nature 290:304-10); the promoter contained in the 3' long terminal repeat of Rous sarcoma virus (Yamamoto, et al., 1980, Cell 22:787-97); the herpes thymidine kinase promoter (Wagner et al., 1981, Proc. Natl. Acad. Sci. U.SA. 78:1444-45); and the regulatory sequences of the metallothionine gene (Brinster et al., 1982, Nature 296:39-42). Preferably, the promoter is a cytomegalovirus or SV40 promoter. Thus, in especially preferred embodiments, the recombinant vector comprises one of the above-described wild-type ABC1 polynucleotides, one of the above-described variant ABC1 polynucleotides, or one of the above-described mutant ABC1 polynucleotides and a cytomegalovirus promoter. In another especially preferred embodiment, the recombinant vector comprises, in any combination, two or more of the above-described wild-type, variant, or mutant ABC1 polynulceotides and a cytomegalovirus promoter.

The vector also preferably contains an enhancer sequence to increase the transcription of a polynucleotide, such as ABC1. Suitable enhancers for the activation of eukaryotic promoters include viral enhancers, such as the SV40, cytomegalovirus early promoter, polyoma, and adenovirus enhancers.

Expression vectors of the invention may be constructed from a starting vector, such as a commercially available vector, which may or may not contain all of the desired flanking sequences. Where one or more of the flanking sequences described herein are not already present in the vector, they may be individually obtained and ligated into the vector. Methods used for obtaining each of the flanking sequences are well known to one skilled in the art.

Preferred vectors are those which are compatible with bacterial, insect, and mammalian host cells. Vectors preferred for use in bacteria include, for example, pQE70, pQE60, and pQE-9 (Quiagen, Inc.), pBluescript vectors, Phagescript vectors, pNH16A, pNH18A, pNH46A (Stratagene Cloning Systems, Inc.), ptrc99a, pKK223-3, pDR540, pRIT5 (Pharmacia Biotech, Inc.), and pCEP4 (Invitrogen Corp., Carlsbad, CA). Preferred eukaryotic vectors include, but are not limited to, pWLNEO, pSV2CAT, pOG44, pXTI and pSG (Stratagene), pSVK3, pBPV, pMSG, and pSVL (Pharmacia), and pGL3 (Promega, Madison, WI). Other suitable vectors will be readily apparent to the skilled artisan.

The recombinant vector pCEPhABC1, which is described in Example 4 and shown in Figure 3. comprises the plasmid pCEP4 (Invitrogen), an expression vector containing the cytomegalovirus promoter and enhancer. The vector pCEPhABC1 further comprises an ABC1 polynucleotide operatively linked to the heterologous cytomegalovirus promoter. The ABC1 polynucleotide comprises SEQ ID NO: 1, which contains the full-lenth ABC1 cDNA, including non-coding 5' flanking (i.e., native ABC1 promoter) and 3' flanking sequences.

In addition, the present invention provides recombinant vectors comprising ABC1 5' flanking sequence polynucleotides that contain promoter activity. Thus, in a preferred embodiment, the recombinant vector comprises the polynucleotide having the sequence identified as SEQ ID NO: 3. In another preferred embodiment, the recombinant vector comprises the polynucleotide having the sequence identified as nucleotides 1-1532, 1080-1643; 1181-1643, 1292-1643, 1394-1532, or 1394-1643 of SEQ ID NO: 3. In an especially preferred embodiment, the polynucleotide has the sequence identified as nucleotides 1394-1532 of SEQ ID NO: 3. In yet another embodiment, the polynucleotide has a nucleotide sequence that has at least 80%, more preferably at least 90%, and even more preferably at least 95% identity over its entire length to the polynucleotide having the sequence identified as SEQ ID NO: 3 or the polynucleotide having the sequence identified as nucleotides 1-1532, 1080-1643, 1181-1643, 1292-1643, 1394-1532 or 1394-1643 of SEQ ID NO: 3. The recombinant vector also comprises a polynucleotide that is complementary to any of the above-described 5' flanking sequences.

An isolated ABC1 flanking sequence polynucleotide, such as any of the above-described 5' flanking sequence polynucleotides, is inserted into a vector using well-known ligation and cloning techniques. Any of the previously described vectors can be used. Preferably, the vector is compatible with bacterial, insect, or mammalian host cells. Also preferably, the vector is an expression vector. The vector may be, for example, a phage, plasmid, viral, or retroviral vector.

In addition to the ABC1 flanking sequence, the vector may contain one or more of the following flanking nucleotide sequences: a promoter, one or more enhancer sequences, an origin of replication, a transcriptional termination sequence, a complete intron sequence containing a donor and acceptor splice site, a sequence encoding a leader sequence for polypeptide secretion, a ribosome binding site, a polyadenylation sequence, a polylinker region for inserting the nucleic acid encoding the polypeptide to be expressed, and a selectable marker element. Any of the previously described flanking nucleotide sequences are suitable. The flanking sequences may be homologous, heterologous, hybrid, or synthetic. Also, the flanking sequences may be native sequences which normally function to regulate ABC1 polypeptide expression. The source of a flanking sequence may be any prokaryotic or eukaryotic organism, any vertebrate or invertebrate organism, or any plant, provided that the flanking sequence is functional in, and can be activated by, the host cell machinery.

Preferred vectors are those which are compatible with bacterial, insect, and mammalian host cells. Suitable vectors have been previously described and include pQE70, pQE60, pQE9, pBluescript vectors, Phagescript vectors, pNH16A, pNH18A, pNH46A, ptrc99a, pKK223-3, pDR540, pRIT5, and pCEP4 for use in bacteria and pWLNEO, pSV2CAT, pOG44, pXTI, pSG, pSVK3, pBPV, pMSG, pSVL, and pGL3 for use in eukaryotic cells.

In one particularly preferred embodiment, the recombinant vector comprises a polynucleotide having the 5' flanking region of the ABC1 gene and further comprises at least one polynucleotide encoding a heterologous polypeptide. The heterologous polynucleotide is operatively linked to the ABC1 5' flanking sequence containing the ABC1 promoter. Thus, preferably, the 5' flanking sequence has the sequence set forth in SEQ ID NO: 3. Equally preferably, the 5' flanking sequence has nucleotides 1-1532, 1080-1643, 1181-1643, 1292-1643, 1394-1532 or 1394-1643 of SEQ ID NO: 3. The heterologous polynucleotide preferably encodes a polypeptide that is a complete protein or a biologically active fragment of a protein. The vector may also contain more than one heterologous polynucleotide. More preferably, the heterologous polynucleotide encodes a reporter protein. In such case, the recombinant vector preferably does not contain any additional promoter sequences. Examples of suitable reporter proteins include luciferase, β-galactosidase, chloramphenicol acetyl transferase, and green fluorescent protein. Preferably, the reporter polypeptide is luciferase. Thus, in one especially preferred embodiment, the recombinant vector comprises the 5' flanking sequence set forth in SEQ ID NO: 3 and a luciferase reporter polynucleotide. In other equally preferred embodiments, the recombinant vector comprises a polynucleotide comprising the polynucleotide identified as nucleotides 1-1532, 1080-1643, 1181-1643, 1292-1643, 1394-1532 or 1394-1643 of SEQ ID NO: 3 and a luciferase reporter polynucleotide.

Expression vectors comprising the ABC1 5' flanking sequence can be constructed from a starting vector, such as a commercially available vector, which contains a reporter polynucleotide. Examples of suitable expression vectors include pGL3-Basic, which contains a luciferase reporter gene (Promega, Madison, WI) and pβGa1-Basic (Clontech, Palo Alto, CA). A preferred vector is the pGL3-Basic luciferase reporter vector, which is promoterless; A 5' flanking sequence containing the ABC1 promoter, for example SEQ ID NO: 3, can be ligated into one of the above expression vectors using well-known methods, including the methods described herein (see Example 15). Thus, in an especially preferred embodiment, the recombinant vector is pAPR1, a reporter gene construct comprising SEQ ID NO: 3 and a luciferase reporter gene in a pGL3 vector (see Figure 11).

The present invention also relates to host cells comprising any one of the above-described recombinant vectors. After the vector has been constructed, the completed vector can be inserted into a suitable host cell for amplification and/or polypeptide expression. Host cells may be prokaryotic host cells (such as *E*. *coli*) or eukaryotic host cells (such as a yeast cell, an insect cell, or a vertebrate cell). The host cell, when cultured under appropriate conditions, synthesizes an ABC1 polypeptide or, alternatively, a reporter polypeptide, which can be subsequently measured. The host cell can be a mammalian host cell, such as a primate cell line or a rodent cell line, including transformed cell lines. Normal diploid cells, cell strains derived from *in vitro* culture of primary tissue, as well as primary explants, are also suitable. Candidate host cells may be genotypically deficient in the selection gene, or may contain a dominantly acting selection gene.

A number of suitable host cells are known in the art and many are available from the American Type Culture Collection (ATCC), Manassas, VA. Suitable mammalian host cells include, but are not limited to, chinese hamster ovary cells (CHO), CO DHFR- cells (Urlaub et al., 1980, Proc. Natl. Acad Sci., 97:4216-20), human embryonic kidney (HEK) 293 or 293T cells, or 3T3 cells, monkey COS-1 and COS-7 cell lines, and the CV-1 cell line. Other suitable mammalian cell lines include but are not limited to, mouse neuroblastoma N2A cells, HeLa cells, mouse 1-929 cells, 3T3 lines derived from Swiss, Balb-c or NIH mice, BHK or HAK hamster cell lines, Thp-1, HepG2, and mouse RAW cell lines. Each of these cell lines is known by and available to those skilled in the art of protein expression. A preferred host cell is the mouse monocytic cell line RAW 264.7, which use is described in Example 8.

Suitable bacterial host cells include various strains of *E*. *coli* (e.g., HB101, DH50, DH10, and MC1061), which are well-known as host cells in the field of biotechnology, Various strains of *B. subtilis, Pseudomonas spp.,* other *Bacillus spp., Streptomyces spp.,* and the like may also be employed in this method. Also, many strains of yeast cells are also available as host cells for the expression of ABC1 polypeptides. Preferred yeast cells include, for example, *Saccharomyces cerivisae* and *Pichia pastoris.* Additionally, insect cell systems may be suitable host cells. Insect cell systems are described, for example, in Kitts et al., 1993, Biotechniques, 14:810-17; Lucklow, 1993, Curr. Opin. Biotechnol. 4:564-72; and Lucklow et al., 1993, J. Virol., 67:4566-79. Preferred insect cells are Sf-9 and Hi5 (Invitrogen).

As described in Example 17 and shown in Figure 12, ligands for nuclear receptors can up-regulate the gene expression of ABC1. Transfection studies using pAPR1, which contains a luciferase reporter gene under the control of the ABC1 promoter, showed that the ABC1 promoter was activated in the presence of ligands for LXR and RXR nuclear receptors. Specifically, macrophage cells transfected with pAPR1 produced a 19-fold increase in luciferase reporter activity in the presence of 20 OH-chol, a 16-fold increase in luciferase activity in the presence of 9-*cis* RA, and a 280-fold increase in luciferase activity in the presence of both ligands compared with EtOH control. The results indicate that both sterols and retinoids elicit a strong transcription response from the ABC1 promoter. Further, there is an apparent synergistic effect between the two classes of compounds, as can be seen by the dramatic increase in luciferase activity found in cells treated with both ligands.

### Methods for Identifying Therapeutic Compounds

Another aspect of the present invention relates to methods for screening a compound to determine whether the compound modulates (i.e., up-regulates or down-regulates) the gene expression of ABC1. Such compounds may be useful in the development of therapeutic compounds that increase ABC1 expression and thereby promote cholesterol efflux and raise blood levels ofHDL-cholesterol. Accordingly, methods for identifying compounds that may be useful in the treatment of cardiovascular disease are provided. In one embodiment, the present invention provides a method for screening a test compound for ABC1 expression modulating activity comprising the steps of: (a) operatively linking a reporter cDNA with the 5' flanking region of the mammalian ABC1 gene comprising SEQ ID NO: 3 or a polynucleotide comprising nucleotides 1-1532, 1080-1643, 1181-1643, 1292-1643, 1394-1643 or 1394-1532 of SEQ ID NO: 3 to produce a recombinant reporter construct; (b) transfecting the recombinant reporter construct into a population of isolated host cells; (c) assaying the level of reporter gene expression in a sample of the transfected host cells; (d) contacting the transfected host cells with the test compound being screened; (e) assaying the level of reporter gene expression in a sample of the transfected host cells after contact with the test compound; and (f) comparing the relative change in the level of reporter gene expression caused by exposure to the test compound, thereby determining the ABC1 expression modulating activity.

First, a recombinant reporter construct comprising a heterologous reporter gene operatively linked to the 5' flanking region of the ABC1 gene is constructed. The ABC1 5' flanking polynucleotide and reporter gene can be inserted into a vector using well-known ligation and cloning techniques, such as those described herein and in standard laboratory manuals, including Davis et al., Basic Methods in Molecular Biology (1986); Sambrook et al., Molecular Cloning: A Laboratory Manual, 2nd Ed., (Cold Spring Harbor Press, Cold Spring Harbor, N.Y. (1989)); and Ausubel et al. eds., Current Protocols in Molecular Biology, (Wiley and Sons (1994)). Alternatively, the ABC1 5' flanking polynucleotide can be inserted into a commercially available reporter construct, such as those previously described. Any vector suitable for ABC1 polynucleotide and reporter gene insertion can be used. The chosen vector should be functional in the particular host cell employed. Preferably, the vector is compatible with mammalian host cells.

Also, preferably the heterologous reporter is selected from the, group consisting of polynucleotides that encode the luciferase, β-galactosidase, chloramphenicol acetyl transferase, and green fluorescent proteins. More preferably, the heterologous reporter is a polynucleotide that encodes the luciferase protein. In a particularly preferred embodiment, the recombinant reporter construct is pAPR1, shown in Figure 11.

Next, the recombinant reporter construct is transfected into a population of isolated host cells. The recombinant reporter construct can be introduced into the host cells using any of the previously described transfection methods, as well as the methods described in Examples 8 and 15. For example, the reporter construct can be transfected using calcium phosphate transfection, DEAE-dextran mediated transfection, cationic lipid-mediated transfection, electroporation, transduction, infection, or any of the other known and described methods (see, e.g., Davis et al., Basic Methods in Molecular Biology (1986))*.* The host cell can be any cell that, when cultured under appropriate conditions, synthesizes a reporter polypeptide, which can be subsequently measured. Preferably, the host cell is a mammalian host cell. More preferably, the mammalian host cell is a macrophage, fibroblast, hepatic, or intestinal cell. Most preferably, the host cell is selected from the group consisting of RAW 264.7 cells, Thp-1 cells, and HepG2 cells. The concentration of reporter construct and duration of the transfection can vary, depending on the transfection method and the type and concentration of host cell used. Determination of the appropriate concentration of reporter construct and transfection time is well within the skill of the ordinary artisan.

Following transfection, a sample of transfected host cells that was not exposed to the test compound is assayed to determine the level of reporter gene expression. The level of reporter gene expression found in the sample of unexposed transfected host cells provides a control measurement. The transfected host cells are lysed and the level of reporter gene expression is assayed using any of the methods well-known in the art. The assays used to measure the level of reporter gene expression differ, depending on the reporter construct used in the transfections. For example, if a luciferase reporter construct is used, the luciferase activity of the cell lysate is measured as light units using a luminometer, as described in Example 15.

A different sample of the transfected host cells is contacted with the test compound being screened and the level of reporter gene expression found in these cells is subsequently measured. Preferably, the transfected host cells are contacted with the test compound for about 4-48 hours. More preferably, the transfected host cells are contacted with the test compound for about 8-36 hours. Even more preferably, the transfected host cells are contacted with the test compound for about 24 hours. The same assay used to measure the level of reporter gene expression in unexposed (control) cells should be used to measure the level of reporter gene expression in the cells exposed to the test compound.

Finally, the level of reporter gene expression found in unexposed control cells is compared with the level of reporter gene expression found in cells exposed to the test compound to determine whether the test compound has ABC1 expression modulating activity. If the level of gene expression in both cell samples are the same or about the same, the test compound does not modulate ABC1 gene expression. A higher level of reporter gene expression in cells exposed to the test compound relative to the level of reporter gene expression found in control cells, indicates that the test compound up-regulates the gene expression of ABC1. A lower level of reporter gene expression in cells exposed to the test compound relative to the level of reporter gene expression found in control cells, indicates that the test compound down-regulates the gene expression of ABC1.

### Kits for Identifying Therapeutic Compounds

The present invention also includes a kit suitable for screening a compound to determine the ABC1 expression modulating activity of a compound. The kit includes, in an amount sufficient to perform at least one assay, a recombinant reporter construct comprising a reporter cDNA operatively linked to an expression modulating portion of the mammalian ABC gene, as a separately packaged reagent. Instructions for use of the packaged reagent(s) are also typically included. The expression modulating portion of the mammalian ABC1 gene comprises the 5' flanking sequence. In one embodiment, the expression modulating portion of the mammalian ABC1 gene comprises the polynucleotide having the sequence identified as SEQ ID NO: 3. In other embodiments, the expression modulating portion of the mammalian ABC1 gene comprises the polynucleotide having the sequence identified as nucleotides 1-1532, 1080-1643, 1181-1643, 1292-1643, 1394-1643, or 1394-1532 of SEQ ID NO: 3. The reporter cDNA can be any suitable reporter gene, including luciferase, β-galactosidase, chloramphenicol acetyl transferase, and green fluorescent protein cDNA. In a particularly preferred embodiment, the recombinant reporter construct is pAPR1. In another embodiment, the kit further comprises means for detecting the reporter protein. Thus, the kit comprises reagents, such as buffers and substrates, used for reporter protein detection.

As used herein, the term "package" refers to a solid matrix or material such as glass, plastic (e.g., polyethylene, polypropylene or polycarbonate), paper, foil and the like capable of holding within fixed limits a recombinant vector of the present invention. Thus, for example, a package can be a glass vial used to contain milligram quantities of a contemplated vector.

"Instructions for use" typically include a tangible expression describing the reagent concentration or at least one assay method parameter such as the relative amounts of reagent and sample to be admixed, maintenance time periods for reagent/ sample admixtures, temperature, buffer conditions and the like.

### Microarrays

It will be appreciated that DNA microarray technology can be utilized in accordance with the present invention. DNA microarrays are miniature, high-density arrays of nucleic acids positioned on a solid support, such as glass. Each cell or element within the array contains numerous copies of a single nucleic acid species that acts as a target for hybridization with a complementary nucleic acid sequence (*e.g*., mRNA). In expression profiling using DNA microarray technology, mRNA is first extracted from a cell or tissue sample and then converted enzymatically to fluorescently labeled cDNA. This material is hybridized to the microarray and unbound cDNA is removed by washing. The expression of discrete genes represented on the array is then visualized by quantitating the amount of labeled cDNA that is specifically bound to each target nucleic acid molecule. In this way, the expression of thousands of genes can be quantitated in a high throughput, parallel manner from a single sample of biological material.

This high throughput expression profiling has a broad range of applications with respect to the ABC1 molecules, including, but not limited to: the identification and validation of ABC1 disease-related genes as targets for therapeutics; molecular toxicology of related ABC1 molecules and inhibitors thereof; stratification of populations and generation of surrogate markers for clinical trials; and enhancing related ABC polypeptide small molecule drug discovery by aiding in the identification of selective compounds in high throughput screens.

As discussed herein at Example 2, a method has been developed that uses samples of RNA derived from cells of an individual with a genetic abnormality and compares them to the RNA from a normal individual. Historically, identification of the cause of inherited diseases resulted from years of biochemical analysis or, more recently, from years of gene mapping and positional cloning to identify the suspect gene within an interval of millions of base pairs which had been shown to be closely linked to the defect in inheritance studies (linkage analysis). The use of multigene expression analysis, most notably via "gene chips" can revolutionize the pace of such discovery. Comparing the expression of samples of RNA derived from cells from an abnormal individual with a genetic disease versus RNA from an normal individual can quickly reveal genes whose corresponding mRNA is missing, severely underrepresented or severely overrepresented in the abnormal diseased cell.

The term "individual" and used herein refers to any living organism that has RNA such as, for example, mammals, plants. This method is preferably used to identify the source of human genetic abnormalities. More preferably, the method is useful for detecting genetic sources of human cardiac and cardiovascular disorders such as identifying ABC1 as the genetic defect in Tangier's disease.

The term "abnormality" as it is used herein refers to genetic differences that causes a physiological deviation in small number of individuals in a species in comparison to the majority of individuals of the species. The abnormality may be a positive abnormality or a negative abnormality. For example, a positive plant abnormality would be a genetic difference that causes some individual plants to be drought resistant in comparison to the other individuals in the species. A negative abnormality would be one that causes an individual in the same species of plant to me more prone to drought damage than a normal plant.

The method can best be described by reference to our investigation into the genetic cause of Tangier's disease. We began our investigation by using RNA from cells cultured from an individual with Tangier disease to probe microarrays containing nearly 60,000 normal human genes, and we were able to use the probe results to identify ABC1 as the defective gene in this monogenic disease in which patients have near zero levels of circulating high density lipoprotein (HDL) and an increased risk of heart disease. It is not necessary that the defective gene results in a zero level of detectable mRNA signal in such an experiment. In this successful example, roughly 175 out of the 58,800 probes on the micro-array were more than 2.5 fold underexpressed in the Tangier disease RNA versus normal. Several additional steps may be taken to confirm the identity of the culprit gene. They include repeating such a micro-array probe with an unrelated individual with Tangier disease, determining the chromosome map location of each gene to compare with a reported large genetic interval that was linked to the disease, consideration of the likely function of the candidate proteins and their homologs, biochemical tests, and sequencing the best candidate gene in patients to find mutations. In these ways, gene expression micro-array analysis can lead to the identification of inherited genetic defects such as the identification of ABC1 as the defect in Tangier disease.

A further utility in this method is that other genes that are either under- or over-expressed in the disease sample vs. normal should include those that are differentially regulated in consequence of the genetic defect in the patient, either as compensatory responses or as contributors to the disease pathology. This could provide identification of other proteins in the relevant biological pathways that may be amenable to drug development and help elucidate the pathology of the disease, with implications for treatment and diagnosis. In the cases where a gene deletion or other mutation causes complete absence of mRNA, as observed in many examples of thalassemia (globin gene defects) and other genetic diseases, gene expression analysis of disease versus normal samples can lead to the identification of the missing gene in a more straightforward manner.

Although in these examples, the gene expression array that was probed with RNA samples was of the type in which probe samples were cDNAs arrayed on microscope slides, alternative array technologies would suffice. These would include, but not limited to those which array DNA samples on filter membranes or use oligonucleotide probes synthesized on "gene chips" by photolithography.

Generally, the term microarray refers to an array of distinct oligonucleotides synthesized on a substrate, such as paper, nylon or other type of membrane, filter, chip, glass slide, or any other suitable solid support. Microarrays may be prepared, used, and analyzed using methods known in the art. (See, e.g., Brennan, T. M. et al. (1995) U.S. Pat. No. 5,474,796; PCT application W095/251116; Shalon, D. et al. (1995) PCT application W095/35505; and U.S. Pat. No. 5,605,662 the specifications of each of which are incorporated herein by reference)

A chemical coupling procedure and an ink jet device can be used to synthesize array elements on the surface of the substrate. An array analogous to a dot or slot blot may also be used to arrange and link elements to the surface of a substrate using thermal, UV, chemical, or mechanical bonding procedures. A typical array may be produced by hand or using available methods and machines and contain any appropriate number of elements. After hybridization, nonhybridized probes are removed and a scanner used to determine the levels and patterns of fluorescence. The degree of complimentrity and the relative abundance of each probe which hybridizes to an element on the microarray may be assessed through analysis of the scanned images.

Full-length cDNAs, Expressed Sequence Tags (ESTs), or fragments thereof may comprise the elements of the microarray. Fragments suitable for hybridization can be selected using software well known in the art such as LASERGENE software (DNASTAR). Full-length cDNAs, ESTs, or fragments thereof corresponding to the nucleotide sequences of an abnormal individual and a normal individual are arranged on an appropriate substrate, e.g., a glass slide. The cDNA is fixed to the slide using, e.g., UV cross-linking followed by thermal and chemical treatments and subsequent drying. (See, e.g., Schena, M. et al. (1995) Science 270:467-470; Shalon, D. et al. (1996) Genome Res. 6:639-645.) Probes, such as fluorescent probes are prepared and used for hybridization to the elements on the substrate.

In order to conduct sample analysis using the microarrays, the RNA or DNA from a biological sample is made into hybridization probes. The mRNA is isolated, and cDNA is produced and used as a template to make antisense RNA (aRNA). The aRNA is amplified in the presence of fluorescent nucleotides, and labeled probes are incubated with the microarray so that the probe sequences hybridize to complementary oligonucleotides of the microarray. Incubation conditions are adjusted so that hybridization occurs with precise complementary matches or with various degrees of less complementarity. After removal of nonhybridized probes, a scanner is used to determine the levels and patterns of fluorescence. The scanned images are examined to determine degree of complementarity and the relative abundance of each oligonucleotide sequence on the microarray. The biological samples may be obtained from any bodily fluids (such as blood, urine, saliva, phlegm, gastric juices, etc.), cultured cells, biopsies, or other tissue preparations. A detection system may be used to measure the absence, presence, and amount of hybridization for all of the distinct sequences simultaneously. This data may be used for large scale correlation studies on the sequences, mutations, variants, or polymorphisms among samples.

The microarray is preferably composed of a large number of unique, single-stranded nucleic acid sequences, usually either synthetic antisense oligonucleotides or fragments of cDNAs fixed to a solid support. Microarrays may contain oligonucleotides which cover the known 5', or 3', sequence, or contain sequential oligonucleotides which cover the full length sequence; or unique oligonucleotides selected from particular areas along the length of the sequence. Polynucleotides used in the microarray may be oligonucleotides that are specific to a gene or genes of interest in which at least a fragment of the sequence is known or that are specific to one or more unidentified cDNAs which are common to a particular cell type, developmental or disease state.

In order to produce oligonucleotides to a known sequence for a microarray, the gene of interest is examined using a computer algorithm which starts at the 5' or more preferably at the 3' end of the nucleotide sequence. The algorithm identifies oligomers of defined length that are unique to the gene, have a GC content within a range suitable for hybridization, and lack predicted secondary structure that may interfere with hybridization. The oligomers are synthesized at designated areas on a substrate using a light-directed chemical process. The substrate may be paper, nylon or other type of membrane, filter, chip, glass slide or any other suitable solid support. An array may, be produced by hand or using available devises (slot blot or dot blot apparatus) materials and machines (including robotic instruments) and contain grids of 8 dots, 24 dots, 96 dots, 384 dots, 1536 dots or 6144 dots, or any other multiple which lends itself to the efficient use of commercially available instrumentation.

Once the genetic causes of the inherited abnormality are narrowed or identified, the potentially or actual defective portions of the genes can be used as targets in a microarray. The microarray can be used to monitor the expression level of a large number of genes to develop and monitor the activities of potential therapies and therapeutic agents.

The following examples further illustrate the present invention but should not be construed to limit the present invention in any way. Examples relating to subject-matter which is not encompassed by the claims are given for comparative purposes only.

### EXAMPLE 1

This example demonstrates that patients with Tangier Disease (TD) have an absence of apo A-1-mediated lipid efflux.

Cell Cultures: Human fibroblasts were obtained from skin explants from two normal subjects (NL1) and three unrelated patients with Tangier disease (TD). TD1 cells were obtained from a 53 year-old female with extremely low plasma HDL cholesterol and apo A-1 levels and clinical symptoms typical of Tangier disease. TD2 cells were obtained from a 56 year-old male with clinical, morphological, and biochemical features of Tangier disease, including very low levels of plasma HDL cholesterol and apoA-I (Francis, et al., J. Clin.-Invest.; 96, 78-87 (1995)). TD3 cells were obtained from an 18 year-old male with Tangier disease who presented with orange tonsil remnants, asymmetrical motor neuropathy, plasma HDL cholesterol of 5 mg/dL, and LDL cholesterol of 16mg/dL (Lawn et al., J. Clin. Invest., 104, R25-R31 (1999)). The normal cells and TD subject cells were immortalized as described in Oram et al., J. Lipid Res., 40: 1769-1781 (1999). Briefly, the cells were transfected with amphotropic retroviruses containing vectors with inserts of human papillomavirus 16 oncogenes E6 and E7 and a neomycin resistance selectable marker. Control cells were infected with vector alone (mock-infected). Pooled cell populations were selected in the presence of G418 for two passages, after which G418 was excluded from the medium. Fibroblasts were used between the fifth and sixteenth passage (primary) or sixth and fourteenth passage (immortalized). The immortalized normal and TD cells were seeded into 16-mm wells or 35-mm dishes and grown to confluence in Dulbecco's modified Eagle's medium (DMEM) plus 10% fetal bovine serum (FBS) before experimental use. RAW 264.7 mouse monocytic cells (American Type Culture Collection, Rockville, MD) were also maintained in DMEM containing 10% FBS.

Assay to Measure Lipid Effux: Apo AI-mediated efflux of cholesterol and phospholipid was assayed according to the method described in Francis, et al., J. Clin. Invest., 96: 78-87 (1995). The cultured skin fibroblasts from normal and TD subjects were labeled by growth to confluence in the presence of 0.2-0.5 µCi/ml [³H]cholesterol (40-60 Ci/mmol, Amersham Corp., Arlington Heights, IL). The radioactive cholesterol was added to serum-containing growth medium when the cells were 60-80% confluent. After 3 days, the cells were washed twice with PBS/BSA and simultaneously growth-arrested and cholesterol-loaded to maximize apolipoprotein-mediated lipid efflux. This was achieved by incubating the cells for 48 hours in serum-free DMEM plus 2 mg/ml fatty acid-free bovine serum albumin (DMEM/BSA) (Sigma Chemical Co., St. Louis, MO) and 30 µg/ml non-radioactive cholesterol. RAW 264.7 cells were cholesterol-loaded through the scavenger receptor by 24-hour incubation with acetylated LDL as described in Smith et al., J. Biol. Chem., 271:30647-30655 (1996). Briefly, RAW 264.7 cells in 24-well dishes were cholesterol-loaded and labeled overnight in 0.5 ml of DMEM supplemented with 50 µl/ml 1M glucose, 10 µl/ml 200 mM glutamine and 2% BSA and with 50 µg/ml acetylated low density lipoprotein (AcLDL) and [³H]-cholesterol which had been pre-incubated for 30 minutes at 37°C with AcLDL to yield a final concentration of 0.33 µCi/ml [³H]-cholesterol. Cells were subsequently washed five times with PBS containing 1 % BSA and incubated overnight (16-18 hours) in DMEM/BSA to allow for equilibrium of cholesterol pools.

After equilibrium of cholesterol pools, cells were rinsed four times with PBSBSA and incubated for one hour at 37°C with DMEM/BSA before the efflux incubations. Efflux medium (DMEMBSA) containing either albumin alone (control), albumin plus HDL (40µg protein/ml), or albumin plus apo A-I (10 µg/ml, Biodesign International, Kennebunk, ME) was added and the cells were incubated for 4, 24, or 48 hours. Phospholipids were labeled by including 10 µCi/ml [³H]choline (75-85 Ci/mmol, Amersham Corp.) in the DMEM/BSA overnight equilibrium medium. The radioactivity found in the culture medium was measured by scintillation counting after a 15-minute centrifugation at 12,000g. The radioactivity in the cells was measured by scintillation counting after solubilization in 0.5 ml of 0.2M NaOH (Smith et al.. J. Biol. Chem., 271:30647-30655 (1996)) or extraction in hexane:isopropanol (3:2 v/v) as described in Francis, et al., J. Clin. Invest., 96, 78-87 (1995). Cells containing labeled phospholipids were extracted with 1 ml of isopropanol for 1 hour and then with hexane:isopropanol as described above. The efflux of cholesterol or phospholipid was expressed as the percentage of tritiated lipid counts in the medium over the total tritiated lipid counts recovered from the cells and medium (cpm medium / cpm (medium + lysate) x 100).

As shown in Figure 1A and C, the addition of HDL or apo A-1 results in the removal of cholesterol from cholesterol-laden fibroblasts obtained from normal subjects. However, in TD cells, the ability of HDL to remove cholesterol is slightly diminished and the ability of apo A-1 to remove cholesterol is completely absent. Figure 1 shows that normal and TD fibroblast cells release about 3-4% of the cellular [³H]-cholesterol into the medium during 48-hour incubation with albumin. Addition of HDL to the albumin medium increased the efflux of [³H]-cholesterol from both normal and TD fibroblasts, although to a lesser extent with TD cells (Figure 1B, D). Addition of apo A-I promoted the efflux of [³H]-cholesterol from normal fibroblast (Figure 1A, C), but had little or no effect on [³H]-cholesterol efflux from TD fibroblasts (Figure 1B, D).

### EXAMPLE 2

This example demonstrates that 175 genes show at least 2.5-fold decreased expression and 375 genes show at least 2.5-fold increased expression in TD cells compared with normal cells. The differential gene expression was determined using gene-expression microarray (GEM) analysis of cDNAs from normal individuals (non-TD) and from patients with TD.

Cell Cultures: The immortalized cell cultures obtained from normal individuals and TD patients described in Example 1 were used. Confluent cultures were maintained in DMEM/BSA and supplemented for 24 hours with 1mM 8-Bromo cyclic adenosine monophosphate (8-Br-cAMP, Sigma Chemical Co., St. Louis, MO).

mRNA Extraction and cDNA Synthesis: mRNA from both normal and TD fibroblast cells was prepared from total RNA extracted from cells with Trizol (Life Technologies Inc., Bethesda, MD, Cat. # 15596-026). The mRNA was isolated using the Oligotex mRNA kit (Qiagen Inc., Valencia, CA, Cat. # 70022) according to vendor's protocols. The mRNA was reverse transcribed using Cy3 or Cy5 fluorescent dye to create fluorescently labeled cDNA according to the method described in DeRisi et al., Science, 24:680-686 (1997). The resultant cDNA from TD cells was labeled with Cy3 fluorescent dye and cDNA from normal cells was labeled with Cy5 fluorescent dye (Incyte Genomics, Palo Alto, CA).

Microarray Analysis: To analyze differential gene expression in cells from individuals with TD and normal individuals, Cy3 and Cy5 fluorescent labeled cDNA samples prepared as described above were hybridized to a set of Gene Album microarrays (GEMs) on microscope slides (Incyte Genomics, Palo Alto, CA). Each of six slides contained about 9,800 human cDNA samples plus 200 control samples, resulting in a microarray of 58,800 partial cDNAs. Therefore, allowing for estimates of redundancy, approximately 30-50% of expressed human genes were represented. The hybridization of Cy3-labeled cDNA prepared from TD1 cells and Cy5-labled cDNA from normal cells allowed comparison of the-relative RNA content of TD cells versus normal cells for the expressed genes. In addition, Cy3-labeled cDNA prepared from TD2 cells and Cy5-labled cDNA from normal cells were hybridized to the same set of microarrays to examine the variation of gene expression between different TD patients.

Results: Data were analyzed using GemTools software (Incyte Genomics, Palo Alto, CA) and expressed as ratios of TD cell to normal cell mRNA. The results indicated that the majority of genes are comparably expressed in TD1 and normal cells. As shown in Figure 2 (in the section above and to the left of the diagonal) only 175 genes were more than 2.5-fold underexpressed in TD1 cells compared with normal cells, whereas 375 genes were more than 2.5-fold overexpressed in TD1 cells compared with normal cells (below and to the right of the diagonal). Genes more highly expressed in the TD cells could include those that are differentially regulated as a consequence of the Tangier mutation, either as a compensatory response or as a contributor to the disease pathology. Among the genes that could contribute to the observed phenotype of TD and are more highly expressed in TD cells include interferon-β (IFN-β), macrophage inflammatory protein-2α, granulocyte chemotactic protein-2, IL-11, prostaglandin endoperoxide synthase-2 (COX-2), thrombospondin, and monocyte chemotactic proteins 1, 3, and 4 (Lawn et al., J. Clin. Invest., 104:R25-R31 (1999)).

No single RNA that was expressed in the normal fibroblasts was found completely absent in either the TD 1 or TD2 cells. Also, comparison of the differentially expressed genes in TD1 and TD2 revealed very little variation between the individual TD patients. For instance, of the most highly down-regulated genes in TD2 cells, 92% were also under-expressed in TD1 cells compared with normal cells. One of the genes more than 2.5-fold under-expressed in TD1 or TD2 versus normal cells was the gene for ABC1 protein. The ABC1 gene was pursued due to the ascribed functions of some of its homologues and also because the gene was localized to the approximate chromosome region reported as the TD gene region.

### EXAMPLE 3

This example demonstrates that the ABC1 gene is localized to the human chromosome 9q31.

Previous genetic linkage analysis mapped the TD gene to the 7-cM region of human chromosome 9q31 (Rust et al., Nat. Genet., 20, 96-98 (1998)). In addition, *in situ* hybridization analyses revealed that the ABC1 gene was localized to the broader chromosomal interval 9q22 - 9q31 (Luciani et al., Genomics, 21, 150-159 (1994)). Using PCR methods with the GeneBridge 4 panel of human/hampster radiation hybrids (Research Genetics, Inc., Huntsville, AL), human ABC1 was determined to be located between the markers WI-14706 and WI-4062, corresponding to the 7-cM region of human chromosome 9q31. DNA from 93 human/hampster hybrid cell lines was amplified by PCR using human ABC1-specific primers LF: CCTCTCATTACACAAAAACCAGAC (SEQ ID NO: 11) and LR: GCTTTCTTTCACTTCTCATCCTG (SEQ ID NO: 12). Each line was scored as positive or negative for the human ABC1 amplification product and the mapping of ABC1 derived from analysis of this data was accomplished using the Whitehead Institute/MIT Center for Genome Research software, accessed via the internet (http://carbon.wi.mit.edu:8000/cgi-bin/contig/rhmapper.pl). These results were further confirmed by southern blot hybridization to human genomic/yeast artificial chromosome clones (Research Genetics, Inc.) from the equivalent interval. In addition, public database searching (GeneMap'98; National Center for Biotechnology Information) and radiation hybrid mapping eliminated the other significantly underexpressed genes in the microarray data from the location in the reported genetic interval. These complementary data demonstrate that the ABC1 gene is located on human chromosome 9q31 and further indicate that the ABC1 gene is associated with Tangier disease.

### EXAMPLE 4

This example shows the determination of the nucleotide sequence of the wildtype ABC1 gene, including the flanking regions and the entire coding region.

DNA sequencing was performed using an ABI Prism 310 Genetic Analyzer or by Davis Sequencing (Davis, CA). Both strands were sequenced throughout. The sequence of the open reading frame of the ABC1 gene from a normal subject was determined from a full-length cDNA clone obtained from an expression plasmid library constructed from normal fibroblast RNA. To construct the plasmid library, cDNA was synthesized according to the Stratagene kit protocol (Stratagene, La Jolla, CA). Briefly, first strand cDNA was synthesized from mRNA using an oligo-dT primer with an XhoI site and MMLV reverse transcriptase in the presence of 5-methyl dCTP. The second strand was synthesized using RNase H and DNA polymerase I in the presence of unmodified dNTPs. After the cDNA was blunt-ended with pfu DNA polymerase, an EcoRI linker was ligated to the cDNA. The cDNA was then digested with XhoI, creating XhoI ends at the 3' end of the cDNA. The internal Xhol sites were protected from this digestion due to the semi-methylation during the first strand synthesis. The synthesized cDNA was cloned into the HindIII and XhoI sites of the plasmid pCEP4 (Invitrogen Corp., Carlsbad, CA #VO44-50), an expression vector containing the cytomegalovirus promoter/enhancer. A 585 bp ABC1 probe was generated by reverse transcriptase polymerase chain reaction (RT-PCR) using primers based on known ABC1 sequence, which were 5'-TCCTTGGGTTCAGGGGATTATC (SEQ ID NO: 13) and 5'-CAATGTI`ITTGTGGCTTCGGC (SEQ ID NO: 14). Using this ABC1 probe, a clone containing a 10.5 kb insert of human ABC DNA was recovered from the library using the CloneCapture selection kit according to the manufacturer's protocol (CLONTECH Laboratories, Inc., Palo Alto, CA). This clone is shown in Figure 3 as pCEPhABC1. The 10.5 kb ABC1 cDNA insert sequence is shown in SEQ ID NO: 1. Sequence determination confirmed that pCEPhABC1 contains the human ABC1 open reading frame of 6783 nucleotides plus 5' and 3' untranslated regions, having a larger open reading frame than the cDNA sequence reported by Langmann et al. in Biochem. Biophys. Res. Comm., 257, 29-33 (1999) (GenBank Accession No. AJO12376).

### EXAMPLE 5

This example demonstrates the sequence differences between the wildtype ABC1 gene and the TD1, TD2, and TD3 gene sequences.

cDNA Synthesis of TD1, TD2, and TD3: cDNA was prepared from TD1, TD2, and TD3 cells by reverse transciption polymerase chain reaction (RT-PCR) using the Superscript Choice cDNA system and the Advantage cDNA polymerase mix following the manufacturer's protocol (CLONTECH, Palo Alto, CA; Cat. #8417-1) using two sets of primer pairs designed from the normal human ABC1 gene sequence, designated: (1) sacIhabcf, 5'-AGTCGAGCTCCAAACATGTCAGCTGTTACTGGAAGTGGCC (SEQ ID NO: 15); habcr3851, 5'- TCTCTGGATTCTGGGTCTATGTCAG (SEQ ID NO: 16) and (2) habcf3585, 5'-GGGAGCCTTTGTGGAACTCTTTC (SEQ ID NO: 17); habcrsalI, 5'-ACTGGTCGACCATTGAATTGCATTGCATTGAATAGTATCAG (SEQ ID NO: 18). Amplification of 0.2-0.5 µg polyA+ RNA with these primers at a final concentration of 0.4µM generated two overlapping templates of approximately 3.5 kb. The templates were gel-purified using the QIAEX II system (QIAGEN, Inc., Valencia, CA; Cat. #20021) and adjusted to a concentration of 100ng/µl.

Sequencing of TD1, TD2. and TD3 cDNAs: Eight µl of each template generated as described above was sequenced in a reaction with individual sequencing primers designed on the basis of wildtype ABC1 sequence at a final concentration of 0.5µM. The primers were as follows: 1F: 5'- TTTCCTGGTGGACAATGAA (SEQ ID NO: 19), 2F: 5'-AGTGACATGCGACAGGAG (SEQ ID NO: 20); 3F: 5'- GATCTGGAAGGCATGTGG (SEQ ID NO: 21); 4F: 5'- CCAGGCAGCATTGAGCTG (SEQ ID NO: 22); 5F: 5'-GGCCTGGACAACAGCATA (SEQ ID NO: 23); 6F: 5'- GGACAACCTGTTTGAGAGT (SEQ ID NO: 24); 7F: 5'- AAGACGACCACCATGTCA (SEQ ID NO: 25); 8F: 5'-ATATGGGAGCTGCTGCTG (SEQ ID NO: 26); 9F: 5'-GGGCATGAGCTGACCTATGTGCTG (SEQ ID NO: 27); 10F: 5'-AAGAGACTGCTAATTGCC (SEQ ID NO: 28); 11F: 5'- AGCGACAAAATCAAGAAG (SEQ ID NO: 29); 12F: 5'- TGGCATGCAATCAGCTCT (SEQ ID NO: 30); 13F: 5'-TCCTCCACCAATCTGCCT(SEQ ID NO: 31); 14F: 5'- TTCTTCCTCATTACTGTT (SEQ ID NO: 32); 15F: 5'- GATGCCATCACAGAGCTG (SEQ ID NO: 33); 16F: 5'-AGTGTCCAGCATCTAAA (SEQ ID NO: 34); 1R: 5'- CAAAGTTCACAAATACTT (SEQ ID NO: 35); 2R: 5'- CTTAGGGCACAATTCCACA (SEQ ID NO: 36); 3R: 5'-TGAAAGTTGATGATTTTC (SEQ ID NO: 37); 4R: 5'- TTTTTCACCATGTCGATGA SEQ ID NO: 38); 5R: 5'- CTCCACTGATGAACTGC (SEQ ID NO: 39); 6R: 5'-GTTTCTTCATTTGTTTGA (SEQ ID NO: 40); 7R: 5'- AGGGCGTGTCTGGGATTG (SEQ ID NO: 41); 8R: 5'- CAGAATCATTTGGATCAG (SEQ ID NO: 42); 9R: 5'-CATCAGAACTGCTCTGAG (SEQ ID NO: 43); 10R: 5'- AGCTGGCTTGTTTTGCTTT SEQ ID NO: 44), 11 R: 5'- TGGACACGCCCAGCTTCA (SEQ ID NO: 45), 12R: 5'-CCTGCCATGCCACACACA (SEQ ID NO: 46), 13R: 5'- CTCATCACCCGCAGAAAG (SEQ ID NO: 47), 14R: 5'- CACACTCCATGAAGCGAG (SEQ ID NO: 48), 15R: 5'-TCCAGATAATGCGGGAAA (SEQ ID NO: 49), 16R: 5'- TCAGGATTGGCTTCAGGA (SEQ ID NO: 50), UTR1R: 5'- AAGTTTGAGCTGGATTTCTTG (SEQ ID NO: 51).

Results: The nucleotide numbering follows the numbering found in Lawn et al. (1999). Patient TD1 retained the full open reading frame, with 2 substantial differences from the wild-type sequence (SEQ ID NO: 8). One of these is an A to G substitution, resulting in a change from a glutamine to arginine residue at position 537 of the 2201 amino acid sequence, as published by Lawn et al. (1999). The location of this residue is within the NH₂-terminal hydrophilic domain, near the first predicted transmembrane domain. Patient TD2 also retained the open reading frame with an arginine to tryptophan substitution at residue 527 (SEQ ID NO: 10). Thus, both TD 1 and TD2 contain a substitution altering the charge of an amino acid in the same region of the protein. TD3 DNA contains a 14 nucleotide insertion in its ABC1 cDNA following nucleotide 5697 in one allele and a 138 bp insertion after nucleotide 5062 in the other allele.

Genomic sequencing of the TD1, TD2, and TD3 DNAs confirmed the changes found in the respective cDNAs. The genomic sequence was generated by PCR amplification of a 156 bp region of genomic DNA isolated from fibroblasts that contained the mutations found in the cDNA from TD1 and TD2. The genomic sequencing also indicated that patient TD1 was homozygous for the glutamine to arginine substitution. Genomic DNA analysis showed that TD2 was a compound heterozygote with one allele containing the detected substitution and the second allele (which failed to produce detectable mRNA) containing an undetermined defect. Neither of the substitution mutations was found in more than 80 alleles of geneomic DNA of non-TD individuals. TD3 insertions were identified by sequence analysis and confirmed by RT-PCR using primers surrounding the insertion points. The 14-bp insertion following nucleotide 5697 causes a frameshift, resulting in the replacement of the wild-type amino acid sequence from a location before the second ATP binding domain, up to the point of a premature protein termination. The 138 bp insertion following nucleotide 5062 in the other allele contains an inframe stop codon.

### EXAMPLE 6

This example demonstrates that inhibitors of ABC1 transport activity also inhibit apo A-1-mediated cholesterol efflux from fibroblast cells.

To test whether inhibition of ABC1 could affect the process of apolipoprotein-mediated cholesterol efflux, two compounds reported to be ABC1 inhibitors were tested in assays which monitor apolipoprotein mediated cholesterol efflux. The compounds 4,4-diisothiocyanostilbene-2,2'-disulfonic acid (DIDS) and sulphobromophthaleine (BSP) were reported to inhibit anion transport activities of ABC1 in a dose-dependent fashion (Becq et al., J. Biol. Chem., 272:2695-2699 (1997); Hamon et al., Blood, 90:2911-2915 (1997)). The apolipoprotein-mediated cholesterol efflux assays were performed as described in Example 1 with the noted changes. Cholesterol-loaded and [³H]cholesterol-labeled normal fibroblasts (n=3) were incubated for 6 hours with or without 5µg/ml apo A-I and either 0, 0.2mM, or 0.4mM DIDS. In addition, cholesterol-loaded and [³H]cholesterol-labeled normal fibroblasts (n=3) were incubated for 6 hours with or without 5µg/ml apo A-I and either 0, 0.2mM, or 0.4mM BSP. [³H]cholesterol efflux was measured by scintillation counting as described in Example 1 and calculated as the percentage of total radiolabeled cholesterol appearing in the medium. The results are shown in Figure 5 as the mean ± SD (n=3) of efflux in the presence of apo A-I after subtraction of values for apo A-I-free medium. Figure 5 shows that both DIDS and BSP inhibit the 6-hour efflux of tritiated cholesterol mediated by apolipoprotein A-I. In addition, similar inhibition was observed with the efflux of tritiated phosphatidyl choline using DIDS and BSP (data not shown). The results of these tests mimic the efflux defect in fibroblasts derived from patients with TD, described in Example 1.

### EXAMPLE 7

This example demonstrates that antisense inhibition of ABC1 mRNA expression inhibits apo A-1-mediated cholesterol efflux from fibroblast cells.

Normal skin fibroblasts were labeled with [³H]cholesterol as described in Example 1. The cells were then loaded with oligonucleotide by scraping in the presence of either 30µM control Morpholino oligonucleotide (5'-CCTCTTACCTCAGTTACAATTTATA-3' corresponding to the antisense complement of a β-globin thalassemic mRNA; SEQ ID NO: 52) or 30µM ABC1 antisense Morpholino oligonucleotide (5'-CATGTTGTTCATAGGGTGGGTAGCTC-3'; SEQ ID NO: 53) and reseeding on new dishes. Control cells were mock-loaded after [³H]cholesterol-labeling by scraping and reseeding in the absence of oligonucleotide. Apo A-I-mediated efflux was measured after 12 hours by scintillation counting as the percentage of total radiolabeled cholesterol appearing in the medium. The results are shown in Figure 6 as the mean ± SEM of three separate experiments, normalized to the value for apo A-I-specific efflux in the absence of oligonucleotide in each experiment. As shown in Figure 6, antisense oligonucleotides directed against ABC1 mRNA caused a 50% reduction in cholesterol efflux from normal fibroblasts compared with control antisense oligonucleotide (β-globin antisense oligonucleotide).

### EXAMPLE 8

This example demonstrates that over expression of the human ABC1 gene results in an increase in apo A-I-mediated cholesterol efflux from monocyte cells.

Stable Transfection of RAW 264.7 Cells: Mouse monocytic RAW 264.7 cells were stably transfected with the pCEPhABC1 expression plasmid for human ABC1. Construction of the pCEPhABC1 plasmid containing the open reading frame of human ABC1 is described in Example 4. Approximately I x 10⁶ RAW 264.7 cells were transfected for 5 hours with 2 µg of pCEPhABC1 DNA and 12µl Geneporter transfection reagent (Gene Therapy Systems, Inc., San Diego, CA; Cat. #T201007) in 0.8ml serum-free DMEM. Two days later, cells were split at ratios ranging from 1:2 - 1:50 and selection applied by adding 150 µg/ml hygromycin to the culture medium. After two weeks, hygromycin-resistant colonies were picked and expanded.

Apo A-I-mediated Cholesterol Efflux Assay: Parental RAW 264.7 cells and three clonal lines (L3, L5, and L6) stably expressing human ABC1 were grown to confluence. The cells were cholesterol-loaded and labeled by incubation for 24 hours with 0.5 µCi/ml [³H]cholesterol and 50µg/ml acetylated LDL as described in Example 1. After equilibrium of cholesterol pools by an overnight incubation in DMEM/BSA, cells were washed and the efflux medium was added as described in Example 1. Apo A-I-mediated cholesterol efflux was measured as previously described by scintillation counting of the tritiated cholesterol in the cell medium, expressed as a percentage of the total counts recovered from the cells and medium. The results are presented as the mean ± SEM of three separate experiments normalized to the value for apo A-I-specific efflux from parental RAW 264.7 cells within each experiment. Figure 7 shows the apo A-I-mediated cholesterol efflux from parental RAW 264.7 cells and L3, L5, and L6 transfected cell lines. As can be seen, transfection with the ABC1 expression vector results in a 4-fold (L6) to 8-fold (L3 and L5) increase in apo A-I-mediated cholesterol efflux. These results indicate that overexpression of the ABC1 gene can substantially increase the amount of cholesterol efflux from macrophage cells.

### EXAMPLE 9

This example demonstrates that ABC1 mRNA expression is regulated by cellular conditions related to cholesterol efflux in normal skin fibroblasts, but not in TD fibroblasts.

To determine whether ABC1 plays a rate-limiting role in cellular sterol efflux, the synthesis of ABC1 was measured under various cellular conditions related to cholesterol efflux processes. Specifically, normal fibroblasts and TD fibroblasts were individually exposed to conditions of excess cAMP, cholesterol, or Apo A-I. Cell cultures of normal skin fibroblasts and TD1 and TD2 fibroblasts were prepared as described in Example 1. The level of ABC1 mRNA was measured by RT-PCR.

Cell cultures: Immortalized cell cultures of normal skin fibroblasts and TD1 and TD2 fibroblasts were prepared as described in Example 1. Cells were grown to subconfluence in DMEM/10% FBS before replacement with DMEM/BSA and the indicated additive for 24 or 48 hours. RNA was prepared as described in Example 2.

RT-PCR: Quantitative PCR was carried out using the GeneAmp 5700 Sequence Detection System (Perkin-Elmer Applied Biosystems, Foster City, CA). Briefly, 500ng of DNase-treated mRNA was reverse transcribed using random hexamer primers at 2.5µM. Approximately 5% of this reaction was amplified by PCR using the SYBR green core kit (PE Applied Biosystems, Foster City, CA; Cat. #4304886) and human ABC1 primers LF: 5'-CCTCTCATTACACAAAAACCAGAC (SEQ ID NO: 11) and LR: 5'-GCTTTCTTTCACTTCTCATCCTG (SEQ ID NO: 12) to yield an 82 bp fragment corresponding to nucleotides 7018-7099 of human ABC1. PCR cycle conditions were as follows: 10 minutes 95°C; followed by 40 cycles of 95°C, 15 seconds; and 60°C, 60 seconds. The mRNA in each sample was quantitated by detecting the increase in fluorescence caused by SYBR green binding to the double-stranded amplification product generated during each PCR cycle. All samples were run in triplicate and normalized against β-actin mRNA, amplified in parallel reactions with primers actinF: 5'-TCACCCACACTGTGCCATCTACGA (SEQ ID NO: 54) and actinB: 5'- CAGCGGAACCGCTCATTGCCAATGG (SEQ ID NO: 55). Standard curves were run for both ABC1 and β-actin on the same PCR plate.

8-Br-cAMP Assay: Normal, TD1, and TD2 fibroblast cells were grown to subconfluence in DMEM/10% FBS and then treated with 1 mM 8-Br-cAMP in DMEM/BSA for 24 hours.

Cholesterol Assay: Normal, TD1, and TD2 fibroblast cells were grown to subconfluence in DMEM/10% FBS and then treated with 30µg/ml free cholesterol in DMEM/BSA for 48 hours followed by 18-24 hours of equilibrium in DMEM/BSA.

Apo A-I Assay: Normal, TD1, and TD2 fibroblast cells were grown to subconfluence in DMEM/10% FBS and then treated with 30µg/ml free cholesterol in DMEM/BSA for 48 hours followed by 18-24 hours of equilibrium in DMEM/BSA plus 10µg/ml apo A-I.

Results: Figure 8 shows that in normal fibroblasts ABC1 mRNA is increased approximately 10-fold by exposure to 8-Br-cAMP and increased approximately 17-folk by exposure to cholesterol in serum-free medium. Subsequent exposure of cholesterol-loaded cells to Apo A-I results in a marked decrease in ABC1 mRNA expression. Although the mechanism has not been demonstrated, previous studies have shown that cholesterol efflux is promoted in the presence of such compounds as cAMP and cholesterol (Hokland et al., J. Biol. Chem., 268:25343-25349 (1993)). The present results indicate that in normal fibroblasts, ABC1 mRNA is induced by these known effectors of the cholesterol efflux pathway and repressed by exposure to an apolipoprotein cholesterol acceptor, demonstrating that the expression of ABC1 is regulated by cellular conditions related to apolipoprotein-mediated cholesterol efflux. In contrast, fibroblast cells from TD patients are not regulated by effectors of cholesterol efflux. First, the cAMP-inducible level of ABC1 mRNA in both TD1 and TD2 cells is only approximately 40% of that in normal cells. Further, exposure of cholesterol-loaded cells to Apo A-I either did not alter ABC1 expression (TD1 cells) or slightly increased ABC1 expression (TD2 cells). These results reflect the defect in Apo-A-I-mediated cholesterol efflux described for TD cells. Interestingly, growth of cells in serum-containing medium suppressed ABC1 message to near the limit of detection (data not shown). This may reflect the fact that functioning of the lipid efflux pathway requires cell quiescence or other cellular states of reduced cholesterol need. In conclusion, conditions that are associated with increased efflux of cellular cholesterol (i.e., cholesterol loading, cAMP treatment, serum deprivation) also result in increased expression of ABC1 mRNA in normal fibroblast cells. Conversely, exposure of cholesterol-loaded normal fibroblast cells to apo A-I reduces ABC1 expression.

### EXAMPLE 10

This example demonstrates that ligands for the LXR nuclear receptor, such as 20-hydroxycholesterol, and ligands for the RXR receptor, such as 9-cis retinoic acid, can increase ABC1 gene expression in mouse RAW 264.7 cells.

LXR nuclear receptors are transcription factors that form obligate heterodimers with the nuclear receptor RXR, and are activated to enhance transcription of their target genes by binding a class of oxysterols including 22-hydroxycholesterol and 20-hydroxycholesterol (Janowski et al., Nature, 383:728-731 (1996)). As such, they are candidates for the mediation of cholesterol-induced gene transcription. Further, in light of studies which showed that ABC mRNA and protein increase in fibroblasts and macrophages in response to cholesterol loading, and other studies which showed that LXR and RXR expression increase in cholesterol-loaded macrophage cells by exposure to oxidized LDL, the LXR and RXR nuclear receptors are highly plausible candidates for transcriptional activators of the ABC1 gene. To determine whether LXR and RXR receptors play a role in ABC1 gene expression, the level of ABC1 mRNA was measured in response to 20-hydroxycholesterol and 9-cis retinoic acid.

Mouse RAW 264.7 cells were grown to subconfluence in DMEM/10% FBS and then treated for 24 hours in serum-free DMEM/BSA with either 9-*cis* retinoic acid (10µM), 20-hydroxycholesterol (10µM), or both ligands together (20µM total). Control cells received ethanol vehicle only (0.1% v/v). RNA was extracted, treated with DNase, and ABC1 mRNA measured by RT-PCR using PE Biosystems SYBR Green Technology as described in Example 9. Figure 9 shows that treatment with either 20-hydroxycholesterol or *9-cis* retinoic acid results in an increase in ABC1 mRNA expression. In addition, Figure 9 shows that treatment with both ligands together results in a markedly synergistic effect, with an approximate 6-fold increase over the ABC1 expression observed with either ligand alone. These results demonstrate that ligands for the nuclear receptors LXR and RXR can increase the expression of the ABC1 gene.

### EXAMPLE 11

This example demonstrates that enhanced expression of ABC1 protein in the plasma membrane is associated with lipid efflux.

Cell-surface labeling and immunoprecipitation was used to determine whether increased expression of ABC1 protein in the plasma membrane is correlated with an increase in cholesterol efflux (Figure 10). The relative amount of ABC1 on the cell surface was determined by cross-linking surface proteins on intact cells with the membrane-impermeable agent sulfo-NHS-biotin, followed by the steps of membrane solubilization, immunoprecipitation with ABC1 antibody, SDS-PAGE, and detection with streptavidin.

Cell Culture: Normal and TD1 fibroblast cells were immortalized as described in Example 1. Both normal and TD1 cells were cultured under control conditions and conditions known to increase apolipoprotein-mediated cholesterol afflux (Oram, et al., J. Lip. Res., 40: 1769-1781 (1999)). Control cells were grown to confluence in DMEM/10% FBS and then incubated in DMEM/BSA for 18 hours with no additives (control). cAMP-treated cells were grown to confluence in DMEM/10% FBS and then incubated in DMEM/BSA for 18 hours with 1mM 8-Br-cAMP(cAMP). Cholesterol-loaded cells were grown to confluence in DMEM/10% FBS and then incubated in DMEM/BSA for 48 hours with 30µg/ml cholesterol plus 18 hours with no additives (cholesterol). Cholesterol-loaded cells treated with cAMP were grown to confluence in DMEM/10% FBS and then incubated in DMEM/BSA for 48 hours with 30µg/ml cholesterol plus 18 hours with 1mM 8-Br-cAMP (cholesterol + cAMP).

Cell-Surface Labeling: For selective labeling of plasma membrane ABC1, the cells were incubated for 30 minutes at 0°C with PBS containing 1 mg/ml sulfo-NHS-biotin (Pierce, Rockford, IL; Cat. #21217) to biotinylate cell-surface proteins (see Walker et al., Biochemistry, 50:14009-14014 (1993)).

Immunoprecipitation: Rabbit antiserum for ABC1 was raised against a synthetic peptide corresponding to the deduced peptide KNQTVVDAVLTSFLQDEKVKES located at the C-terminus of human ABC1. Immunoprecipitation was performed by solubilizing the cells in PBS containing 1% Triton X-100 (Sigma, St. Louis, MO) and protease inhibitors leupeptin (1mM), pepstatin (1mM), and aprotinin (1mM). The cell extract was incubated overnight at 4°C with anti-ABC1 antiserum at 1:200 dilution followed by an additional 1 hour incubation with 5µl proteinA-coated magnetic beads (Dynal, Lake Success, NY; Cat. #1001.01). The antibody-antigen complex was sedimented with a magnet, the beads were washed twice with 1 % Triton-X/PBS, and the proteins were eluted with 1% acetic acid.

Detection of ABC1 Protein: The eluted biotinylated proteins were subjected to SDS-PAGE (6% gel; 150V, 5 hours) and transferred to nitrocellulose membrane (200mA, 18 hours). The nitrocellulose was probed with streptavidin-horse radish peroxidase (Amersham Pharmacia, Piscataway, NJ; Cat. #RPN 1231) diluted 300-fold and detected by enhanced chemiluminescence labeling (ECL) according to vendor's protocol (Amersham Pharmacia, Piscataway, NJ). To test for possible biotinylation of intracellular proteins, the post-immunoprecipitation supernatant was treated with a mouse monoclonal antibody to the intracellular protein β-COP and immunoprecipitated biotinylated β-COP was assayed by streptavidin blotting. None was detected.

Results: As shown in Figure 10, the 240 kDa ABC1 protein appears as a doublet. The ABC1 protein is partially localized to the plasma membrane in both normal (10A) and TD1 (10B) fibroblast cells. Similar results were seen with a second normal fibroblast cell line and with TD2 fibroblasts (data not shown). Cell-surface expression of ABC1 was increased slightly when cells grown in serum (normal and TD1 cells) were treated with 8-Br-cAMP. Serum deprivation and cholesterol-loading of both normal and TD1 cells markedly increased cell-surface expression of ABC1, which was further enhanced by cAMP treatment. These results indicate that expression of ABC1 at the cell surface is regulated by conditions that enhance apolipoprotein-mediated lipid efflux, consistent with the idea that its localization to the plasma membrane plays a key role in its lipid transport function. The mutations in TD1 and TD2 cells do not appear to severely impair expression or processing of ABC1, implying that secondary effects on lipid transport or interactions with accessory proteins depend on its NH₂-terminal domain, where the mutations occur.

### EXAMPLE 12

This example shows that agents that inhibit the degradation of 3', 5' cyclic AMP, such as phosphodiesterase inhibitors, increase apolipoprotein A-I-mediated efflux from macrophage cells.

As shown in Figure 10, cAMP increases the activity of ABC1. The present studies were performed to determine the cholesterol efflux from macrophage cells in the presence of elevated cAMP. Elevated levels of cAMP can be attained in the presence of agents that either stimulate cAMP synthesis or inhibit the degradation of cAMP. For example, rolipram is a compound that regulates cAMP levels by inhibiting phosphodiesterases, a group of enzymes that degrade cAMP. The effect of elevated cAMP on cholesterol efflux was determined using the apolipoprotein-mediated cholesterol efflux assays described in Example 1. Briefly, RAW 264.7 cells suspended at a density of 1.25 x 10⁵ cells/ml were grown in DMEM/10% FBS supplemented with pyruvate. After 24 hours, the medium was removed and replaced with DMEM/BSA plus radiolabeled cholesterol (1 µCi/ml ³[H]-cholesterol) and 50 µg/ml of acetylated LDL for 24 hours. The cells were then maintained for 24 hours in equilibrium medium consisting of DMEM/BSA plus either apo A-I alone (20 µg/ml), apo A-I and 8-bromo 3', 5' cAMP (1mM) or apo A-I and rolipram (50 µM). After 12-24 hours, [³H]cholesterol efflux was measured by scintillation counting as described in Example 1 and calculated as the percentage of total radiolabeled cholesterol appearing in the medium. The results indicated that cholesterol-loaded control cells that received no apo A-I showed a 3% cholesterol efflux, while cells that received apo A-I only showed a 5% efflux. Cholesterol-loaded cells that received apo A-I and cAMP showed a 32% cholesterol efflux, demonstrating that elevated cAMP promotes cholesterol efflux. Similarly, cells that received apo A-I and a phosphodiesterase inhibitor (rolipram) showed a 17% cholesterol efflux.

### EXAMPLE 13

This example shows that agents that are ligands for nuclear receptors, such as LXR, RXR, and PRAR nuclear receptors, increase apolipoprotein A-I-mediated efflux from macrophage cells.

To determine whether ligands for nuclear receptors affect the process of apolipoprotein-mediated cholesterol efflux, various ligands were tested using the apo A-I-mediated cholesterol efflux assay described in Example 12. The nuclear receptor superfamily includes several members, such as the liver receptor LXR, the retinoid receptor RXR, and the peroxisome proliferator-activated receptor PPAR, which have been implicated in lipid metabolism (Russell. D.W., Cell, 97:539-542 (1999); Spiegelman, B.W., Cell, 93:153-155 (1998); Janowski et al., Nature, 383:728-731 (1996)). Further, ligands for some of these receptors have been observed to increase plasma HDL and gene expression profiling (microarray) data have shown that hormone receptors respond to cholesterol loading via oxidized LDL. Using the above-described assay, 9 cis-retinoic acid (RXR ligand), oxysterol (LXR ligand), and fenfibrate (PPAR ligand) was tested to determine the effect on cholesterol efflux. Cholesterol-loaded control cells that received no apo A-I showed a 3% cholesterol efflux, while cells that receive apo A-I only showed a 5% efflux. In contrast, cholesterol-loaded cells that receive apo A-I and 9 *cis*-retinoic acid (30 ng/ml) showed a 16% cholesterol efflux. Cells that receive apo A-I and oxysterol (5 µg/ml) showed a 14% cholesterol efflux. Cells that receive apo A-I and fenfibrate (3 µg/ml) showed a 10% cholesterol efflux. These results indicate that hormone receptors may be modulated to increase the rate of apolipoprotein-mediated cholesterol efflux from macrophages.

Further, when the efflux assay was performed using various concentrations of 9-*cis-*RA (0.3ng/ml, 3.0ng/ml, or 30ng/ml), the results showed that 9-*cis*-RA mediated cholesterol efflux from macrophage cells in a dose-dependent manner. Specifically, control cells (apo A-I only) showed 1890 c.p.m., 0.3 ng/ml 9-*cis*-RA showed 1522 c.p.m., 3.0 ng/ml 9-*cis*-RA showed 3568 c.p.m., and 30 ng/ml 9-*cis*-RA showed 8597 c.p.m. In addition, using a similar assay where RAW 264.7 cells were cholesterol-loaded for 48 hours, other nuclear receptor activators, such as 22-hydroxycholesterol (LXR ligand) and benzfibrate, were shown to increase cholesterol efflux (data not shown).

### EXAMPLE 14

This example shows that eicosanoids, such as prostaglandin E1 and prostacyclin PG12, increase apolipoprotein A-I-mediated efflux from macrophage cells.

Eicosanoids, such as prostaglandins and prostacyclins, have been shown to be effective in the treatment of hypercholesterolemia. To determine whether eicosanoids affect the process of apolipoprotein-mediated cholesterol efflux, PGE1 and PG12 were tested using the apo A-I-mediated cholesterol efflux assay described in Example 12. This assay showed that cholesterol-loaded control cells that receive no apo A-I have a 3% cholesterol efflux, while cells that receive apo A-I only have a 5% efflux. Cholesterol-loaded cells that receive apo A-I and PG12 (25 nm) showed a 10% cholesterol efflux. Cells that receive apo A-I and PGE1 (25 nM) showed a 15% cholesterol efflux. These results demonstrate that eicosanoids can increase the rate of apolipoprotein-mediated cholesterol efflux from macrophages.

### EXAMPLE 15

This example demonstrates that a reporter gene under the control of an ABC1 promoter can be used to test compounds for the ability to regulate ABC1 gene expression.

The pGL3 luciferase reporter vector system (Promega, Madison, WI) was used to create a recombinant plasmid to measure reporter gene expression under control of the ABC1 promoter.

Construction of Reporter Plasmids: Plasmid pGL3-Basic (Promega, Madison, WI; Cat. #E1751) was used as a control plasmid containing the promoterless luciferase gene. The reporter construct containing the ABC1 promoter and luciferase gene was made by cloning a genomic fragment from the 5' flanking region of the ABC1 gene (hAPR1 5' promoter, corresponding to nucleotides 1080-1643 of SEQ ID NO: 3) into the SacI site of the GL3-Basic plasmid to generate plasmid GL-6a. Next, plasmid GL-6a was digested with SpeI and Acc65I. A BsiWI-SpeI fragment excised from a lambda subclone, representing the ABC1 genomic sequence corresponding to nucleotides 1-1534 of SEQ ID NO: 3 was ligated into the remaining vector/ABC1 promoter fragment produced by this digestion. The resultant plasmid, pAPR1, encodes the luciferase reporter gene under transcriptional control of 1.75 kb of the human ABC1 promoter sequence.

Transfection of Reporter Constructs: The above-described control or pAPR1 plasmid was transfected into confluent cultures of RAW 264.7 cells maintained in DMEM containing 10% fetal bovine serum. Each well of a 12 well dish was transfected for 5 hours with either pGL3-Basic, pGL3-Promoter or pAPR1 DNA (1µg), luciferase plasmid DNA (1 µg), and 12 µl of Geneporter reagent (Gene Therapy Systems, San Diego, CA; Cat. #T201007). In addition, 0.1 µg of pCMVβ plasmid DNA (Clontech, Palo Alto, CA, Cat. #6177-1) was added as a control for transfection efficiency. After 5 hours, the culture medium was replaced with serum-free DMEM/BSA in the presence of or absence of acetylated LDL (100 µg/ml) and incubated for 24 hours.

For added convenience in high throughput screening, cultured cells can be stably transfected with reporter plasmids using the following procedure. First, 5x10⁶ RAW 264.7 cells are transfected for 5 hours in a 60mm dish with 9µg of the pAPR1 plasmid and pCMVscript (Stratagene, LaJolla, CA) in 10 ml of serum-free DMEM with 50 µl Geneporter transfection reagent (Gene Therapy Systems, San Diego, CA). Subsequently, the transfection medium is replaced with complete medium and the cells incubated overnight at 37°C. Subsequently, the cells are transferred to separate dishes at dilutions ranging from 1:5 to 1:1000 and incubated in selection medium containing 800 µg/ml G418 (Life Technologies, Bethesda, MD) for 20 days. Visible colonies are picked, expanded, and assayed for luciferase activity as described below. Using this method, five clonal cell lines positive for luciferase activity were identified for use in high throughput assays.

Luciferase Assay : Following transfection, the cells in each well were lysed in 70 µl of 1X cell lysis reagent (Promega, Madison, WI, Cat. #E3971), subjected to one freeze-thaw cycle, and the lysate cleared by centrifugation for 5 minutes at 12,000g. After centrifugation, 100 µl of luciferase assay reagent (Promega, Madison, WI; Cat. #E1501) was added to 10 µl of lysate. The luciferase activity of each lysate was measured as light units using a luminometer. Additionally, the β-galactosidase activity of each lysate was measured using the chemiluminescent assay reagents supplied in the Galacto-light kit according to the manufacturer's instructions (Tropix Inc., Bedford, MA: Cat. #BL100G). The normalized luciferase activity for each lysate was determined by dividing the luciferase activity value by the determined β-galactosidase value and reported as relative light units.

Results: The luciferase activity detected in cells transfected with pAPR1 was 3.3-fold higher than the activity detected in control cells transfected with pGL3-Basic plasmid containing luciferase cDNA only. These results indicated that the transcriptional regulatory regions of ABC1 were in place. When the pAPR1 transfected cells were incubated with 100 µg/ml acetyl LDL for 24 hours, the luciferase activity was 3.25-fold higher than in cells that had not been treated with acetyl LDL. These results suggest that the genomic ABC1 sequence contains a "cholesterol responsive" element found in the 5' flanking region which mediates the cholesterol loading response of the native ABC1 gene. This reporter system can also be used to test other compounds to determine whether the compound modulates ABC1 expression.

### EXAMPLE 16

This example demonstrates an additional assay that can be used to test compounds for the ability to regulate ABC1 gene expression using a reporter gene under the control of an endogenous ABC1 promoter.

This assay involves constructing a recombination vector that contains a promoterless reporter gene and a selection marker gene. The vector is linearized and transfected into cells such that the reporter gene is integrated into the cellular genome downstream of the endogenous ABC1 promoter. Using this assay, expression of the reporter gene is driven by the endogenous ABC1 promoter in response to a test compound.

Construction of Reporter Plasmids: The recombination vector containing a promoterless reporter gene can be made starting with a 7 kb EcoRI genomic fragment of ABC1 that contains exon 0 (which includes the ABC1 start sites) and part of intron 1. Using site-directed mutagenesis, a SalI restriction site can be generated in the exon 0 sequence downstream of the two known start sites. The recombination vector is generated by inserting a DNA fragment containing a promoterless reporter gene, such as luciferase, and a promoterless selective marker, such as puromycin resistance, into the SalI site. An internal ribosome entry signal should be inserted between the reporter gene and marker gene so that the genes will be transcribed in the correct orientation. The recombination vector contains two Eco47III sites, one of which must be eliminated, using, for example, site-directed mutagenesis. The remaining Eco47III site, located upsteam of exon 0, is used to linearize the vector.

Transfection of Reporter Constructs: The linearized recombination vector containing the reporter gene and marker gene is introduced into cultured cells, including human cells, by any of the various transfection methods known in the art. For example, the linearized vector can be transfected using the methods described in Example 15. The linearized recombination vector contains ABC1 sequences which allow the vector to integrate into the cellular genome at the site of the endogenous ABC1 gene. The addition of an appropriate antibiotic to the culture medium allows the selection of only those cells in which the reporter gene and marker gene have integrated downstream of the endogenous ABC1 promoter in the proper orientation. For instance, if the vector contains a puromycin resistance gene inserted downstream of the reporter gene, the transfected cells should be grown in the presence of puromycin. Only those cells that have a properly integrated puromycin resistance gene, and can thereby encode a functional protein, will survive in the presence of puromycin. Thus, the transfected cells should be grown under conditions that induce ABC1 promoter activity in the presence of the appropriate antibiotic. Surviving cells can be clonally cultured and the DNA sequenced using PCR or southern blot analysis to test for proper integration of genomic sequences.

The resultant cells containing a reporter gene under the control of the endogenous ABC1 promoter can be used to determine whether a test compound modulates the expression of ABC1. The ABC1 modulating activity of a compound is determined by assaying the level of reporter gene expression found in the cells exposed to the test compound. For example, cells having an integrated luciferase gene can be used to determine the ABC1 modulating activity of a test compound by measuring the amount of luciferase activity found in cells exposed to the compound.

### EXAMPLE 17

This example demonstrates that ligands for nuclear receptors up-regulate the expression of a reporter gene under the control of the ABC1 promoter.

To determine whether ligands for the LXRα, LXRβ and RXRα nuclear receptors could regulate ABC1 gene expression, the pAPR1 plasmid containing the luciferase reporter gene under control of the ABC1 promoter was transfected into RAW 264.7 cells treated with at least one ligand for the nuclear receptors (Figure 12).

Construction and Transfection of Reporter Constructs: Reporter construct pAPR1 and control reporter construct pGL3-Basic were obtained as described in Example 15. RAW 264.7 cells were maintained in culture and transfected with either pGL3-Basic (1µg) or pAPR1 (1 µg) as described in Example 15. The transfected RAW 264.7 cells were treated with either ethanol (EtOH) (0.1 % v/v), 20(S)-hydroxycholesterol (20(S) OH-chol) (10 µM), 9*-cis* retinoic acid (9*-cis* RA) (10µM) or both 20(S) OH-chol and 9*-cis* RA (20µM total) for 24 hours. The luciferase activity was measured and reported as relative light units as described in Example 15.

Results: The results of this study are shown in Figure 12. Control cells transfected with pGL3-Basic showed no luciferase activity (data not shown). Cells transfected with pAPR1 produced a 19-fold increase in luciferase reporter activity in the presence of 20 OH-chol, a 16-fold increase in luciferase activity in the presence of 9-cis RA, and a 280-fold increase in luciferase activity in the presence of both ligands compared with EtOH control. These results indicate that both the sterol and retinoid elicit a strong transcription response from the ABC1 5' flanking sequence in pAPR1. Further, there is an apparent synergistic effect of the two classes of compounds, as can be seen by the dramatic increase in luciferase activity found in cells treated with both ligands. It is known that LXRα and RXRα receptors form active heterodimers. Thus, the ligand-induced activation of both nuclear receptors simultaneously could produce the observed synergistic increase in transcription.

These data demonstrate that hydroxy sterols, such as 20(S) hydroxycholesterol, and retinoids, such as 9-*cis* retinoic acid, activate the ABC1 promoter, indicating that these and related compounds can be useful in the development of therapeutic compounds to increase ABC1 expression in macrophage cells to rid peripheral sites of excess cholesterol. Additionally, the present ABC1 promoter/reporter gene screening assay can be used to screen other compounds that increase ABC1 expression to identify further therapeutic compounds.

### EXAMPLE 18

This example demonstrates the further characterization of the ABC1 promoter region, including the identification of an LXR response element.

To determine which portion of the 5' flanking region of ABC1 retains transcriptional activity in response to nuclear ligands, various plasmids containing a different portion of the 5' flanking region and a luciferase reporter gene were transfected into RAW 264.7 cells treated with at least one ligand for the nuclear receptors. Using this system, a sterol response element corresponding to nucleotides 1480-1510 of SEQ ID NO: 3 was identified. The sterol response element contains a direct repeat-4 element TGACCGatagTAACCT. Confirmation of the sterol response element was obtained using site-directed mutagenesis and band-shift assay techniques.

Construction of Reporter Constructs: Reporter construct PAPR1 and control reporter construct pGL3-Basic were obtained as described in Example 15. Reporter constructs containing either nucleotides 1-1532, 1080-1643, 1181-1643, 1292-1643, or 1394-1643 of SEQ ID NO: 3 were also constructed. A reporter construct containing nucleotides 1080-1643 of SEQ ID NO: 3 (GL-6a) was constructed as described in Example 15. A reporter construct containing nucleotides 1-1532 of SEQ ID NO: 3 was constructed by digestion of pAPR1 with Spe I and Nhe I, and re-ligation of the gel-purified vector fragment. A reporter construct containing nucleotides 1181 to 1643 was constructed by firstly digesting GL-6a with Sty I, blunting the cohesive ends with Klenow enzyme, digesting the resultant vector with Sac I, and isolating the 462 base pair blunt-Sac I cohesive end fragment. This was cloned into a vector obtained by digestion of GL-6a with Acc65 I, blunting of the cohesive ends with Klenow enzyme, digestion with Sac I and gel isolation of the vector fragment. A reporter construct containing nucleotides 1292-1643 was constructed by consecutive digestion of GL-6a with Acc65 I, blunting the ends with Klenow enzyme, digestion with Sac II, blunting the ends with T4 polymerase, and re-ligation of the gel-isolated vector fragment. A reporter construct containing nucleotides 1394-1643 was constructed by digestion of GL-6a with Acc65 I, blunting the ends with Klenow enzyme, subsequent digestion with Apa I, end-blunting with T4 polymerase and re-ligation of the gel-isolated vector fragment.

Transfection of Reporter Constructs: The RAW 264.7 cells were maintained in culture and transfected with either pGL3-Basic (1µg), pAPR1 (1 µg), or one of the other reporter constructs according to the method described in Example 15. The transfected RAW 264.7 cells were treated with either ethanol (EtOH) (0.1 % v/v), 20(S)-hydroxycholesterol (20(S) OH-chol) (10µM), 22(R)-hydroxycholesterol (22(R) OH-chol) (10µM), 9-*cis* retinoic acid (9*-cis* RA) (10µM), or both 20(S) OH-chol and 9-*cis* RA (20µM total) for 24 hours. The luciferase activity was measured and reported as relative light units as described in Example 15.

Site-Directed Mutagenesis: The sterol response element corresponding to nucleotides 1480-1510 of SEQ ID NO: 3 was mutated in the 1080-1643 sequence described above using site-directed mutagenesis. Specifically, the response element containing a direct repeat-4 element TGACCGatagTAACCT was mutated to CTGCACatagTAACCT using the GeneEditor system (Promega, Madison, WI) according to the manufacturer's protocol.

Gel-Shift Assays: Nuclear extract was prepared from RAW 264.7 cells by the method of Ohlsson et al., Cell, 45:35-44 (1986). ³²P-labeled oligonucleotides (5 ng) corresponding to the LXR response element (TCGAGTGACCGATAGTAACCTCTCGA; SEQ ID NO: 56) and its mutated counterpart (TCGAGCTGCACATAGTAACCTCTCGA; SEQ ID NO: 57) were individually incubated with 5 µg of nuclear protein for 30 minutes at room temperature in 20mM HEPES, pH 7.9, 60mM KCL, 1mM MgCl₂, 1mM DTT, 66.6µg/ml poly(dIdC), and 10% glycerol in the presence or absence of 1 µg antiserum to LXRα and LXRβ (Santa Cruz Biotechnology, Cat. No. SC-1591, Santa Cruz, CA) or antiserum to RXR (Santa Cruz Biotechnology, Cat. No. SC-774, Santa Cruz, CA). The protein-DNA complexes were applied to a 4% non-denaturing polyacrylamide gel for 1.5 hours at 150V in 0.5X TBE buffer. The protein-DNA complexes were detected by autoradiography of the dried gel.

Results: Transfection with the individual reporter constructs containing the 5' flanking region corresponding to nucleotides 1-1643 (i.e., pAPR1), 1-1532, 1080-1643, 1181-1643, 1292-1643, or 1394-1643 of SEQ ID NO: 3 each produced the same results. All of the individual constructs produced a 3 to 4-fold increase in luciferase reporter activity in the presence of 20 (S) OH-chol or 22 (R) OH-chol compared with EtOH control. Also, all of the individual constructs produced an 8 to 10-fold increase luciferase reporter activity in the presence of 9-cis RA. In addition, transfection with any of the constructs produced a 25 to 50-fold increase in luciferase activity in the presence of oxysterol ligand (either 20(S) OH-chol or 22(R) OH-chol) and retinoid ligand (9*-cis* RA) together compared with EtOH control, indicating a synergistic interaction. Each of the described constructs demonstrated comparable levels of luciferase activity in response to the ligands tested, indicating that even the shorter 5' flanking sequences contained transcriptional regulatory sequences for sterols and retinoids. Specifically, these results demonstrated that the transcriptional regulatory sequences for sterols and retinoids are located in the 5' flanking region corresponding to nucleotides 1394-1532 of SEQ ID NO: 3.

These results were confirmed by luciferase assays using a reporter construct containing the wild-type sequence corresponding to nucleotides 1080-1643 of SEQ ID NO: 3 and a reporter construct containing a mutated sequence corresponding to nucleotides 1080-1643 of SEQ ID NO: 3, wherein the sterol response element found at nucleotides 1480-1500 was mutated as described above. Transfection with the wild-type sequence produced a transcritional response, as measured by an increase in luciferase activity, in the presence of either 20(S) OH-chol or 9-*cis* RA alone and produced a synergistic response in the presence of both ligands together. In contrast, transfection with the mutated sequence did not produce a transcriptional response in the presence of 20(S) OH-chol or 22(R) OH-chol. Transfection of the mutated sequence preserved a reduced response to 9-*cis* RA, producing a 4 to 5-fold increase in transcriptional activity, rather than the 8 to 10-fold increase observed with the wild-type sequence. Transfection of the mutated sequence also abolished the synergistic transcriptional response seen in the presence of 20(S) OH-chol and 9-*cis* RNA together. These results were further confirmed by gel-shift assays using the sterol consensus sequence (nucleotides 1480-1510) and its mutated counterpart. The gel-shift assays showed that while nuclear binding proteins isolated from RAW 264.7 cells bound to the sterol consensus sequence, nuclear proteins did not bind to the mutated sequence. Furthermore, incubation of nuclear proteins with the wild-type sterol consensus sequence in the presence of LXR antiserum resulted in the formation of supershifted complexes (i.e. antibody-protein-DNA complexes), identifying the sequence as a sterol response element that binds nuclear receptor LXR. In contrast, incubation of nuclear proteins with the wild-type sterol response element in the presence of RXR antiserum did not result in the formation of supershifted complexes, indicating that RXR does not bind to this sequence. These results show that the mutation which destroys nuclear protein binding to the consensus sequence also abolishes the transcriptional response to LXR ligands and diminishes the response to RXR ligands. Furthermore, the nuclear binding studies performed in the presence of LXR or RXR antiserum confirmed that the consensus sequence found at nucleotides 1480-1510 is an LXR response element. This element also mediates a partial response to 9-*cis* RA.

## Claims

1. An isolated polynucleotide having the sequence identified as SEQ ID NO: 3.

2. An isolated polynucleotide having the sequence identified as nucleotides 1-1532, 1080-1643, 1181-1643.1292-1643, 1394-1643, or 1394-1532 of SEQ ID NO: 3.

3. The polynucleotide of claim 2, wherein the polynucleotide has the sequence identified as nucleotides 1394-1532 of SEQ ID NO: 3.

4. An isolated polynucleotide having a nucleotide sequence that is at least 80% identical to the entire length of the polynucleotide of claim 1.

5. An isolated polynucleotide having a nucleotide sequence that is at least 80% identical to the entire length of the polynucleotide of claim 2.

6. An isolated polynucleotide that is complementary to the entire length of the polynucleotide of claim 1.

7. An isolated polynucleotide that is complementary to the entire length of the polynucleotide of claim 2.

8. A composition comprising the polynucleotide of claim 1 and a suitable carrier.

9. A composition comprising the polynucleotide of claim 2 and a suitable carrier.

10. A recombinant vector comprising the polynucleotide of claim 1.

11. A recombinant vector comprising the polynucleotide of claim 2.

12. A host cell comprising the recombinant vector of claim 10*.*

13. A host cell comprising the recombinant vector of claim 11*.*

14. The recombinant vector of claim 10, further comprising at least one polynucleotide encoding a heterologous polypeptide.

15. The recombinant vector of claim 11, further comprising at least one polynucleotide encoding a heterologous polypeptide.

16. The recombinant vector of claim 14, wherein said heterologous polypeptide is selected from the group consisting of luciferase, β-galactosidase, chloramphenicol acetyl transferase, and green fluorescent protein.

17. The recombinant vector of claim 16, wherein said heterologous polypeptide is luciferase.

18. The recombinant vector of claim 17, which comprises the polynucleotide of Seq IDNO: 3 and the polynucleotide encoding luciferase in a PGL3 vector.

19. A method for screening a test compound for ABC1 expression modulating activity comprising the steps of:
operatively linking a reporter cDNA with the 5' flanking region of the mammalian ABC1 gene comprising SEQ ID NO: 3 or a polynucleotide comprising nucleotides 1-1532, 1080-1643, 1181-1643, 1292-1643, 1394-1643, or 1394-1532 of SEQ ID NO: 3 to produce a recombinant reporter construct;
transfecting the recombinant reporter construct into a population of isolated host cells;
assaying the level of reporter gene expression in a sample of the host cells;
contacting the host cells with the test compound being screened;
assaying the level of reporter gene expression in a sample of the host cells after contact with the test compound; and
comparing the relative change in the level of reporter gene expression caused by exposure to the test compound, thereby determining the ABC1 expression modulating activity.

20. The method of claim 19, wherein the reporter cDNA is selected from the group consisting of luciferase, β-galactosidase, chloramphenicol acetyl transferase, and green fluorescent protein cDNA.

21. The method of claim 19, wherein the host cell is a mammalian cell.

22. The method of claim 20, wherein the recombinant reporter construct comprises the polynucleotide of SEQ ID NO: 3 and the luciferase reporter cDNA in a pGL3 vector.

23. A kit suitable for screening a compound to determine the ABC1 expression modulating activity of the compound comprising a recombinant reporter construct comprising a reporter cDNA operatively linked to an expression modulating portion of the mammalian ABC1 gene comprising the polynucleotide of claim 1 or 2 in an amount sufficient for at least one assay and instructions for use.

24. The kit of claim 23, wherein the reporter cDNA is selected from the group consisting of luciferase, β-galactosidase, chloramphenicol acetyl transferase, and green fluorescent protein cDNA.

25. The kit of claim 23, wherein the reporter cDNA is a luciferase cDNA.

26. The kit of claim 25, wherein the recombinant reporter construct comprises the polynucleotide of SEQ ID NO: 3 and the luciferase reporter cDNA in a pGL3 vector.

27. The kit of claim 23, further comprising means for detecting the reporter gene.

## Patentansprüche

1. Isoliertes Polynukleotid mit der als SEQ ID NO: 3 identifizierten Sequenz.

2. Isoliertes Polynukleotid mit der als Nukleotide 1-1532, 1080-1643, 1181-1643, 1292-1643, 1394-1643 oder 1394-1532 von SEQ ID NO: 3 identifizierten Sequenz.

3. Polynukleotid nach Anspruch 2, wobei das Polynukleotid die als Nukleotide 1394-1532 von SEQ ID NO: 3 identifizierte Sequenz aufweist.

4. Isoliertes Polynukleotid mit einer Nukleotidsequenz, die mindestens 80% zu der gesamten Länge des Polynukleotids nach Anspruch 1 identisch ist.

5. Isoliertes Polynukleotid mit einer Nukleotidsequenz, die mindestens 80% zu der gesamten Länge des Polynukleotids nach Anspruch 2 identisch ist.

6. Isoliertes Polynukleotid, das zu der gesamten Länge des Polynukleotids nach Anspruch 1 komplementär ist.

7. Isoliertes Polynukleotid, das zu der gesamten Länge des Polynukleotids nach Anspruch 2 komplementär ist.

8. Zusammensetzung, die das Polynukleotid nach Anspruch 1 und einen geeigneten Träger umfasst.

9. Zusammensetzung, die das Polynukleotid nach Anspruch 2 und einen geeigneten Träger umfasst.

10. Rekombinanter Vektor, der das Polynukleotid nach Anspruch 1 umfasst.

11. Rekombinanter Vektor, der das Polynukleotid nach Anspruch 2 umfasst.

12. Wirtszelle, die den rekombinanten Vektor nach Anspruch 10 umfasst.

13. Wirtszelle, die den rekombinanten Vektor nach Anspruch 11 umfasst.

14. Rekombinanter Vektor nach Anspruch 10, der ferner mindestens ein Polynukleotid umfasst, das für ein heterologes Polypeptid kodiert.

15. Rekombinanter Vektor nach Anspruch 11, der ferner mindestens ein Polynukleotid umfasst, das für ein heterologes Polypeptid kodiert.

16. Rekombinanter Vektor nach Anspruch 14, wobei das heterologe Polypeptid ausgewählt ist aus der Gruppe, bestehend aus Luciferase, β-Galactosidase, Chloramphenicol-Acetyl-Transferase und grün-fluoreszierendem Protein.

17. Rekombinanter Vektor nach Anspruch 16, wobei das heterologe Polypeptid Luciferase ist.

18. Rekombinanter Vektor nach Anspruch 17, der das Polynukleotid von SEQ ID NO: 3 und das Polynukleotid, das für Luciferase kodiert, in einem pGL3-Vektor umfasst.

19. Verfahren zum Screenen einer Testverbindung bezüglich ABC1-Expression-modulierender Aktivität, das die Schritte umfasst:
operables Koppeln einer Reporter-cDNA mit der 5'-flankierenden Region des Säuger-ABC1-Gens, die SEQ ID NO: 3 oder ein Polynukleotid umfasst, das die Nukleotide 1-1532, 1080-1643, 1181-1643, 1292-1643, 1394-1643 oder 1394-1532 von SEQ ID NO: 3 umfasst, um ein rekombinantes Reporterkonstrukt zu erzeugen,
Transfektion des rekombinanten Reporterkonstrukts in eine Population von isolierten Wirtszellen,
Testen des Grads an Reportergenexpression in einer Probe der Wirtszellen, In-Kontakt-Bringen der Wirtszellen mit der zu screenenden Testverbindung,
Testen des Grads an Reportergenexpression in einer Probe der Wirtszellen nach Kontaktieren mit der Testverbindung und
Vergleichen der relativen Änderung hinsichtlich des Grads an Reportergenexpression, die durch die Exposition gegenüber der Testverbindung hervorgerufen wurde, wodurch die ABC1-Expression-modulierende Aktivität bestimmt wird.

20. Verfahren nach Anspruch 19, wobei die Reporter-cDNA ausgewählt ist aus der Gruppe, bestehend aus Luciferase-, β-Galactosidase-, Chloramphenicol-Acetyl-Transferase- und grün-fluoreszierendes Protein-cDNA.

21. Verfahren nach Anspruch 19, wobei die Wirtszelle eine Säugerzelle ist.

22. Verfahren nach Anspruch 20, wobei das rekombinante Reporterkonstrukt das Polynukleotid von SEQ ID NO: 3 und die Luciferase-Reporter-cDNA in einem pGL3-Vektor umfasst.

23. Kit, der zum Screenen einer Verbindung, um die ABC1-Expression-modulierende Aktivität der Verbindung zu bestimmen, geeignet ist, wobei der Kit ein rekombinantes Reporterkonstrukt, umfassend eine Reporter-cDNA in operabler Kopplung an einen Expression-modulierenden Teil des Säuger-ABC1-Gens, der das Polynukleotid nach Anspruch 1 oder 2 umfasst, in einer Menge, die für mindestens einen Test ausreichend ist, und Anleitungen für eine Verwendung umfasst.

24. Kit nach Anspruch 23, wobei die Reporter-cDNA ausgewählt ist aus der Gruppe, bestehend aus Luciferase-, β-Galactosidase-, Chloramphenicol-Acetyl-Transferase- und grün-fluoreszierendes Protein-cDNA.

25. Kit nach Anspruch 23, wobei die Reporter-cDNA eine Luciferase-cDNA ist.

26. Kit nach Anspruch 25, wobei das rekombinante Reporterkonstrukt das Polynukleotid von SEQ ID NO: 3 und die Luciferase-Reporter-cDNA in einem pGL3-Vektor umfasst.

27. Kit nach Anspruch 23, der ferner Mittel für einen Nachweis des Reportergens umfasst.

## Revendications

1. Polynucléotide isolé ayant la séquence identifiée comme étant SEQ ID NO: 3.

2. Polynucléotide isolé ayant la séquence identifiée comme étant les nucléotides 1-1532, 1080-1643, 1181-1643, 1292-1643, 1394-1643, ou 1394-1532 de SEQ ID NO: 3.

3. Polynucléotide selon la revendication 2, dans lequel le polynucléotide a la séquence identifiée comme étant les nucléotides 1394-1532 de SEQ ID NO: 3.

4. Polynucléotide isolé ayant une séquence nucléotidique qui est identique pour au moins 80% à la longueur totale du polynucléotide selon la revendication 1.

5. Polynucléotide isolé ayant une séquence nucléotidique qui est identique pour au moins 80% à la longueur totale du polynucléotide selon la revendication 2.

6. Polynucléotide isolé qui est complémentaire de la longueur totale du polynucléotide selon la revendication 1.

7. Polynucléotide isolé qui est complémentaire de la longueur totale du polynucléotide selon la revendication 2.

8. Composition comprenant le polynucléotide selon la revendication 1 et un support approprié.

9. Composition comprenant le polynucléotide selon la revendication 2 et un support approprié.

10. Vecteur recombinant comprenant le polynucléotide selon la revendication 1.

11. Vecteur recombinant comprenant le polynucléotide selon la revendication 2.

12. Cellule hôte comprenant le vecteur recombinant selon la revendication 10.

13. Cellule hôte comprenant le vecteur recombinant selon la revendication 11.

14. Vecteur recombinant selon la revendication 10, comprenant en outre au moins un polynucléotide codant pour un polypeptide hétérologue.

15. Vecteur recombinant selon la revendication 11, comprenant en outre au moins un polynucléotide codant pour un polypeptide hétérologue.

16. Vecteur recombinant selon la revendication 14, dans lequel ledit polypeptide hétérologue est choisi dans le groupe consistant en luciférase, β-galactosidase, chloramphénicol acétyl transférase, et protéine fluorescente verte.

17. Vecteur recombinant selon la revendication 16, dans lequel ledit polypeptide hétérologue est la luciférase.

18. Vecteur recombinant selon la revendication 17, qui comprend le polynucléotide de SEQ ID NO: 3 et le polynucléotide codant pour la luciférase dans un vecteur pGL3.

19. Procédé pour le criblage d'un composé d'essai pour l'activité de modulation de l'expression de ABC1, comprenant les étapes de:
liaison opérationnelle d'un ADNc reporter avec la région d'encadrement 5' du gène ABC1 mammalien comprenant SEQ ID NO: 3 ou un polynucléotide comprenant les nucléotides 1-1532, 1080-1643, 1181-1643, 1292-1643, 1394-1643, ou 1394-1532 de SEQ ID NO: 3 pour produire une construction reporter recombinante;
transfection de la construction reporter recombinante dans une population de cellules hôtes isolées;
détermination du niveau d'expression du gène reporter dans un échantillon des cellules hôtes;
mise en contact des cellules hôtes avec le composé d'essai à cribler; détermination du niveau d'expression du gène reporter dans un échantillon des cellules hôtes après mise en contact avec le composé d'essai; et
comparaison de la modification relative du niveau d'expression du gène reporter provoquée par l'exposition au composé d'essai, déterminant ainsi l'activité de modulation de l'expression de ABC1.

20. Procédé selon la revendication 19, dans lequel l'ADNc reporter est choisi dans le groupe consistant en l'ADNc de luciférase, de β-galactosidase, de chloramphénicol acétyl transférase, et de protéine fluorescente verte.

21. Procédé selon la revendication 19, dans lequel la cellule hôte est une cellule mammalienne.

22. Procédé selon la revendication 20, dans lequel la construction reporter recombinante comprend le polynucléotide de SEQ ID NO: 3 et l'ADNc reporter de luciférase dans un vecteur pGL3.

23. Kit convenant pour le criblage d'un composé pour la détermination de l'activité de modulation de l'expression de ABC1 du composé comprenant une construction reporter recombinante comprenant un ADNc reporter lié de manière opérationnelle à une portion de modulation de l'expression du gène ABC1 mammalien comprenant le polynucléotide selon la revendication 1 ou 2 en une quantité suffisante pour au moins un essai et des instructions d'utilisation.

24. Kit selon la revendication 23, dans lequel l'ADNc reporter est choisi dans le groupe consistant en l'ADNc de luciférase, de β-galactosidase, de chloramphénicol acétyl transférase, et de protéine fluorescente verte.

25. Kit selon la revendication 23, dans lequel l'ADNc reporter est un ADNc de luciférase.

26. Kit selon la revendication 25, dans lequel la construction reporter recombinante comprend le polynucléotide de SEQ ID NO: 3 et l'ADNc reporter de luciférase dans un vecteur pGL3.

27. Kit selon la revendication 23, comprenant en outre des moyens pour la détection du gène reporter.
